# EUROPEAN PATENT APPLICATION

(11) **EP 4 324 446 A1**
(43) Date of publication of application: **21.02.2024**
(21) Application number: 22787924.4
(22) Date of filing: 16.03.2022
(51) Int. Cl.: A61J 3/00, G16H 20/10, G16H 40/40

(54) **MEDICINE DISPENSING SYSTEM, ADJUSTMENT SYSTEM, AND PROGRAM**

(30) Priority: 16.04.2021 JP 2021069764; 07.01.2022 JP 2022001830
(71) Applicant: YUYAMA MFG. CO., LTD., Toyonaka-shi, Osaka 561-0841 (JP)
(72) Inventor: TERADA, Tetsuya, Toyonaka-shi, Osaka 561-0841 (JP); MIYAGAWA, Tomoya, Toyonaka-shi, Osaka 561-0841 (JP); FUJII, Noriyoshi, Toyonaka-shi, Osaka 561-0841 (JP)
(74) Representative: Zoli, Filippo
(86) International application number: PCT/JP2022/011817
(87) International publication number: WO 2022/219998

(57) **Abstract**

A medicine dispensing system and a program which can support dispensing of medicines using a medicine dispensing part capable of dispensing different types of medicines are provided. A medicine dispensing system (500) according to the present invention includes a determination processing part (212) that determines whether or not a medicine has been dispensed from a medicine dispensing part (2) that can be adjusted to a plurality of states capable of dispensing different types of medicine and a storage processing part (213) that stores dispensing confirmation information in a storage part (206, 202) in a state in which a correspondence relationship with the medicine dispensing part (2) can be specified when the determination processing part determines that the medicine has been dispensed from the medicine dispensing part (2).

## Description

### TECHNICAL FIELD

The present invention relates to a medicine dispensing system provided with a medicine dispensing part such as a medicine cassette capable of dispensing different types of medicines.

### BACKGROUND ART

Generally, a medicine dispensing device capable of automatically dispensing medicines from a medicine cassette based on prescription data is known. Further, as a medicine cassette used in this kind of medicine dispensing device, for example, there is known a configuration in which dimensions of a medicine dispensing path can be adjusted, and which can be used to dispense a plurality of types of medicines by adjusting the dimensions of the medicine dispensing path (see, for example, Patent Document 1).

### RELATED ART DOCUMENT

### PATENT DOCUMENT

Patent Document 1: WO2020/209100

### SUMMARY OF THE INVENTION

### PROBLEMS TO BE SOLVED BY THE INVENTION

By the way, it is also conceivable that the medicine cassette is manually adjustable by a user. In this case, a medicine to be dispensed cannot be properly dispensed from the medicine cassette unless the dimensions of the medicine dispensing path are correctly adjusted in advance according to the type of the medicine to be dispensed.

An object of the present invention is to provide a medicine dispensing system and a program which can support dispensing of medicines using a medicine dispensing part capable of dispensing different types of medicines.

### MEANS FOR SOLVING THE PROBLEMS

A medicine dispensing system according to the present invention comprises: a determination processing part that determines whether or not a medicine has been dispensed from a medicine dispensing part that can be adjusted to a plurality of states capable of dispensing different types of medicine; and a storage processing part that stores dispensing confirmation information in a storage part in a state in which a correspondence relationship with the medicine dispensing part can be specified when the determination processing part determines that the medicine has been dispensed from the medicine dispensing part.

A program according to the present invention is a program for causing a processor to execute a determination step for determining whether or not a medicine has been dispensed from a medicine dispensing part that can be adjusted to a plurality of states capable of dispensing different types of medicine and a storage step for storing dispensing confirmation information in a storage part in a state in which a correspondence relationship with the medicine dispensing part can be specified when the determination step determines that the medicine has been dispensed from the medicine dispensing part.

### EFEECTS OF THE INVENTION

According to the present invention, a medicine dispensing system and a program which can support dispensing of medicines using a medicine dispensing part capable of dispensing different types of medicines are provided.

### BRIEF DESCRIPTION OF THE DRAWINGS

FIG. 1 is a perspective view of a medicine dispensing device equipped with an adjusting device.
FIG. 2 is a perspective view of a medicine cassette and a base.
FIG. 3 is a perspective view of a state where a lid of the medicine cassette is removed.
FIG. 4 is a perspective view seen from a bottom of the medicine cassette.
FIG. 5 is a cross-sectional view of the cassette body.
FIG. 6 is a perspective view of the medicine cassette with a rotor removed.
FIG. 7 is an exploded perspective view of a rotor driving part of the cassette body.
FIG. 8A is an exploded perspective view of a partition adjusting mechanism of the cassette body.
FIG. 8B is a cross-sectional view of the cassette body showing an entry position of the partition member.
FIG. 9 is an overall perspective view of the rotor.
FIG. 10 is a bottom perspective view of the rotor.
FIG. 11 is an exploded perspective view of a depth adjusting mechanism.
FIG. 12 is an exploded perspective view of a height adjusting mechanism.
FIG. 13 is an exploded perspective view of a width adjusting mechanism.
FIG. 14 is a cross-sectional view of the cassette body for explaining a state of depth adjustment by the depth adjusting mechanism.
FIG. 15 is a cross-sectional view of the cassette body for explaining a state of height adjustment by the height adjusting mechanism.
FIGs. 16(a) and 16(b) are a plan view and a bottom view of a movable member and the width adjusting member for explaining a state of width adjustment by the width adjusting mechanism.
FIG. 17 is a perspective view of the adjusting device.
FIG. 18 is a perspective view of the adjusting device.
FIG. 19 is an internal perspective view of a base part of a device body.
FIG. 20 is an internal perspective view of a guide part of the device body.
FIG. 21 is a plan view showing a rotation mechanism of a rotating member.
FIG. 22(a) is a front view of the rotating member, FIG. 22(b) is a vertical sectional view of the rotating member, and FIG. 22(c) is a lateral sectional view of the rotating member.
FIG. 23 is a perspective view of an adjusting member.
FIG. 24 is an exploded perspective view of the adjusting member.
FIG. 25 is a bottom perspective view of an outer member of a grip part.
FIG. 26 is a perspective view of a first member of a shaft part viewed from below.
FIG. 27 is a perspective view of a second member of the shaft part viewed from below.
FIG. 28(a) is a cross-sectional view of the adjusting member, FIG. 28(b) is a cross-sectional view when the grip part is raised, and FIG. 28(c) is a cross-sectional view when a torque limiter is activated.

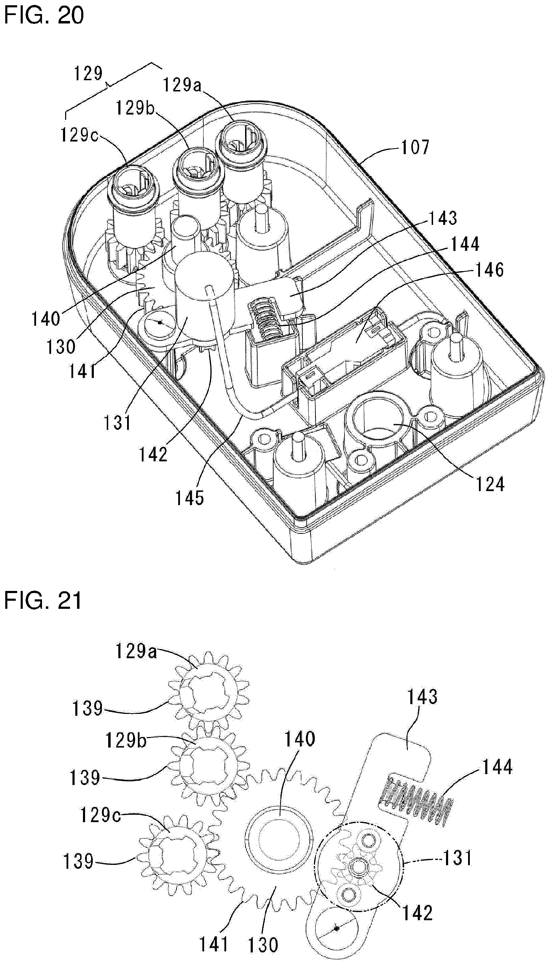

FIG. 21 is a perspective view of a tool.

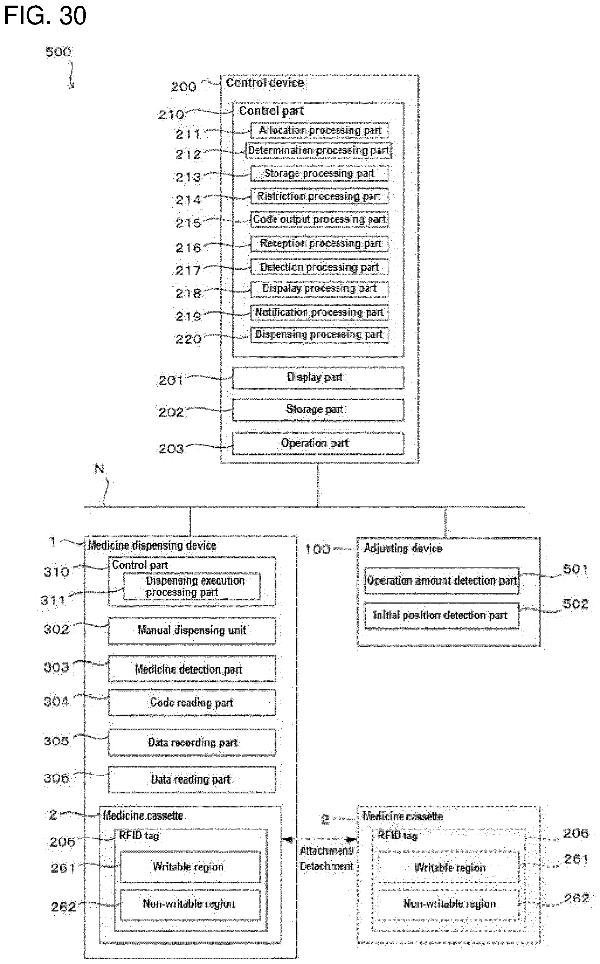

FIG. 30 is a system configuration diagram of a medicine dispensing system.

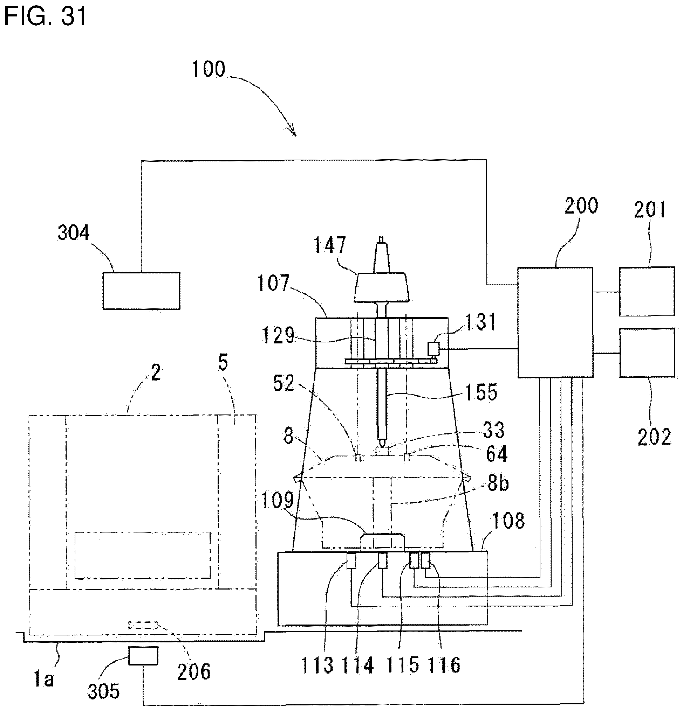

FIG. 31 is a system configuration diagram of the adjusting device.

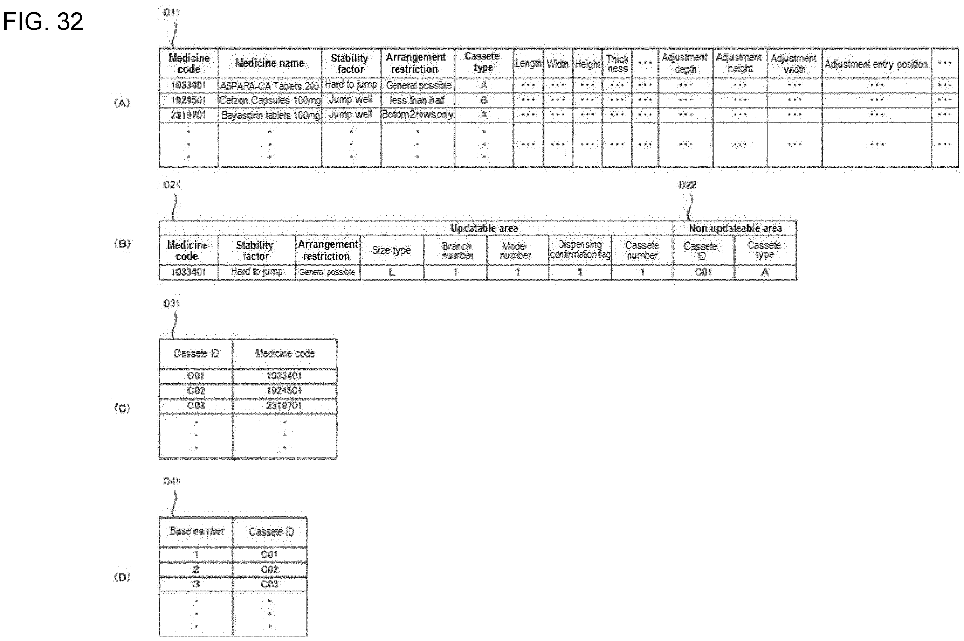

FIG. 32 is a diagram showing an example of information used in the medicine dispensing system.

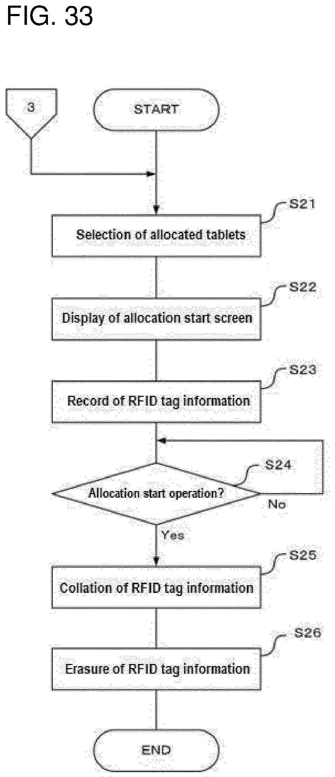

FIG. 33 is a flowchart showing an example of medicine allocation processing executed by the medicine dispensing system.

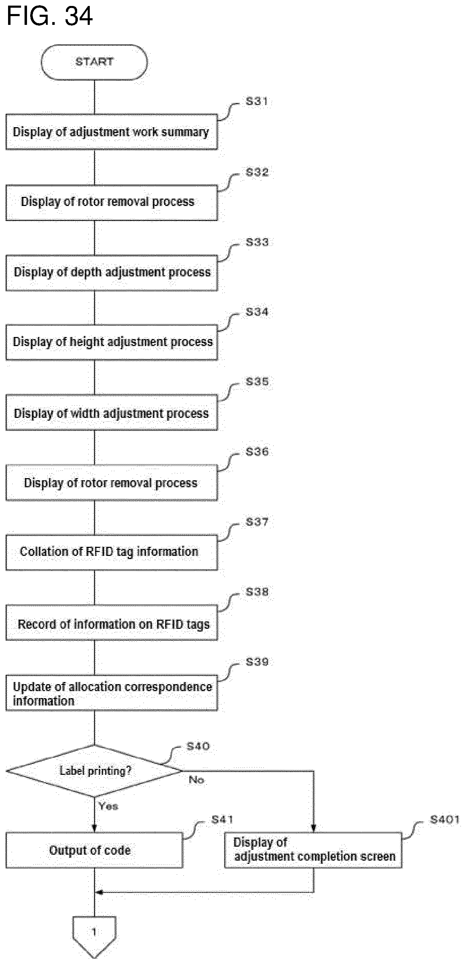

FIG. 34 is a flowchart showing an example of adjustment support processing executed by the medicine dispensing system.

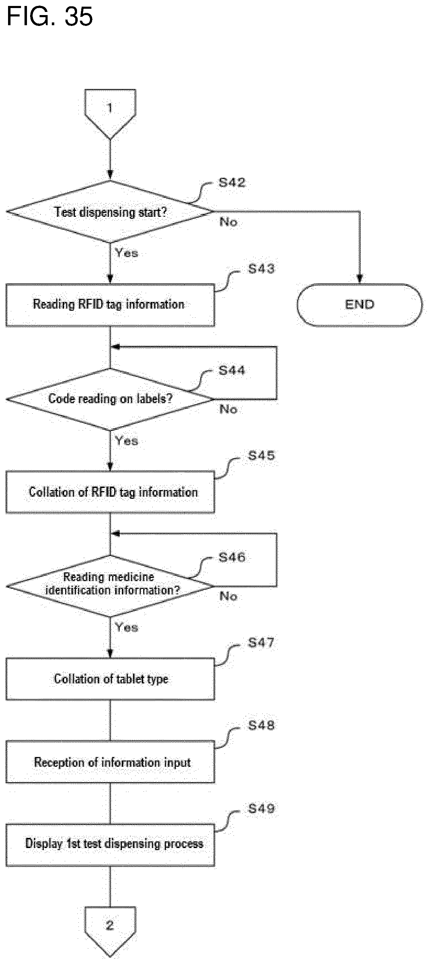

FIG. 35 is a flowchart showing an example of adjustment support processing executed by the medicine dispensing system.

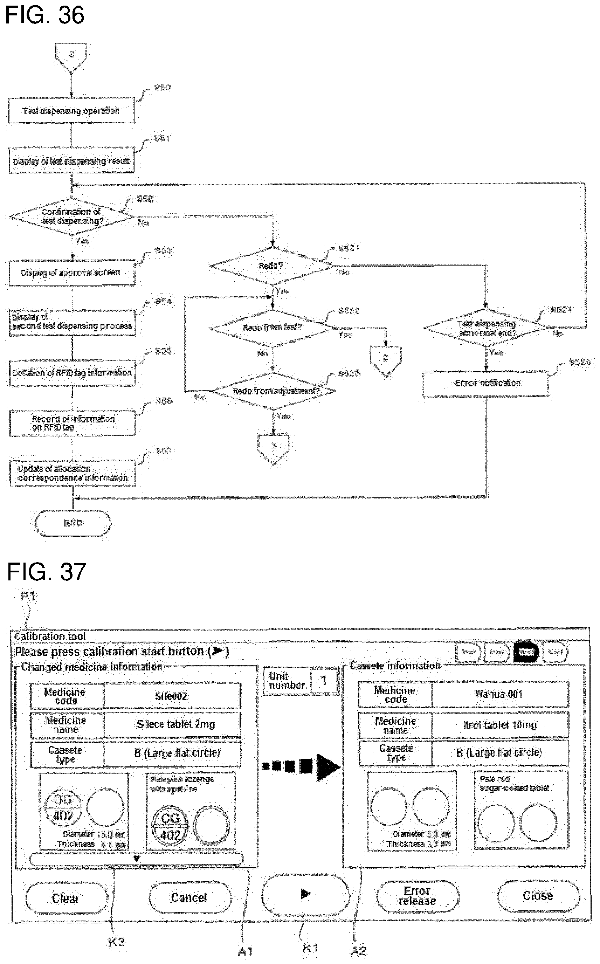

FIG. 36 is a flowchart showing an example of adjustment support processing executed by the medicine dispensing system.
FIG. 37 is a diagram showing an example of a display screen displayed by the medicine dispensing system.

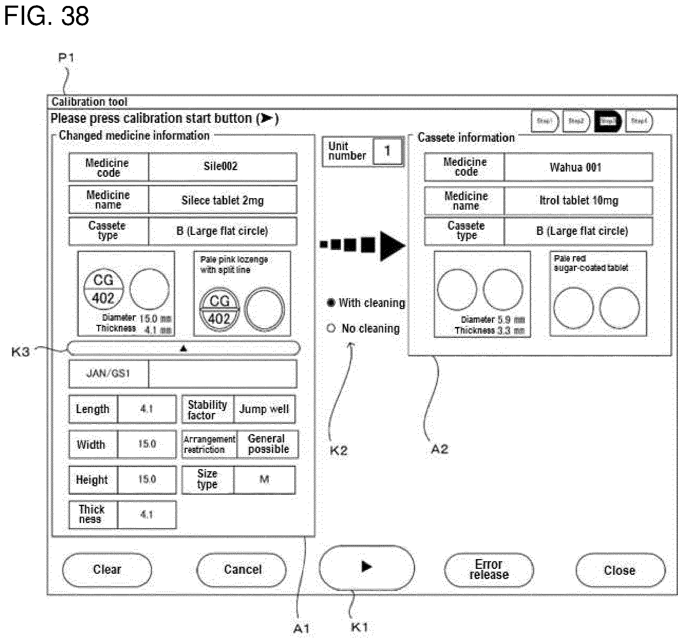

FIG. 38 is a diagram showing an example of a display screen displayed by the medicine dispensing system.

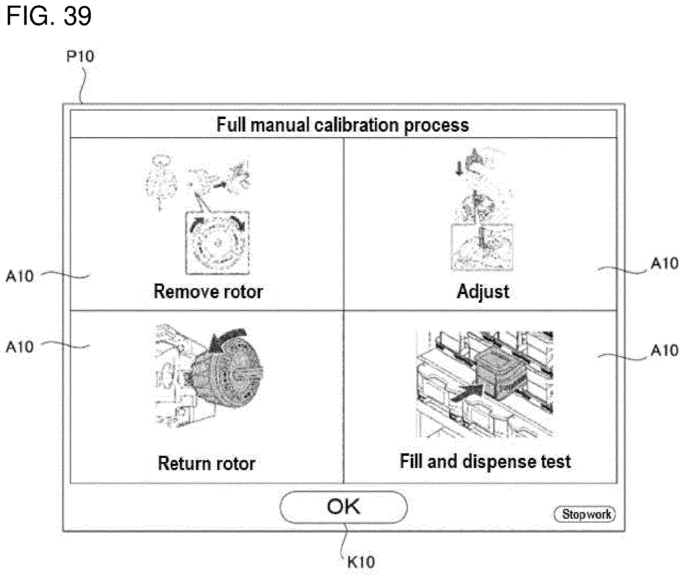

FIG. 39 is a diagram showing an example of a display screen displayed by the medicine dispensing system.

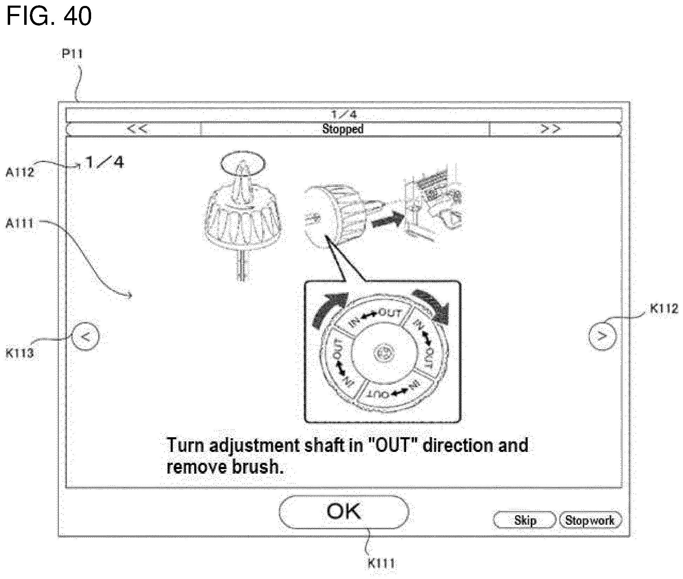

FIG. 40 is a diagram showing an example of a display screen displayed by the medicine dispensing system.

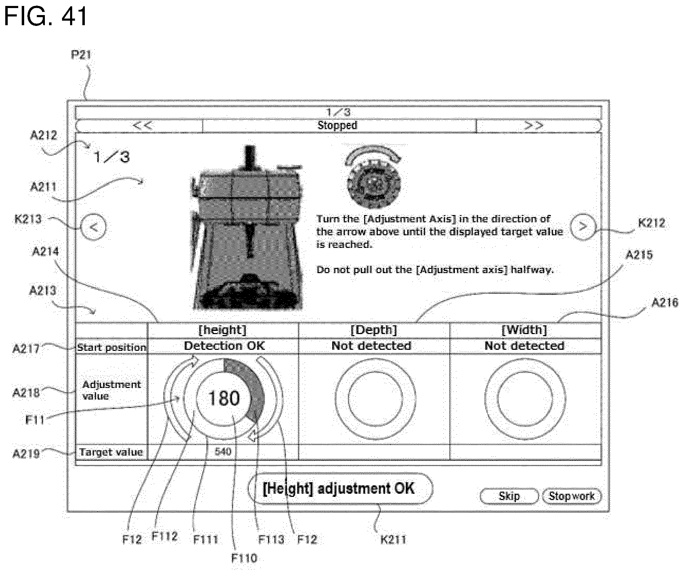

FIG. 41 is a diagram showing an example of a display screen displayed by the medicine dispensing system.

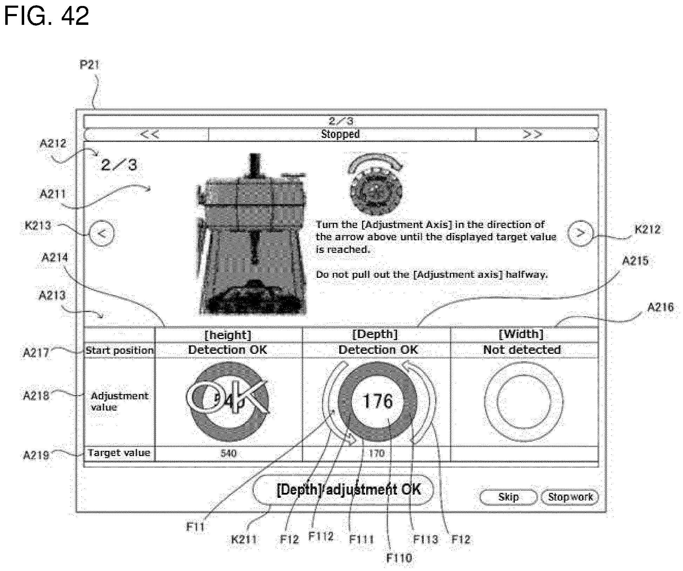

FIG. 42 is a diagram showing an example of a display screen displayed by the medicine dispensing system.

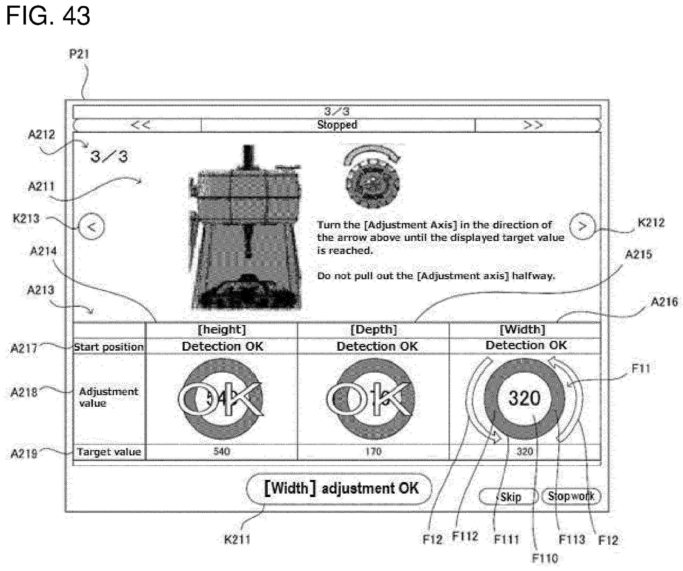

FIG. 43 is a diagram showing an example of a display screen displayed by the medicine dispensing system.

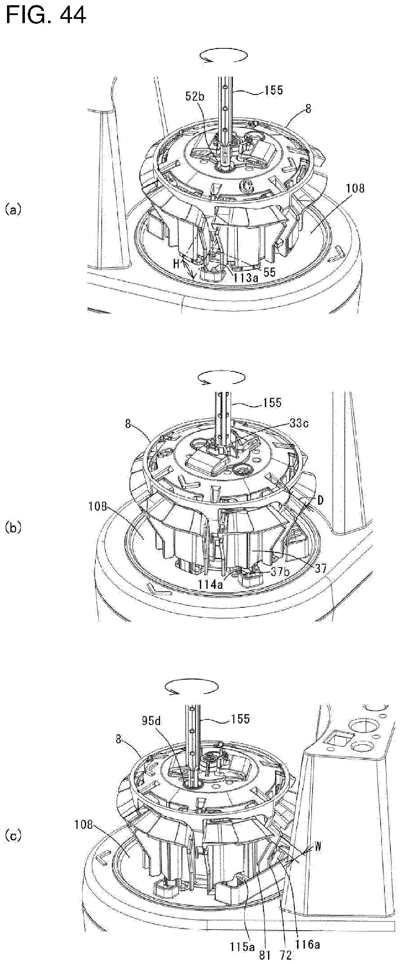

FIG. 44 is a diagram showing adjusted states of the rotor.

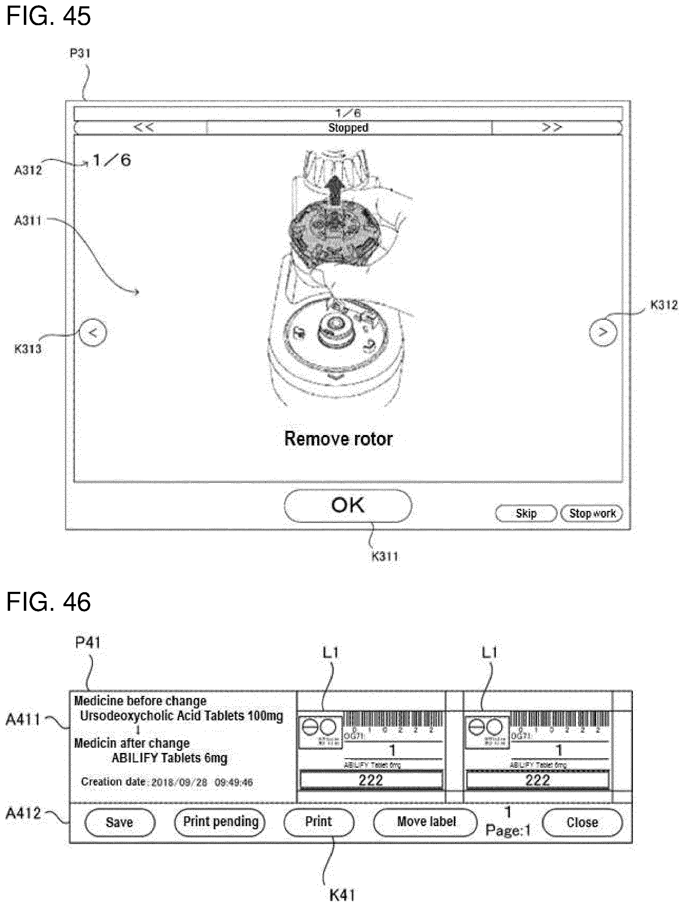

FIG. 45 is a diagram showing an example of a display screen displayed by the medicine dispensing system.
FIG. 46 is a diagram showing an example of a display screen displayed by the medicine dispensing system.

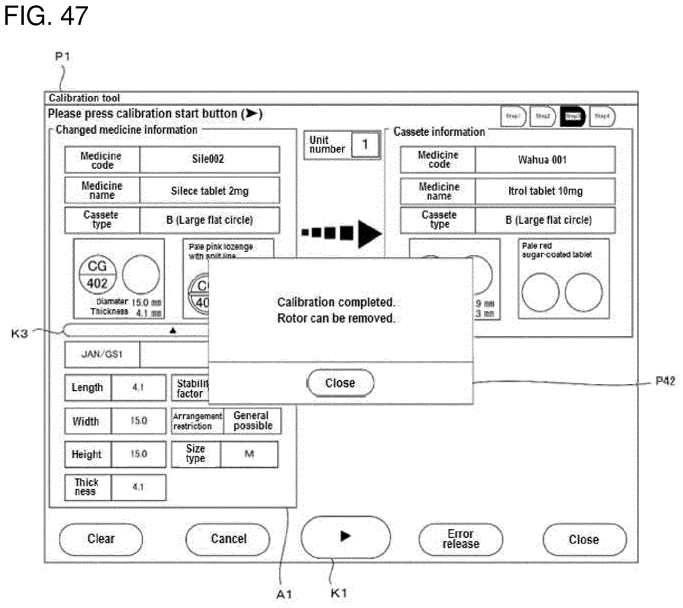

FIG. 47 is a diagram showing an example of a display screen displayed by the medicine dispensing system.

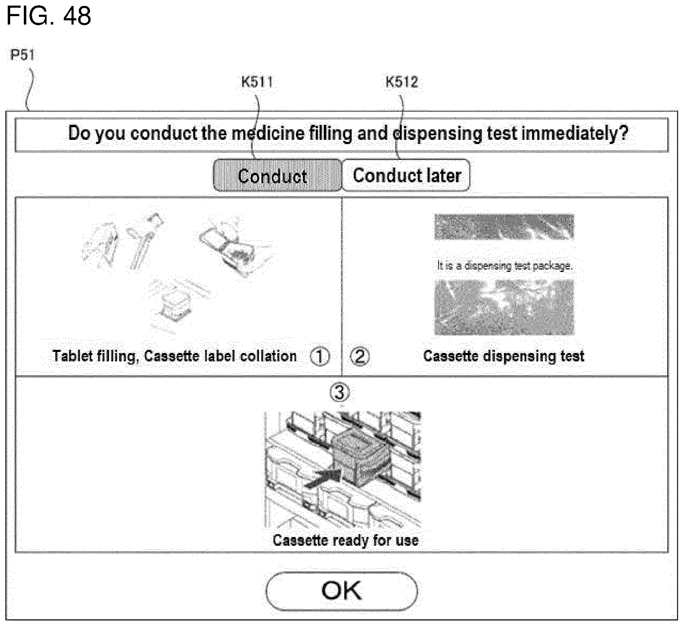

FIG. 48 is a diagram showing an example of a display screen displayed by the medicine dispensing system.

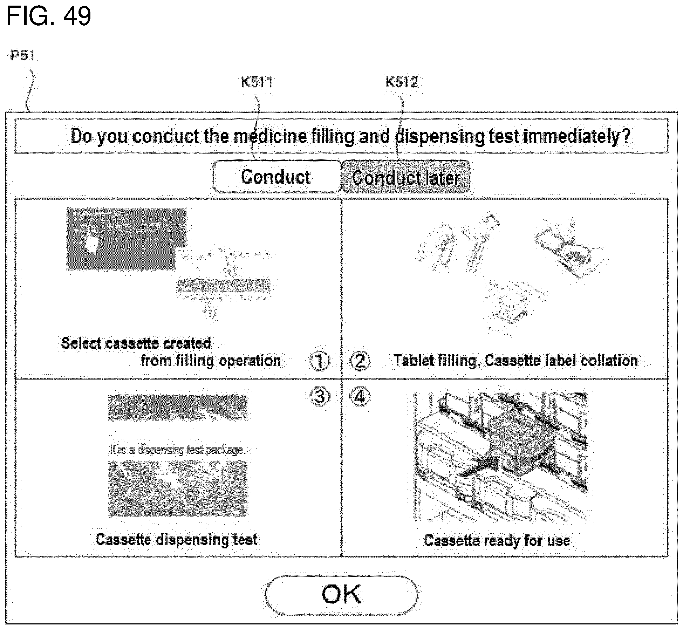

FIG. 49 is a diagram showing an example of a display screen displayed by the medicine dispensing system.

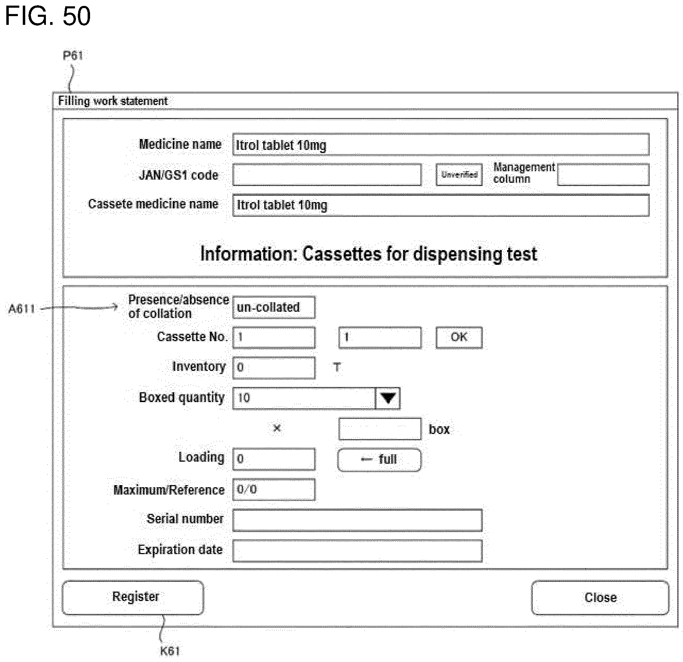

FIG. 50 is a diagram showing an example of a display screen displayed by the medicine dispensing system.

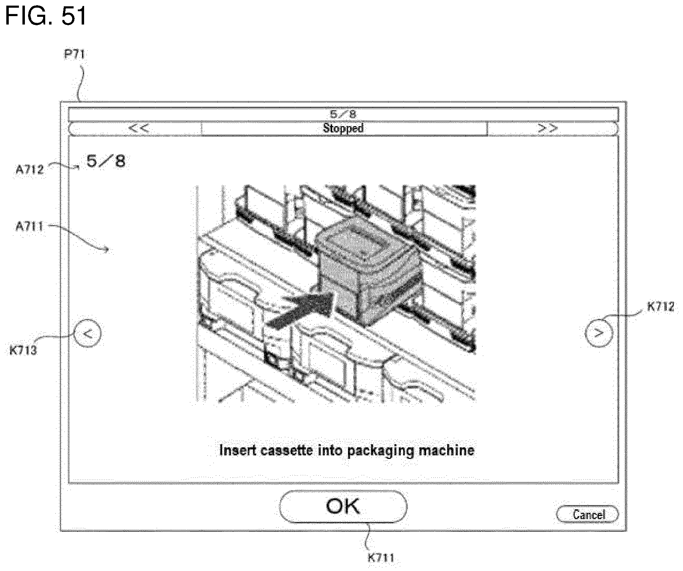

FIG. 51 is a diagram showing an example of a display screen displayed by the medicine dispensing system.

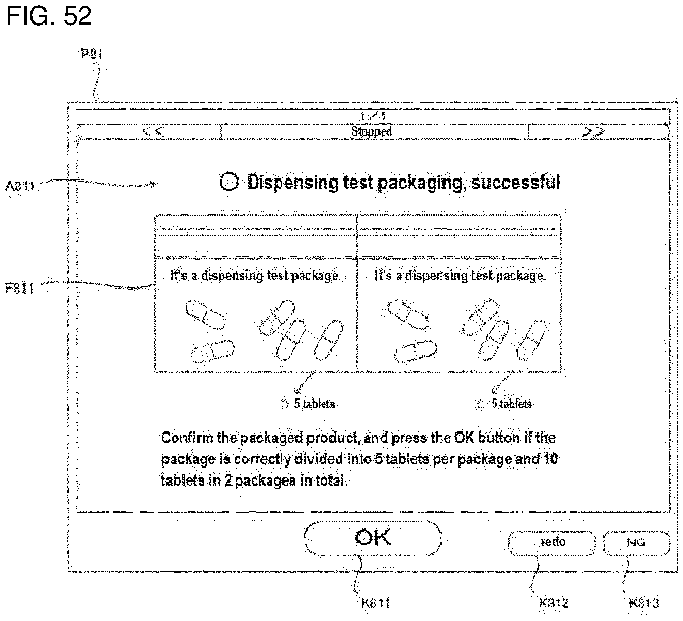

FIG. 52 is a diagram showing an example of a display screen displayed by the medicine dispensing system.

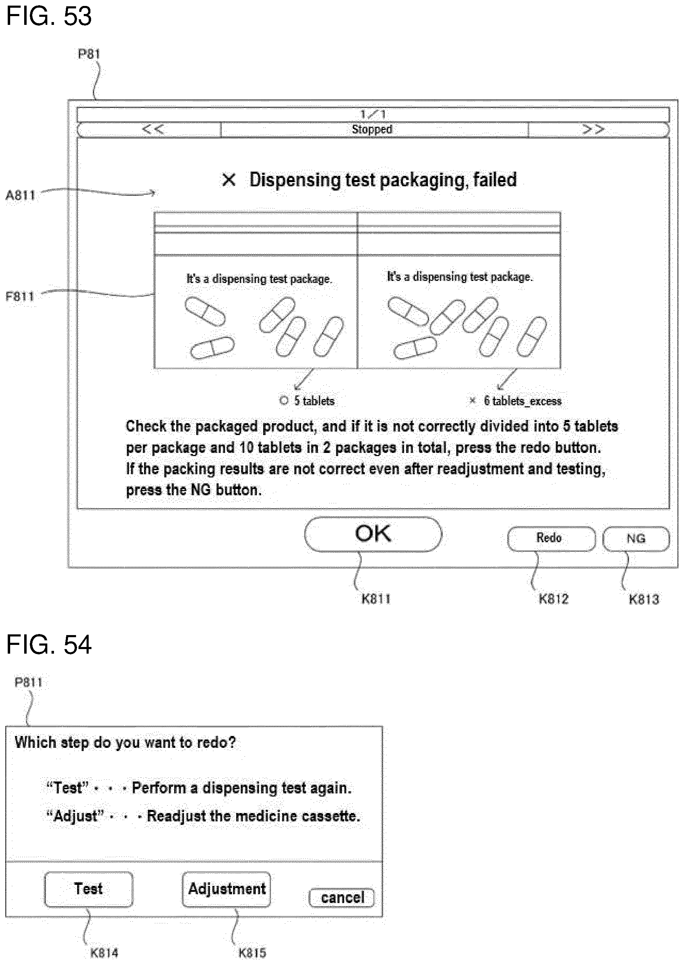

FIG. 53 is a diagram showing an example of a display screen displayed by the medicine dispensing system.
FIG. 54 is a diagram showing an example of a display screen displayed by the medicine dispensing system.

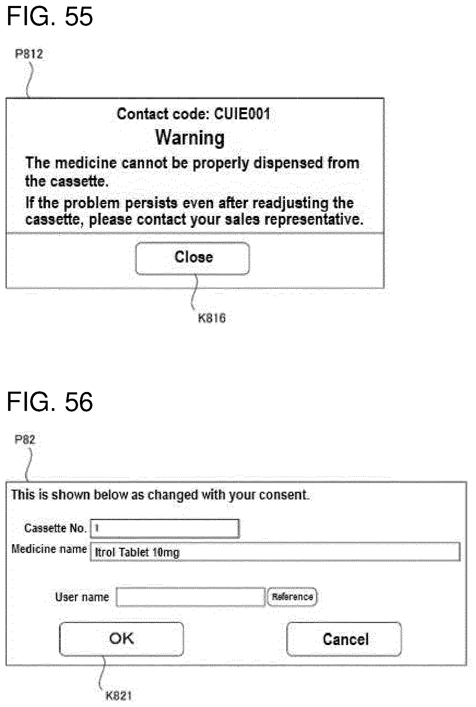

FIG. 55 is a diagram showing an example of a display screen displayed by the medicine dispensing system.
FIG. 56 is a diagram showing an example of a display screen displayed by the medicine dispensing system.

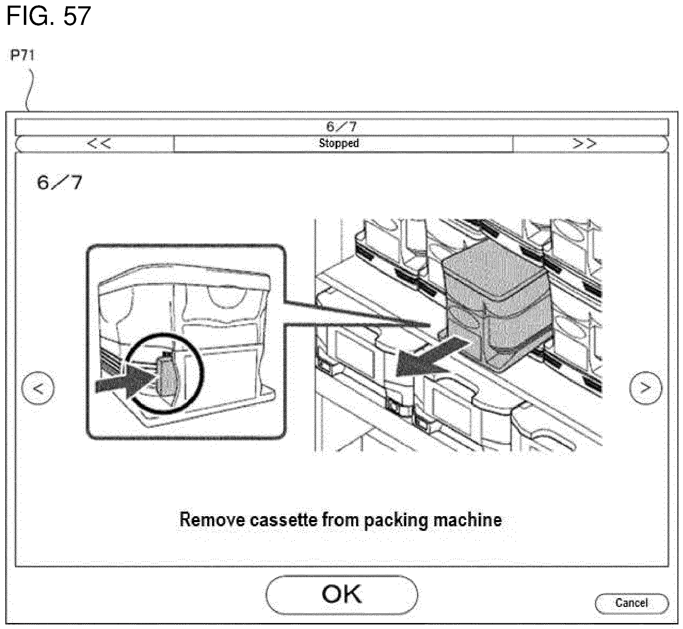

FIG. 57 is a diagram showing an example of a display screen displayed by the medicine dispensing system.

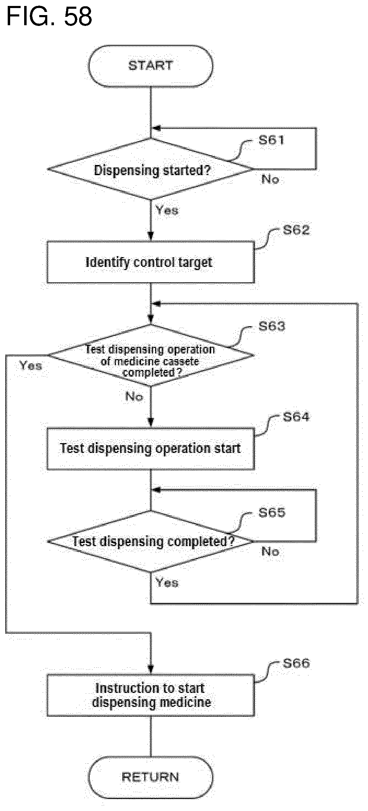

FIG. 58 is a flow chart showing an example of medicine dispensing processing executed by the medicine dispensing system.

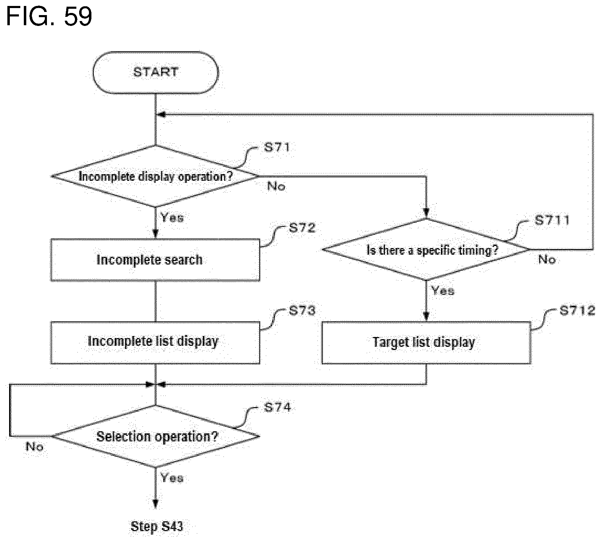

FIG. 59 is a flow chart showing an example of work support display processing executed by the medicine dispensing system.

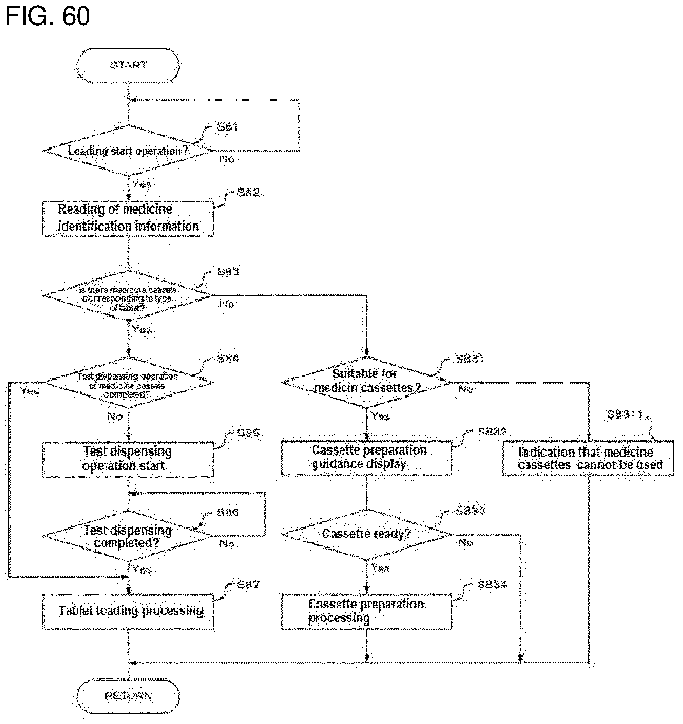

FIG. 60 is a flow chart showing an example of medicine loading support processing executed by the medicine dispensing system.

### MODE FOR CARRYING OUT THE INVENTION

The following embodiments are specific examples of the present invention and are not intended to limit the technical scope of the present invention.

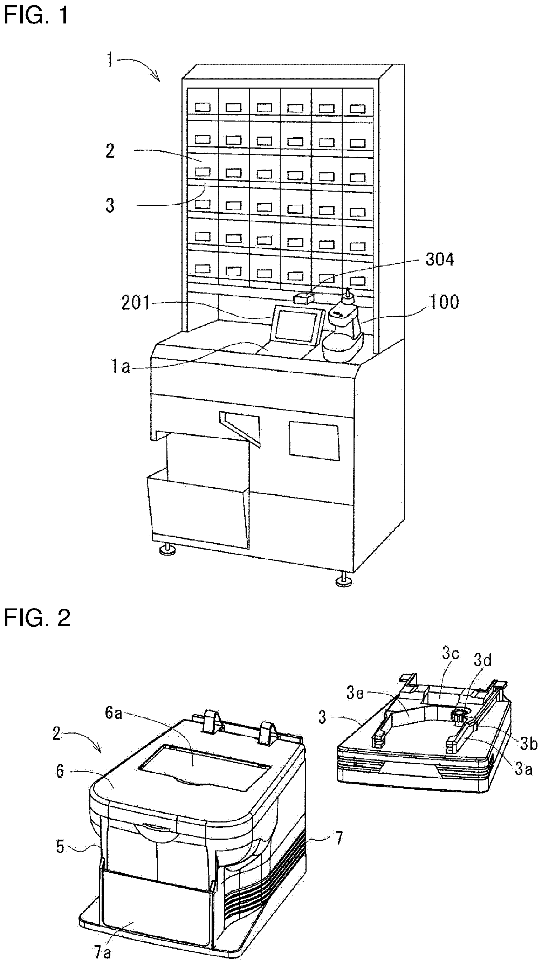

As shown in FIG. 1, a medicine dispensing device 1 according to this embodiment includes a plurality of bases 3, a plurality of medicine cassettes 2 detachable from each of the bases 3, a code reading part 304 and an adjusting device 100.

The medicine dispensing device 1 dispenses tablets to be dispensed from one or more medicine cassettes 2 attached to the medicine dispensing device 1 based on medicine dispensing data such as prescription data input from a host system, and for example, it is possible to perform a medicine dispensing operation to package medicines in corresponding medicine wrapping papers for each predetermined unit such as the timing of taking medicine. The prescription data is an example of medicine dispensing data, and the medicine dispensing device 1 may execute the medicine dispensing operation based on packaging data generated based on the prescription data.

The medicine cassette 2 is an example of a medicine dispensing part that can be adjusted to a plurality of states capable of dispensing different types of tablets. Specifically, the medicine cassette 2 is provided with an adjusting part, which will be described later, for adjusting a tablet dispensing path (tablet guide path 8b, which will be described later) in the medicine cassette 2 to the plurality of states capable of dispensing different types of tablets. In the medicine cassette 2, the size of the dispensing path is adjusted by the adjusting unit, so that the medicine cassette 2 can be displaced into the plurality of states capable of dispensing different types of tablets. The adjusting device 100 is used for manual adjustment work in which a user manually adjusts the state of the medicine cassette 2 by operating the adjusting part of the medicine cassette 2 of the medicine dispensing device 1. In addition, the tablet in this embodiment is an example of the medicine in the present invention.

However, the medicine to be dispensed cannot be properly dispensed from the medicine cassette 2 unless the user has adjusted the adjusting part correctly in advance according to the type of medicine to be dispensed. On the other hand, in a medicine dispensing system 500 according to the present embodiment, it is possible to support the dispensing of medicines using the medicine cassettes 2 capable of dispensing different types of medicines.

### <Structure of medicine cassette>

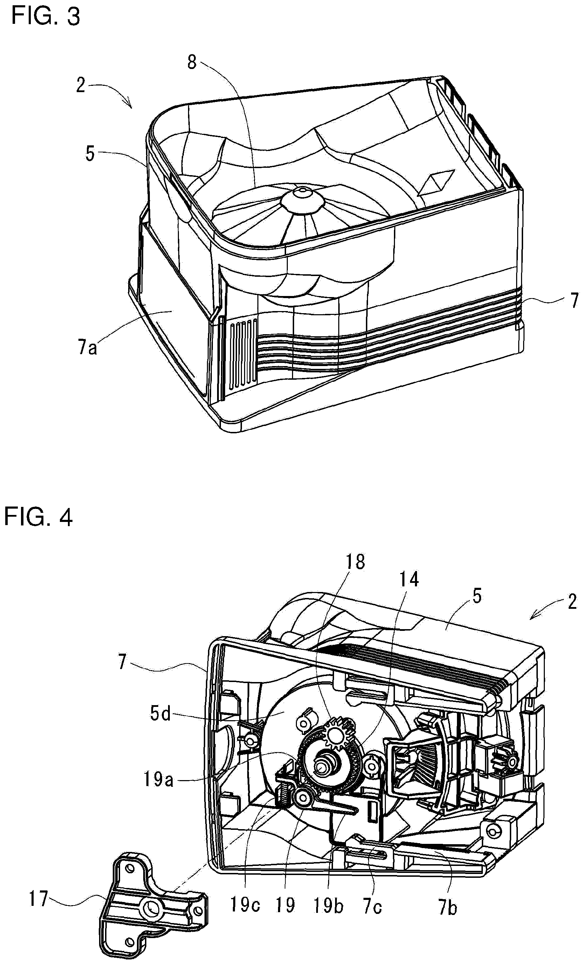

FIG. 2 shows the medicine cassette 2 and the base 3. The medicine cassette 2 includes a cassette body 5, a lid 6 that opens and closes and detachably covers an upper opening of the cassette body 5, a skirt part 7 provided at a bottom of the cassette body 5, and a rotor 8 accommodated in the cassette body 5 as shown in FIG. 3.

Further, an RFID tag 206 is provided at the bottom of the cassette body 5. Note that, in this embodiment, the RFID tag 206 is an example of a storage part according to the present invention. On the other hand, the base 3 is provided with a data reading part 306 at a position where information can be read from the RFID tag 206 of the medicine cassette 2 when the medicine cassette 2 is attached.

An upper surface of the lid 6 and a front surface of the skirt part 7 are formed with pockets 6a and 7a for accommodating labels or cards capable of identifying the tablets stored in the medicine cassette 2. As shown in FIG. 4, an inner surface of the skirt part 7 is provided with a sliding portion 7b that slides on a mounting guide 3a of the base 3 shown in FIG. 2 and an elastic engaging piece 7c that engages with an engaging portion 3b of the mounting guide 3a.

### <Structure of cassette body>

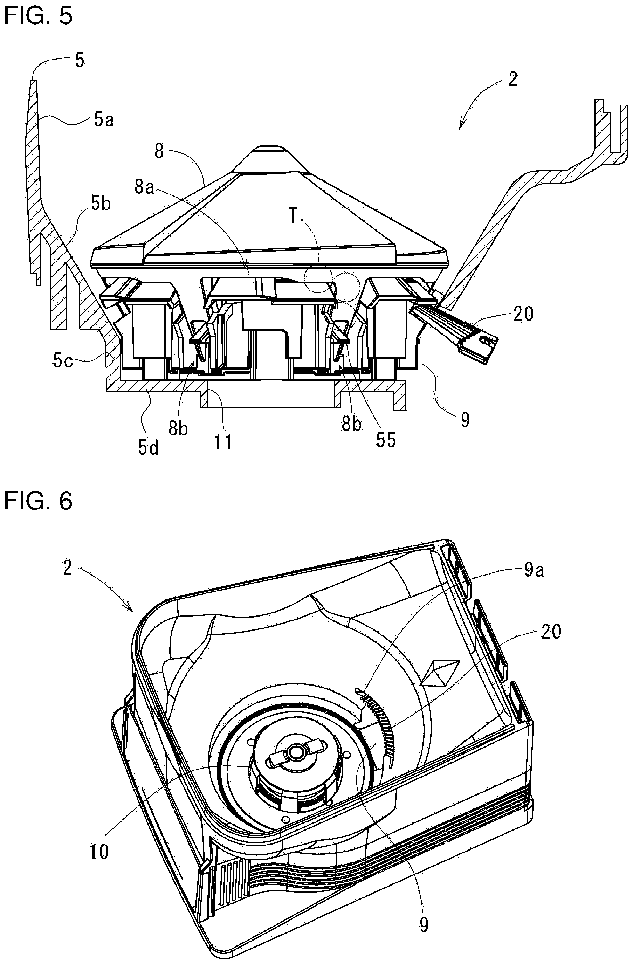

As shown in FIG. 5, the cassette body 5 includes a rectangular upper portion 5a opening upward, an inverted conical inclined portion 5b, a cylindrical tubular portion 5c, and a bottom portion 5d. A rotor 8 is accommodated in an internal space from the bottom portion 5d to the inclined portion 5b, and a large number of tablets T can be stored above the rotor 8. A tablet discharge hole 9 is formed from a lower portion of the inclined portion 5b to the bottom portion 5d. The tablet discharge hole 9 communicates with a tablet discharge path 3c formed in the base 3 shown in FIG. 2. A partition member 20 and a partition adjusting mechanism M1 for adjusting a position of the partition member 20, which will be described later, are attached to an outside of the cassette body 5. A tip of the partition member 20 is inserted inside from an outside of the inclined portion 5b through a slit 9a shown in FIG. 6 formed above the tablet discharge hole 9. A rotor shaft hole 11 is formed in a center of the bottom portion 5d to accommodate the rotor driving part 10 shown in FIG. 6.

### <Rotor driving part>

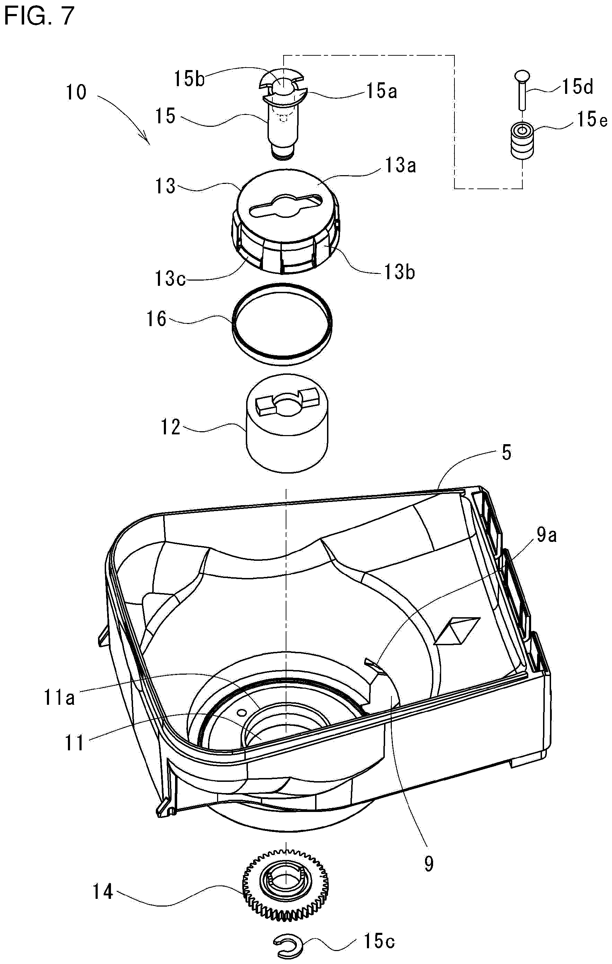

As shown in FIG. 7, the rotor driving part 10 includes a drive shaft 12 passing through a rotor shaft hole 11, an engagement shaft 13 that engages with an upper end of the drive shaft 12 and rotates integrally with the drive shaft 12, a drive gear 14 that engages with a lower end of the drive shaft 12 and rotates integrally with the drive shaft 12, and a central shaft 15 passing through the engagement shaft 13, the drive shaft 12 and the drive gear 14 to integrate them. The engagement shaft 13 includes a circular base portion 13a that contacts an upper end surface of the drive shaft 12, engagement pieces 13b protruding downward from an outer peripheral edge of the base portion 13a and located at six equidistant positions around the circumference thereof, and a connecting portion 13c that connects lower ends of the adjacent engaging pieces 13b. Inner surfaces of the engaging piece 13b and the connecting portion 13c are slidably provided on an outer peripheral surface of an annular projection 11a provided on an edge of the rotor shaft hole 11 via a ring 16.

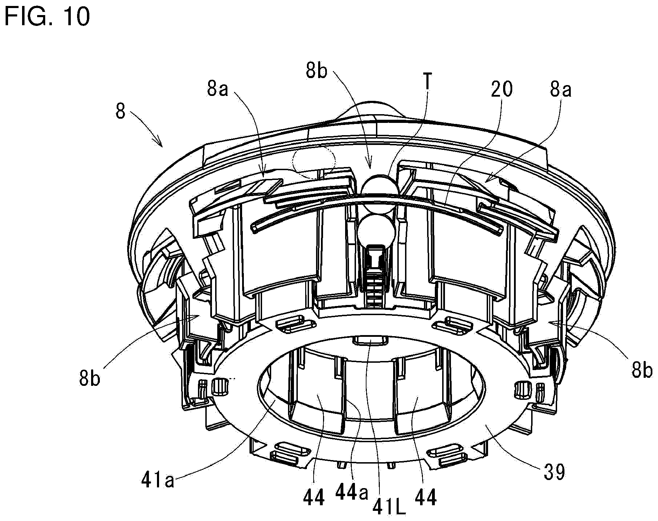

When the rotor 8 is mounted on the engaging piece 13b, the engaging piece 13b engages with slits 44a between the engaging pieces 44 of the engaging recess 41a of the rotor 8 shown in FIG. 10 to transmit the rotational force of the rotor driving part 10 to the rotor 8. Although the rotor 8 has various sizes, in order to prevent erroneous attachment of the rotor 8 to the rotor driving portion 10 of the cassette main body 5, the number of the engaging pieces 13b and the slits 44a may be six, five, or four for each size of the rotor 8.

A collar 15a and a hole 15b are formed at an upper end of the central shaft 15. Three stacked annular magnets 15c are inserted into the hole 15b of the central shaft and fixed with screws 15d. The magnets 15c may be a single cylindrical magnet. The lower end of the central shaft 15 passes through a gear cover 17 attached to the bottom portion 5d of the cassette body 5 shown in FIG. 4 and is retained by a C-shaped retaining ring 15e. The drive gear 14 is driven by being engaged with a motor gear 3d of the base 3 shown in FIG. 2 via an intermediate gear 18 shown in FIG. 4. The motor gear 3d is connected to a motor driven when dispensing tablets from the medicine cassette 2 attached to the base 3, and the tablet is dispensed from the medicine cassette 2 by transmitting a driving force of the motor from the motor gear 3d to the driving gear 14.

As shown in FIG. 4, the drive gear 14 is engaged with an engaging claw 19a at one end of an engaging lever 19 provided on the bottom surface of the cassette body 5. An operating portion 19b at the other end of the engaging lever 19 extends in a mounting direction of the medicine cassette 2. When the medicine cassette 2 is mounted on the base 3, the operating portion 19b of the engaging lever 19 comes into contact with a predetermined contact portion 3e of the base 3 shown in FIG. 2 to rotate the engaging lever 19 against biasing force of a spring 19c. As a result, the engaging claw 19a is disengaged from the drive gear 14, and the drive gear 14 can be driven to rotate. Further, when the medicine cassette 2 is pulled out from the base 3, the operating portion 19b of the engaging lever 19 is separated from the contact portion 3e of the base 3, the engaging lever 19 is rotated by the biasing force of the spring 19c, the engaging claw 19a is engaged with the drive gear 14, and the rotation of the drive gear 14 is prevented. As a result, it is possible to prevent the tablets T from dropping due to unexpected rotation of the rotor 8 of the medicine cassette 2 that has been pulled out.

### <Partition adjusting mechanism>

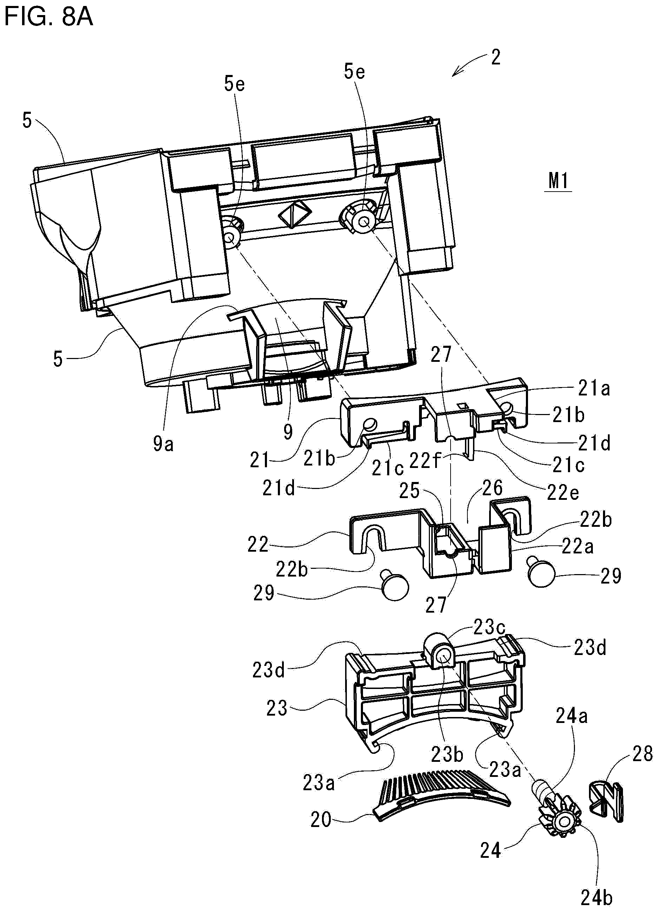

As shown in FIG. 8A, the partition member 20 is formed in a comb shape that is curved upward. The partition member 20 is movable forward and backward with respect to the rotor 8 by the partition adjusting mechanism M1. The partition adjusting mechanism M1 includes a first fixing member 21, a second fixing member 22, a movable member 23, and a partition adjusting member 24.

At a center of the first fixing member 21, an upper case portion 21a in which a slide portion 23c of the movable member 23 and a stopper 28 are accommodated is formed. Mounting holes 21b are formed on both sides of the upper case portion 21a of the first fixing member 21. A pair of elastic pieces 21c are formed on a lower surface of the first fixing member 21 to press and stabilize the movable member 23. Protrusions 21d that engage with grooves 23d of the movable member 23 is formed at a tip of the elastic pieces 21c.

At a center of the second fixing member 22, a lower case portion 22a in which the slide portion 23c of the movable member 23 and the stopper 28 are accommodated is formed. Inverted U-shaped cutouts 22b are formed in both lower edges of the lower case portion 22a of the second fixing member 22. When the upper case portion 21a of the first fixing member 21 and the lower case portion 22a of the second fixing member 22 are combined with each other, a downwardly opening movable member accommodating portion 25 for accommodating the slide portion 23c of the movable member 23 and a stopper accommodating portion 26 for accommodating a stopper 28 are formed. In a lower edge of the upper case portion 21a and an upper edge of the lower case portion 22a of the movable member accommodating portion 25, semicircular notches 27 are formed to support both ends of the partition adjusting member 24 in the axial direction so as not to move in the axial direction.

The movable member 23 has holding portions 23a for holding the partition member 20 at its lower end and the sliding portion 23c having a screw hole 23b at its upper end. The grooves 23d are formed at both ends of the upper surface of the movable member 23.

The partition adjusting member 24 has a threaded portion 24a screwed into the screw hole 23b of the slide portion 23c of the movable member 23 and an engagement gear 24b. A stopper 28 is engaged with the engagement gear 24b so that it can be fixed at a desired position. The movable member 23 is fixed in the rotational direction of the partition adjusting member 24 by engaging the grooves 23d of the movable member 23 with the projections 21d of the first fixing member 21. Therefore, when the partition adjusting member 24 rotates, the movable member 23 moves in the axial direction of the partition adjusting member 24.

In order to assemble the partition adjusting mechanism M1, first, the slide portion 23c of the movable member 23 is accommodated in the lower case portion 22a of the second fixing member 22 from below, and the partition adjusting member 24 is screwed into and penetrates the slide portion 23c. After that, both ends of the partition adjusting member 24 are placed in the notch 27 of the lower case portion 22. Further, the stopper 28 is accommodated in the lower case portion 22a of the second fixing member 22. In this state, when the first fixing member 21 is superimposed on the second fixing member 22 so that the partition adjusting member 24 passes through the hole formed by the notches 27 of the first fixing member 21 and the second fixing member, a claw 22f of an elastic engaging piece 22e provided on the upper case portion 21a is engaged with the lower edge of the lower case portion 22a to be integrally assembled. Further, since a size of the notch 27 is smaller than that of the engagement gear 24b, the engagement gear 24b is fixed to the upper case portion 22a and the lower case portion 22b so as not to move in the axial direction. Subsequently, fixing screws 29 are passed through the notch 22b of the second fixing member 22 and the mounting holes 21b of the first fixing member 21 and screwed into a screw hole 5e on the rear surface of the cassette body 5, thereby fixing to the cassette body 5.

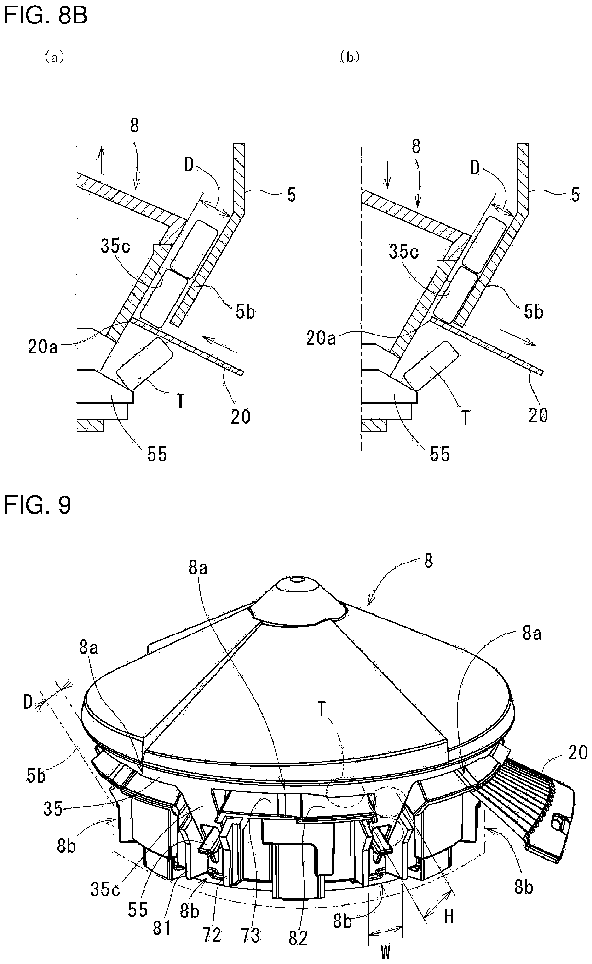

When the partition adjusting member 24 of the partition adjusting mechanism M1 is rotated, the sliding portion 23c moves within the movable member accommodating portion 25 of the upper case portion 21a of the first fixing member 21 and the lower case portion 22b of the second fixing member 22. Therefore, as shown in FIG. 8B, the partition member 20 held by the movable member 23 advances or retreats toward the rotor 8 in the cassette body 5, and a tip position 20a of the partition member 20 can be adjusted. That is, as shown in FIG. 8B(a), when the tablet T is thick, as will be described later in detail, a rotor body 31 of the rotor 8 is elevated to increase a depth D of the groove of the tablet guide path 8b between a lower inclined outer surface 35c and the inclined portion 5b of the cassette body 5, and along with this, the tip of the partition member 20 is also advanced toward the rotor 8. As shown in FIG. 8B(b), when the tablet T is thin, the rotor body 31 of the rotor 8 is lowered to reduce the depth D of the groove of the tablet guide path 8b between the lower inclined outer surface 35c and the inclined portion 5b of the cassette body 5, and along with this, the tip of the partition member 20 is also retracted from the rotor 8.

### <Overall Structure of Rotor>

As shown in FIGs. 9 and 10, the rotor 8 generally has a conical top surface, an inverted conical side surface, and a flat bottom surface. A tablet pocket 8a is provided in the upper side surface of the rotor 8 in the circumferential direction, and a plurality of tablet guide paths 8b extending downward from the tablet pocket 8a are provided at regular intervals in the circumferential direction.

The tablet pocket 8a is formed by an outer peripheral surface of the rotor body 31, which will be described later, and a first horizontal projecting piece 73 of a first movable member 60 and a second horizontal projecting piece 82 of a second movable member 61, which will be described later. Further, he tablet pocket 8a is surrounded by the inclined portion 5b of the cassette body 5, receives the tablets T accommodated in the cassette body 5, and aligns them in the circumferential direction.

The tablet guide path 8b is formed in a groove shape by the lower inclined outer surface 35c of the rotor body 31 to be described later, first vertical projecting pieces 72 of the first movable member 60 to be described later, second vertical projecting pieces 81 of the second movable member 61 to be described later and a tablet support table 55 of an annular elevating member 51 to be described later. Further, the tablet guide path 8b is covered with the inclined portion 5b of the cassette body 5 and receives and guides downward the tablets T aligned in the tablet pockets 8a.

The tablet guide path 8b is required to adjust the depth, height and width of the groove according to the shape or size of the tablets accommodated in the medicine cassette 2 and to allow the tablet to smoothly pass through the tablet guide path 8b and be discharged from the tablet discharge hole 9 shown in FIG. 5. That is, the medicine cassette 2 can dispense any type of tablet by adjusting the tablet guide path 8b of the rotor 8 provided in the medicine cassette 2.

The "depth" of the groove of the tablet guide path 8b is the dimension in the thickness direction of the tablet passing through the tablet guide path 8b and is the dimension D between the inclined portion 5b of the cassette body 5 and the lower inclined outer surface 35c of a downward protrusion 35 of the rotor body 31 (See FIGs. 8B and 9). The "height" of the groove of the tablet guide path 8b is the dimension in the height direction of the tablet passing through the tablet guide path 8b and is the dimension H between the partition member 20 and the tablet support table 55 of the annular elevating member 51 of the rotor 8 (See FIG. 9). The "width" of the groove of the tablet guide path 8b is the dimension in the width direction of the tablet passing through the tablet guide path 8b and is the dimension W between the first vertical projecting piece 72 of the first movable member 60 and the second vertical projecting piece 81 of the second movable member 61 (See FIG. 9).

The rotor 8 has an adjusting part including a depth adjusting mechanism M2, a height adjusting mechanism M3, a width adjusting mechanism M4 to adjust the groove shape of the tablet guide path 8b or the like. The adjusting part can be operated by the user to adjust the rotor 8 of the medicine cassette 2 to the plurality of states capable of dispensing different types of medicine. These will be described in order below.

### <Depth adjusting mechanism>

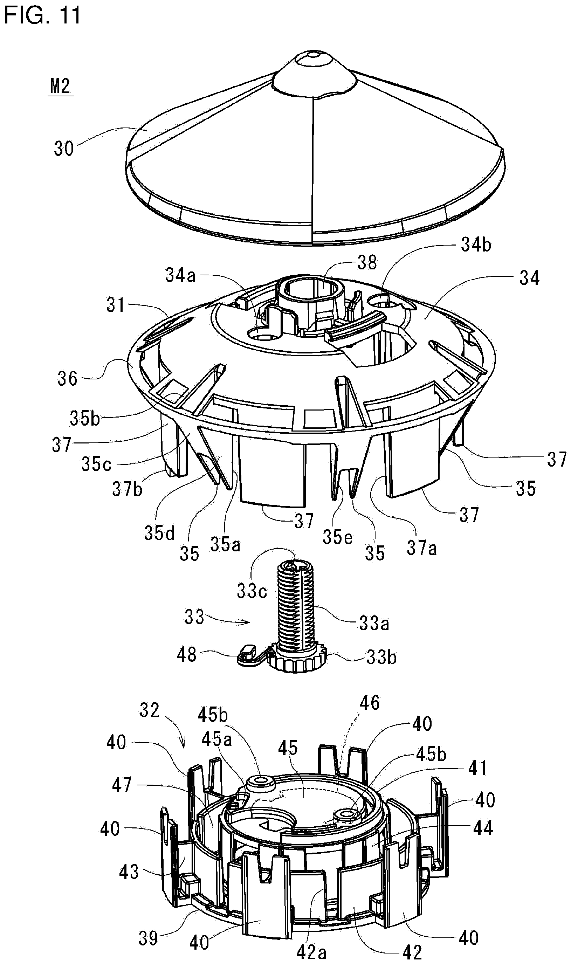

FIG. 11 shows members constituting the depth adjusting mechanism M2. The depth adjusting mechanism M2 includes a rotor cover 30, the rotor body 31, a rotor base 32 and a depth adjusting member 33.

The rotor cover 30 has an overall umbrella shape. An upper surface of rotor cover 30 is formed in a conical shape.

The rotor body 31 has a circular base portion 34, downward protrusions 35, an annular portion 36 and guide portions 37.

A shaft portion 38 is provided in a center of the base portion 34 and a threaded hole (not shown) is formed in the shaft portion 38. Two holes 34a and 34b are formed in an upper surface of the base portion 34 to expose a height adjusting member 52 and a width adjusting member 64, which will be described later.

The downward protrusions 35 extend downward from six equidistant positions on an outer peripheral edge of the base portion 34. The downward protrusion 35 includes a vertical inner surface 35a, an upper inclined outer surface 35b that slopes downward from the outer peripheral edge of the base portion 34, and a lower inclined outer surface 35c that slopes downward and inward from a lower end of the upper sloped outer surface 35b and both side surfaces 35d and is formed in a triangular shape when viewed from the side. The lower inclined outer surface 35c forms a bottom surface of the groove of the tablet guide path 8b. A slit 35e is formed at the lower end of the downward protrusion 35 .

The annular portion 36 is concentrically formed on an outside of the base portion 34 and connected to the base portion 34 via the downward protrusions 35.

The guide portions 37 extend downward from six equidistant positions on the outer peripheral edge of the base portion 34 between the downward protrusions 35. On both sides of an inner surface of the guide portion 37, guide edges 37a with which guide pieces 40 of the rotor base 32, which will be described later, are slidably engaged are formed. The rotor body 31 and the rotor base 32 are integrally rotated by the engagement between the guide piece 40 and the guide edge 37a. At a lower end of any one of the six guide portions 37, a protrusion 37b serving as a detection portion for detecting a zero point is formed.

The rotor base 32 has an annular base portion 39, the guide pieces 40 and engaging portion 41.

An annular wall 42 is formed on an upper surface of the base portion 39. Vertical slits 42a extending in the axial direction are formed in the annular wall 42 at six equidistant positions around the around the circumference of the annular wall 42.

The guide pieces 40 protrude upward between the adjacent vertical slits 42a at six equidistant positions around the outer peripheral edge of the base portion 39. The guide piece 40 is formed so as to be slidably engaged with the guide edge 37a of the guide portion 37 of the rotor body 31. Reinforcing ribs 43 are provided between the guide pieces 40 and the annular wall 42.

The engaging portion 41 has an engaging pieces 44 that rise upward from six equidistant positions around the inner peripheral edge of the base portion 39 and a circular protrusion 45 that is provided at upper ends of the engaging pieces 44. The engaging portion 41 forms an engaging concave portion 41a with which the rotor driving part 10 engages as shown in FIG. 10 when viewed from the rear side. The engaging pieces 13b of the rotor driving part 10 are engaged with the slits 44a between the adjacent engaging pieces 44. A magnetic plate 46 that is attracted to the magnets 15c provided on the central shaft 15 of the rotor drive part 10 is embedded in the circular protrusion 45. the depth adjusting member 33 is supported at a center of the upper surface of the circular protrusion 45. The circular protrusion 45 is formed with a hole 45a for accommodating a stopper 48 that prevents free rotation of the depth adjusting member 33 and two screw holes 45b into which screws (not shown) inserted through two screw insertion holes 93 of a second support member 63, which will be described later, are screwed.

An annular concave portion 47 is formed between the circular protrusion 45 and the annular wall 42 to accommodate a height adjusting mechanism M3, which will be described later.

The depth adjusting member 33 has a male screw portion 33a and a lower end gear portion 33b. The male screw portion 33a is screwed into a threaded hole (not shown) of the shaft portion 38 of the rotor body 31, and the gear portion 33b at the lower end is supported by the circular protrusion 45 of the rotor base 32. An engaging portion 33c is formed at an upper end of the male threaded portion 33a, which protrudes and is exposed from the shaft portion 38 of the rotor body 31, so that the rotation can be adjusted from the outside. A tip of the stopper 48 made of an elastic piece is engaged between the teeth of the gear portion 33b.

In the depth adjusting member 33, the axial movement of the gear portion 33b is restrained by a first support member 62 and the rotor base 32, and the rotation of the rotor body 31 with respect to the rotor base 32 is restrained by engaging the guide edges 37a of the rotor body 31 with the guide pieces 40 of the rotor base 32. In this way, when the depth adjusting member 33 is rotated in a state that the rotor base 32 is not rotating, the rotor body 31 having a threaded hole (not shown) that is screwed with the male threaded portion 33a of the depth adjusting member 33 is elevated or lowered in the rotation axis direction of the rotor 8. Along with this, the lower inclined outer surface 35c of the downward protrusion 35 of the rotor body 31, which forms the bottom surface of the tablet guide path 8b, is also elevated or lowered.

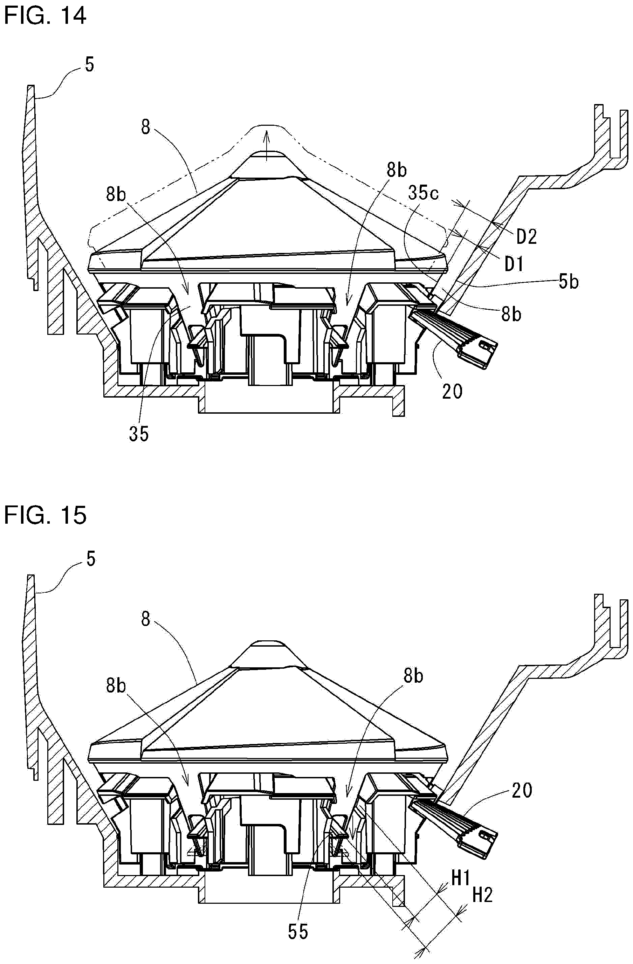

Referring to FIG. 14, the lower inclined outer surface 35c of the downward protrusion 35 is radially inclined from the outside to the inside from top to bottom and is parallel to the inverted conical inclined portion 5b of the cassette body 5. Therefore, when the lower inclined outer surface 35c of the downward protrusion 35 of the rotor body 31 is lowered, a distance between the lower inclined outer surface 35c of the downward protrusion 35 and the conical inclined portion 5b of the cassette body 5 is reduced, and the depth of the tablet guide path 8b can be made shallow (D1). Conversely, when the lower inclined outer surface 35c of the downward protrusion 35 of the rotor body 31 is elevated, the distance between the lower inclined outer surface 35c of the downward protrusion 35 and the inverted conical inclined portion 5b of the cassette body 5 is increased, and the depth of the tablet guide path 8b can be increased (D2). By rotating the depth adjusting member 33 to the left or right in this manner, the depth of the tablet guide path 8b can be adjusted according to the thickness of the tablet T passing through the tablet guide path 8b. Each time the gear portion 33b of the depth adjusting member 33 shown in FIG. 11 rotates, the tip of the stopper 48 climbs over the teeth of the gear portion 33b and engages between the teeth. Therefore, the depth adjusting member 33 can be stopped at an appropriate position to fix the rotor body 31 at a desired height position.

### <Height adjustment mechanism>

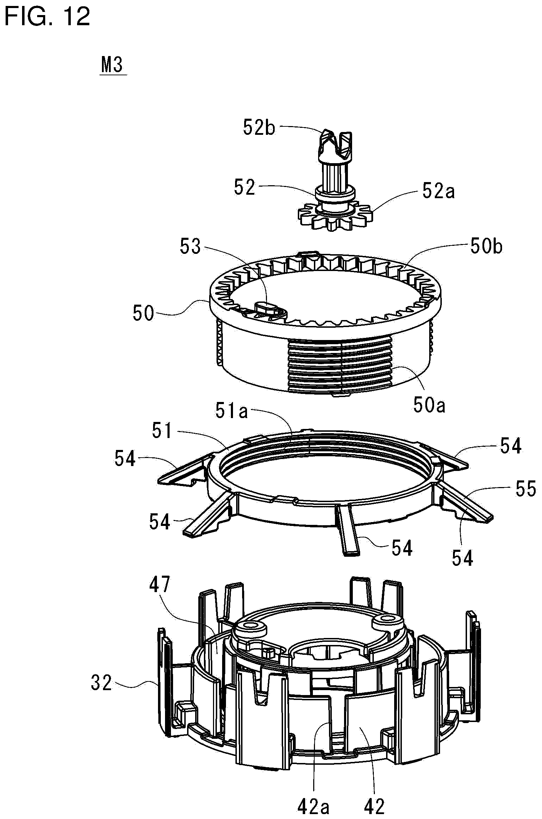

FIG. 12 shows members constituting the height adjusting mechanism M3. The height adjusting mechanism M3 includes a cylindrical rotating member 50, an annular elevating member 51, and a height adjusting member 52.

The cylindrical rotating member 50 has a male threaded portion 50a formed on a lower outer circumference thereof and a driven gear 50b formed on an upper inner circumference thereof. A stopper 53 for preventing free rotation of the cylindrical rotating member 50 is engaged with the driven gear 50b.

The annular elevating member 51 has arms 54 protruding radially at six equidistant positions on the outer periphery thereof and a tablet support table 55 is formed at a tip of each arm 54. The tablet support table 55 is inclined perpendicular to the tablet guide path 8b so as to support the lowermost tablet T in the tablet guide path 8b. A female threaded portion 51a is formed on the inner surface of the annular elevating member 51 so as to be screwed with the male threaded portion 50a of the cylindrical rotating member 50.

The height adjusting member 52 has a drive gear 52a that meshes with the driven gear 50b of the cylindrical rotating member 50 at a lower end of the height adjusting member 52. An engaging portion 52b is formed at an upper end of the height adjusting member 52, which protrudes and is exposed from the holes 34a in the upper surface of the base portion 34 of the rotor body 31, so that the rotation can be adjusted from the outside. The height adjusting member 52 is held by an edge of a hole 90 of the second support member 63 to be described later so as not to move vertically.

The cylindrical rotating member 50 and the annular elevating member 51 are accommodated in the annular concave portion 47 of the rotor base 32 while being screwed together. The arm 54 of the annular elevating member 51 is slidably fitted into the vertical slit 42a of the annular wall 42 of the rotor base 32, and the tablet support table 55 protrudes outside the annular wall 42 of the rotor base 32 and supports the lowermost tablet T in the tablet guide path 8b.

As shown in FIG. 15, in order to adjust the height H of the tablet guide path 8b corresponding to the height of the tablet T, the height adjusting member 52 of the height adjusting mechanism M3 is rotated left or right. In the present invention, since the partition member 20 is fixed to the cassette body 5 in the height direction, in order to adjust the height H of the tablet guide path 8b, the tablet support table 55 below the partition member 20 is elevated and lowered to adjust the distance between the partition member 20 and the tablet support table 55 instead of moving the partition member 20 itself. Thereby, the height H of the partition member 20 from the tablet support table 55 of the tablet guide path 8b is adjusted.

When the height adjusting member 52 is rotated, the cylindrical rotating member 50 is rotated. The vertical movement of the cylindrical rotating member 50 is restrained by the second support member 63 and the rotor base 32. The annular elevating member 51 having the female threaded portion 51a screwed into the male threaded portion 50a of the cylindrical rotating member 50 has the arms 54 passing through the vertical slits 42a in the annular wall 42 of the rotor base 32, and the rotation of the annular elevating member 51 is restrained. Therefore, the rotation of the cylindrical rotating member 50 elevates or lowers the annular elevating member 51, and the tablet support table 55 of the annular elevating member 51 is elevated or lowered.

That is, as shown in FIG. 15, when the cylindrical rotating member 50 rotates in one direction, the tablet support table 55 of the annular elevating member 51 is elevated, and the position of the partition member 20 relative to the tablet support table 55, that is, the height is lowered (H1). Conversely, when the cylindrical rotating member 50 rotates in the other direction, the tablet support table 55 of the annular elevating member 51 is lowered, and the position of the partition member 20 relative to the tablet support table 55, that is, the height becomes higher (H2). Each time the cylindrical rotating member 50 rotates due to the rotation of the height adjusting member 52, the tip of the stopper 53 climbs over the teeth of the driven gear 50b of the cylindrical rotating member 50 and engages between the teeth. Thus, by stopping the height adjusting member 52 at an appropriate position, the tablet support table 55 can be fixed at a desired height position.

### <Width adjusting mechanism>

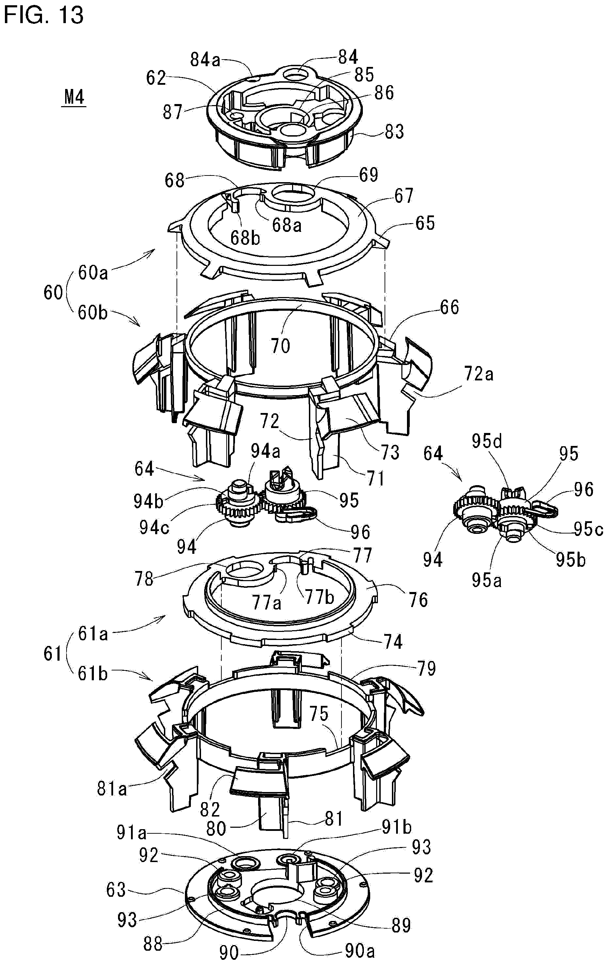

FIG. 13 shows members constituting the width adjusting mechanism M4. The width adjusting mechanism M4 includes the first movable member 60, the second movable member 61, the first support member 62, the second support member 63 and a width adjusting member 64.

As shown in FIG. 13, the first movable member 60 consists of an upper member 60a and a lower member 60b, and engagement protrusions 65 of the upper member 60a and the engagement protrusions 66 of the lower member 60b are engaged with each other so that they can rotate integrally.

In the upper member 60a of the first movable member 60, a substantially semicircular notch 68 and an elongated hole 69 are formed adjacent to each other on an inner circumference of an annular base portion 67. An A projection 68a and a B projection 68b, which face each other in the circumferential direction of the first movable member 60, are formed on edges of the notch 68 facing the center of the notch 68 when viewing the first movable member 60 from above. The A projection 68a and the B projection 68b serve as a cam follower that are in sliding contact with an A cam 94a and a B cam 94b of a first adjusting shaft 94, which will be described later.

The lower member 60b of the first movable member 60 has an annular base portion 70, six wall portions 71, first vertical projecting pieces 72 and first horizontal projecting pieces 73. The six wall portions 71 protrude downward from an outer peripheral edge of the base portion 70 at six equidistant positions. The first vertical protruding pieces 72 protrude outward from a left end of the wall portion 71 when viewed from the front and forms the right side surface of the tablet guide path 8b. The first vertical projecting pieces 72 are formed with a notch 72a into which the partition member 20 is fitted. The first horizontal protruding pieces 73 extend horizontally in the circumferential direction from an upper end of the first vertical protruding pieces 72 toward a right side when viewed from the front and form the bottom surface of the aforementioned tablet pocket 8a.

The second movable member 61, like the first movable member 60, consists of an upper member 61a and a lower member 61b, and engagement protrusions 74 of the upper member 61a and engagement concave portions 75 of the lower member 61b are engaged with each other so that they can rotate integrally.

In the upper member 61a of the second movable member 61, a substantially semicircular notch 77 and an elongated hole 69 are formed adjacent to each other on an inner circumference of an annular base portion 77. An A projection 77a and a B projection 77b, which face each other in the circumferential direction of the second movable member 61, are formed on edges of the notch 77 facing the center of the notch 77 when viewing the second movable member 60 from above. The A projection 77a and the B projection 77b serve as a cam follower that are in sliding contact with an A cam 95a and a B cam 95b of a second adjusting shaft 95, which will be described later.

The lower member 61b of the second movable member 61 has an annular base portion 79, six wall portions 80, second vertical projecting pieces 81 and second horizontal projecting pieces 82. The six wall portions 80 protrude downward from an outer peripheral edge of the base portion 79 at six equidistant positions. The second vertical protruding pieces 81 protrude outward from a right end of the wall portion 80 when viewed from the front and forms the left side surface of the tablet guide path 8b. The second vertical projecting pieces 81 are formed with a notch 81a into which the partition member 20 is fitted. The second horizontal protruding pieces 82 extend horizontally in the circumferential direction from an upper end of the second vertical protruding pieces 81 toward a left side when viewed from the front and form the bottom surface of the tablet pocket 8a described above together with the first horizontal projecting pieces 73 of the first movable member 60. A tip of the second horizontal projecting piece 82 of the second movable member 61 is formed so as to overlap a tip of the first horizontal projecting piece 73 of the first movable member 60.

The first support member 62 has a circular shape with an outer diameter larger than the inner diameter of the upper member 60a of the first movable member 60 and has a circular protrusion 83 on its lower surface. At a center of the first support member 62, holes 84 and 84a through which the width adjusting member 64 (to be described later) pass, a hole 85 through which the depth adjusting member 33 of the depth adjusting mechanism M2 passes, a hole 86 through which the height adjusting member 52 of the height adjusting mechanism M3 passes, and two screw holes 87 are formed.

The second support member 63 has a circular shape with an outer diameter larger than the inner diameter of the upper member 60a of the first movable member 60 and has an annular protrusion 88 formed on its upper surface into which the circular protrusion 83 of the first support member 62 is fitted. At a center of the second support member 63, a hole 89 through which the depth adjusting member 33 of the depth adjusting mechanism M2 passes, a hole 90 and a notch 90a through which the height adjusting member 52 of the height adjusting mechanism M3 pass, a hole 91a through which the first adjusting shaft 94 of the width adjusting member 64, which will be described later, passes, a hole 91b into which second adjustment shaft 95 is fitted, two screw holes 92 into which screws (not shown) inserted through the two screw insertion holes 87 of the first support member 62 are screwed, and two screw holes 93 are formed.

By inserting a screw (not shown) from the screw insertion hole 87 of the first support member 62 into the screw hole 92 of the second support member 63 and tightening it, the first support member 62 and the second support member 63 are integrated with the first movable member 60 and the second movable member 61 sandwiched therebetween.

Further, by inserting a screw (not shown) from the screw insertion hole 93 of the second support member 63 into the screw hole 45b of the rotor base 32 and tightening it, the second support member 63 is fixed to the rotor base 32, and the cylindrical rotating member 50 of the height adjusting mechanism M3 is held between the second support member 63 and the rotor base 32 and axial movement thereof is restrained.

The width adjusting member 64 is composed of the first adjusting shaft 94 and the second adjusting shaft 95. The first adjusting shaft 94 is arranged within the notch 68. The second adjusting shaft 95 is arranged inside the elongated hole 69. The second adjusting shaft 95 is provided with a stopper 96 that prevents the width adjusting member 64 from freely rotating.

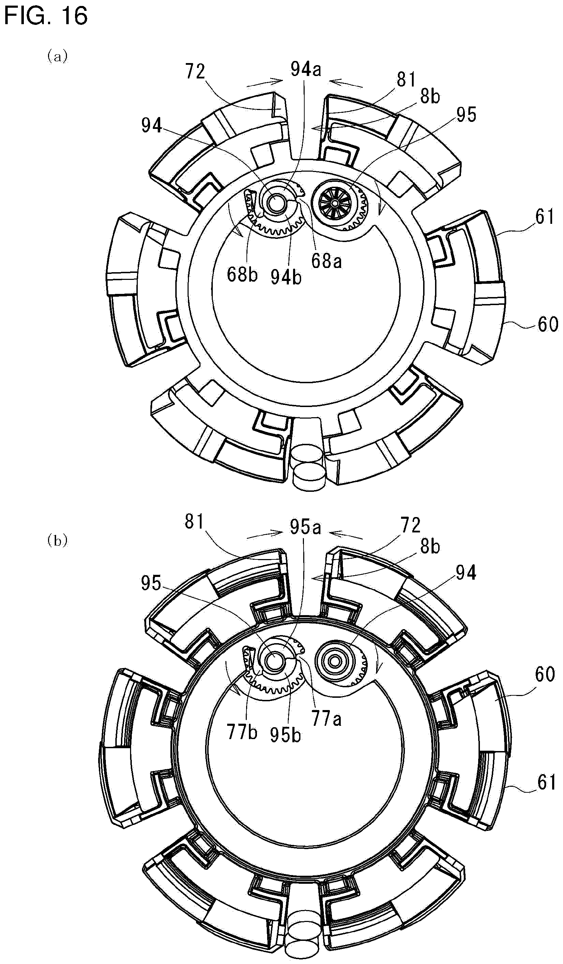

The first adjusting shaft 94 is formed with the A cam 94a, the B cam 94b and a gear 94c in order from the upper end. As shown in FIG. 16, the A cam 94a is formed so that the radius of the cam surface increases within a range of 360° clockwise when the width adjusting member 64 is viewed from above and is in sliding contact with the A projection 68a of the first movable member 60. The B cam 94b is formed so that the radius of the cam surface increases within a range of 360° counterclockwise when the width adjusting member 64 is viewed from above and is in sliding contact with the B projection 68b of the first movable member 60. The maximum radius portion of the A cam 94a and the maximum radius portion of the B cam 94b are located 180 degrees apart. The upper end of the first adjusting shaft 94 is supported by a hole 84a of the first support member 62 and the lower end is supported by a hole 91a of the second support member 63.

Similarly, the second adjusting shaft 95 is formed with the A cam 95a, the B cam 95b, a gear 95c and an engaging portion 95d in order from the lower end. The A cam 95a is formed so that the radius of the cam surface increases within a range of 360° clockwise when the width adjusting member 64 is viewed from below and is in sliding contact with the A projection 77a of the second movable member 61. The B cam 95b is formed so that the radius of the cam surface increases within a range of 360° counterclockwise when the width adjusting member 64 is viewed from below and is in sliding contact with the B projection 77b of the second movable member 61. The maximum radius portion of the A cam 95a and the maximum radius portion of the B cam 95b are located 180 degrees apart. The gear 95c of the second adjusting shaft 95 is configured to mesh with and interlock with the gear 94c. The upper end of the second adjusting shaft 95 passes through the elongated hole 69 of the first support member 62, protrudes from the rotor body 31 and is exposed through the hole 34a, so that the rotation can be adjusted from the outside. The lower end of the second adjusting shaft 95 is supported in the hole 91b of the second support member 63. The upper end of the first adjusting shaft 94 may pass through the first support member 62 and protrude from the rotor body 31 to be exposed so that the rotation can be adjusted from the outside.

When the second adjusting shaft 95 is rotated clockwise in FIG. 16(a), the rotational force is transmitted from the gear 95c of the second adjusting shaft 95 to the gear 94c of the first adjustment shaft 94 and the first adjusting shaft 94 rotates counterclockwise. Since the A cam 94a of the first adjusting shaft 94 slides against and presses the A projection 68a of the first movable member 60 due to the rotation of the first adjusting shaft 94, the first movable member 60 rotates clockwise in FIG. 16(a). On the other hand, since the A cam 95a of the second adjusting shaft 95 slides against and presses the A projection 77a of the second movable member 61 due to the rotation of the second adjusting shaft 95 as shown in FIG. 16(b), the second movable member 61 rotates clockwise in FIG. 16(b) and counterclockwise in FIG. 16(a).

Subsequently, when the second adjusting shaft 95 is rotated counterclockwise in FIG. 16(a), the rotational force is transmitted from the gear 95c of the second adjusting shaft 95 to the gear 94c of the first adjusting shaft 94 and the first adjusting shaft 94 rotates clockwise. Since the B cam 94b of the first adjusting shaft 94 slides against and presses the B projection 68b of the first movable member 60 due to the rotation of the first adjustment shaft 94, the first movable member 60 rotates counterclockwise in FIG. 16(a). On the other hand, since the B cam 95b of the second adjusting shaft 95 slides against and presses the B projection 77b of the second movable member 61 due to the rotation of the second adjusting shaft 95 as shown in FIG. 16(b), the second movable member 61 rotates counterclockwise in FIG. 16(b) and clockwise in FIG. 16(a).

Thus, the first movable member 60 and the second movable member 61 rotate in opposite directions, and the distance between the first vertical projecting piece 72 of the first movable member 60 and the second vertical projecting piece 81 of the second movable member 61, that is, the width of the tablet guide path 8b can be enlarged or reduced.

Next, the operation of the rotor 8 in the medicine cassette 2 configured as above will be described.

As described above, the tablet pocket 8a extending in the circumferential direction on the upper side surface of the rotor 8 and a plurality of tablet guide paths 8b extending downward from the upper side surface of the rotor 8 are provided between the cassette body 5 and the rotor 8 shown in FIG. 5.

Referring to FIG. 5, the tablets T stored in the cassette body 5 enter the tablet pocket 8a while being stirred by the rotation of the rotor 8, and the tablets T enter the tablet guide path 8b from the tablet pocket 8a. When the tablet guide path 8b approaches the tablet discharge hole 9, the partition member 20 fixed to the cassette body 5 enters between the lowermost tablet T in the tablet guide path 8b and the tablets T above the lowermost tablet T. The tablets T above the partition member 20 are prevented from falling downward by the partition member 20. The lowermost tablet T below the partition member 20 is on the tablet support table 55, but since the tablet support table 55 is inclined, the tablet falls down on the tablet support table 55 toward the tablet discharge hole 9, and the tablet is discharged from the tablet discharge hole 9. The tablet T discharged from the tablet discharge hole 9 is discharged through the tablet discharge path 3c of the base 2 shown in FIG. 2. As a result, each time the tablet guide path 8b turns to the tablet discharge hole 9, the tablet T is discharged one by one. By adjusting the rotation angle of the rotor 8, the number of tablets T corresponding to the prescription can be dispensed.

The tablet guide path 8b can adjust the entry position of the partition member 20 with respect to the thickness of the tablet T, the depth D corresponding to the thickness of the tablet T, the height H corresponding to the height of the tablet and the width W corresponding to the width of the tablet T using the partition adjusting mechanism M1, the depth adjusting mechanism M2, the height adjusting mechanism M3 and the width adjusting mechanism M4. Therefore, the tablet guide path 8b can be appropriately sized according to the shape or size of the tablets T to be stored in the cassette body 5. By using the same medicine cassette 2 or rotor 8 and adjusting the tablet guide path 8b to match various tablets T, the tablets can be discharged without exchanging the entire medicine cassette 2 or the rotor 8 for each different tablet T. Such adjustments can be made manually using an adjusting device described below.

### <Adjusting device>

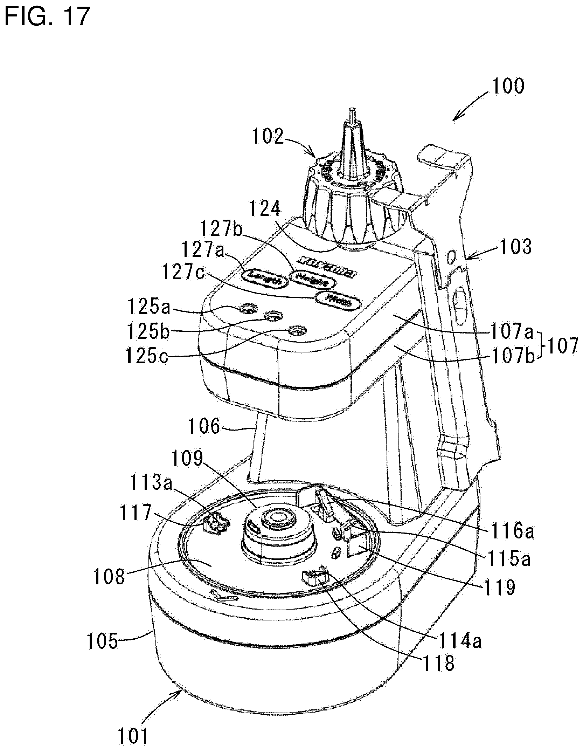

FIG. 17 shows the adjusting device 100 for a tablet cassette according to the present invention. The adjusting device 100 is for manually adjusting the depth, height and width of the groove of the tablet guide path 8b of the medicine cassette 2 already described and the entry position of the partition member 20.

That is, the adjusting device 100 engages with the engaging portions 33c, 52b and 95d of the adjusting members 33, 52 and 64 of the respective depth adjusting mechanism M2, height adjusting mechanism M3 and width adjusting mechanism M4 for adjusting the depth, height and width of the groove of the tablet guide path 8b of the medicine cassette 2 to operate the adjusting mechanism and adjusts the dimensions of the groove of the tablet guide path 8b according to the shape or size of the tablets T stored in the cassette body 5. Further, the adjusting device 100 engages with the engagement gear 24b of the partition adjusting member 24 of the partition adjusting mechanism M1 for adjusting the entry position of the partition member 20 to operate the partition adjusting mechanism M1, and the entry position of the partition member 20 is adjusted according to the shape or size of the tablets T contained in the cassette body 5 and the depth of the groove of the tablet guide path 8b.

The adjusting device 100 includes a device body 101, an adjusting member 102, a tool 103 and a control device 200.

The device body 101 has a base portion 105, an intermediate portion 106 rising upward from a rear portion of the base portion 105 and a guide portion 107 projecting forward from an upper end of the intermediate portion 106.

An upper surface of the base portion 105 serves as a rotor table 108 on which the rotor 8 of the medicine cassette 2 is placed. A rotor mounting protrusion 109 is provided on an upper surface of the rotor table 108. The rotor mounting protrusion 109 has the same shape as the engagement shaft 13 of the rotor drive part 10 of the cassette body 5 and is adapted to mount the medicine cassette 2 thereon.

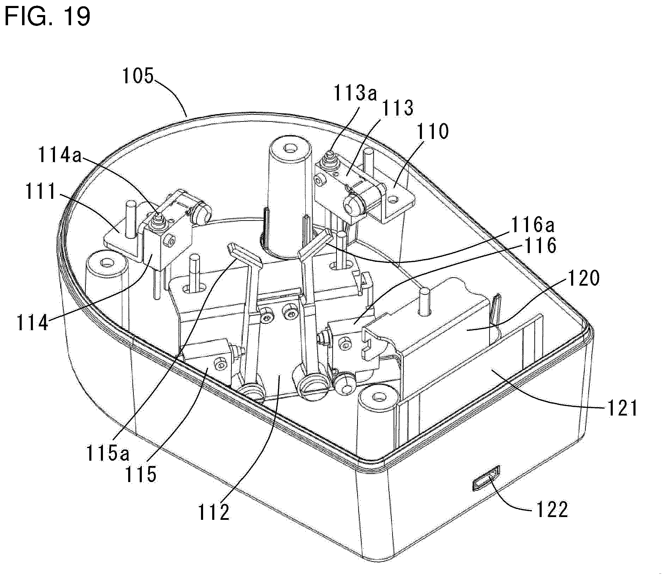

As shown in FIG. 19, on a rear surface of the rotor table 108, a zero point detection sensor 113, a zero point detection sensor 114 and zero point detection sensors 115 and 116 are mounted via brackets 110, 111 and 112 are provided respectively. Each of the sensors 113, 114, 115 and 116 consists of a limit switch and as shown in FIGs. 33, 35 and 36, detection portions 113a, 114a, 115a and 116a of the sensors 113, 114, 115 and 116 protrude upward from the rotor table 108. As shown in FIG. 35, the detection portion 113a of the zero point detection sensor 113 faces the lower surface of the tablet support table 55 of the rotor 8 placed on the rotor table 108 and is capable of coming into contact therewith. Further, as shown in FIG. 34, the detection portion 114a of the zero point detection sensor 114 faces the projection 37b of the guide portion 37 and is capable of coming into contact therewith. Further, as shown in FIG. 33, the detection portions 115a and 116a of the zero point detection sensors 115 and 116 face the lower end of the first vertical projecting piece 72 and the lower end of the second vertical projecting piece 81, respectively and can come into contact with each other. Returning to FIG. 17, the detectors 113a, 114a, 115a and 116a are prevented from coming into contact with foreign matter by U-shaped guard portions 117, 118 and 119 provided on the rotor table 108.

Further, as shown in FIG. 19, a substrate 121 is attached to the rear surface of the rotor table 108 via the bracket 120. The substrate 121 receives signals from the zero point detection sensor 113, the zero point detection sensor 114, the zero point detection sensors 115 and 116 and an encoder 131, which will be described later, and transmits the signals to the control device 200, which will be described later, power them. The base portion 105 is provided with a USB terminal 122 for connecting the substrate 121 and the control device 200.

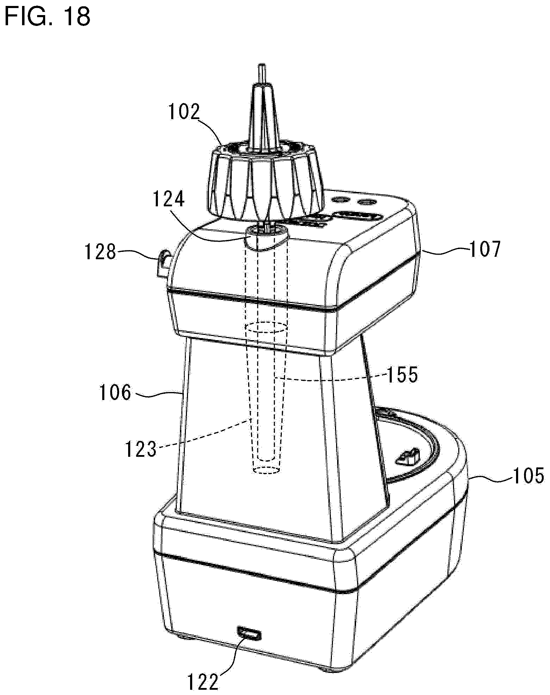

Inside the intermediate portion 106, as shown in FIG. 18, a cylindrical portion 123 in which a central shaft 155 of the adjusting member 102 is accommodated is provided. The cylindrical portion 123 communicates with an accommodating opening 124 of the guide portion 107, which will be described later, and extends vertically, and has a diameter that decreases downward.

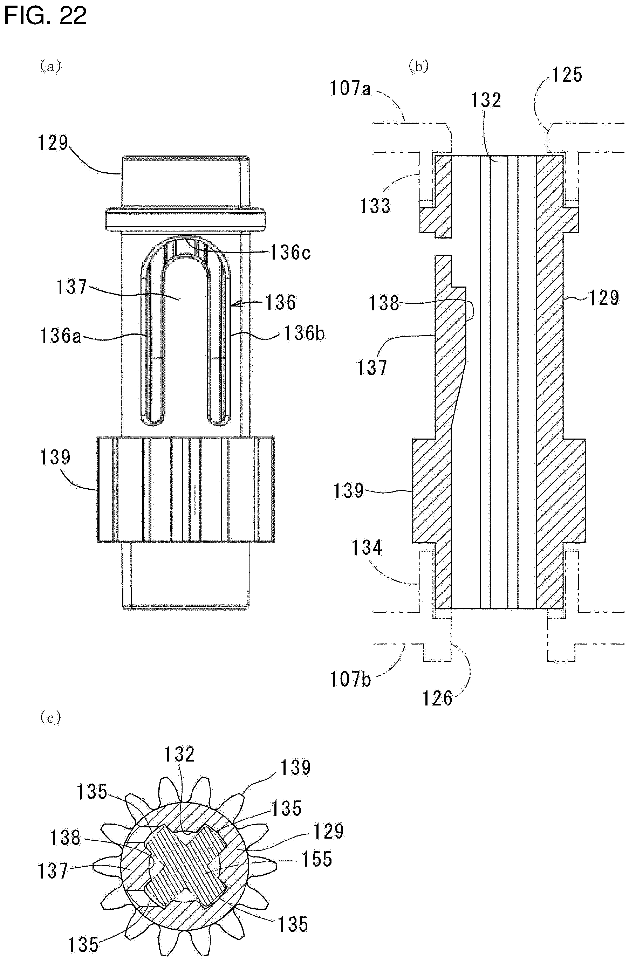

As shown in FIG. 17, the guide portion 107 is composed of an upper case 107a and a lower case 107b. The accommodating opening 124 is formed in the rear portion of the guide portion 107 so as to penetrate from an upper case 107a to a lower case 107b and into which the central shaft 155 is inserted when the adjusting member 102 is accommodated. Three upper guide holes 125a, 125b and 125c into which the central shaft 155 is inserted when the adjusting member 102 is used are formed in a front part of the upper case 107a of the guide part 107, and in the lower case 107b, lower guide holes 126 (see FIG. 22(b)) are formed at positions corresponding to the upper guide holes 125a, 125b and 125c. Indications 127a, 127b and 127c are provided near the upper guide holes 125a, 125b and 125c to indicate what the guide holes are for adjustment. Note that the upper guide holes 125a, 125b and 125c may be collectively referred to as the upper guide hole 125 below. A protrusion 128 (see FIG. 18) for attaching the tool 103 is formed on the rear side surface of the guide portion 107. Inside the guide part 107, three rotating members 129 (that is, a first rotating member 129a, a second rotating member 129b and a third rotating member 129c), an intermediate gear 130 and the encoder 131 are provided.

The three rotating members 129 have the same shape. As shown in FIG. 22, the rotating member 129 has a cylindrical shape with an engaging hole 132 that engages with the central shaft 155 of the adjusting member 102, which will be described later. The upper end of the rotating member 129 is rotatably engaged with an annular rib 133 formed around the upper guide hole 125 so that the engaging hole 132 communicates with the upper guide hole 125. Similarly, the lower end of the rotating member 129 is rotatably engaged with an annular rib 134 formed around a lower guide hole 126 so that the engaging hole 132 communicates with the lower guide hole 126. Four axially extending engaging grooves 135 are formed in an inner surface of the engaging hole 132 of the rotating member 129 at regular intervals in the circumferential direction. The rotating member 129 is provided with an elastic piece 137 by forming an inverted U-shaped slit 136 consisting of two axially extending linear portions 136a and 136b facing two adjacent engaging grooves 135 and an arc portion 136c connecting upper ends of the linear portions 136a and 136b. An inner surface of the elastic piece 137 is formed with a pressing portion 138 that is displaced inward from the inner surface of the engaging hole 132. The pressing portion 138 serves as a backlash prevention portion that prevents backlash between the central shaft 155 of the adjusting member 102 inserted into the engaging hole 132 and the engaging hole 132. A rotating gear 139 is provided below the elastic piece 137 of the rotating member 129. As shown in FIG. 21, the rotating gear 139 of the first rotating member 129a meshes with the rotating gear 139 of the second rotating member 129b and the rotating gear 139 of the third rotating member 129c is separated from the rotating gear 139 of the first rotating member 129a and the rotating gear 139 of the second rotating member 129b.

The intermediate gear 130 has a shaft portion 140 rotatably supported by the upper case 107a and the lower case 107b, and a tooth portion 141 fitted to the shaft portion 140 so as to be freely rotatable. The tooth portion 141 has a slight gap with respect to the shaft portion 140 and is movable in a direction perpendicular to the shaft portion 140. The tooth portion 141 of the intermediate gear 130 meshes with the rotating gear 139 of the second rotating member 129b and the rotating gear 139 of the third rotating member 129c.

The encoder 131 is used to detect an amount of operation of the adjusting part of the rotor 8 in the adjusting device 100. Specifically, the encoder 131 has a detection gear 142 that meshes with the intermediate gear 130. The encoder 131 is attached to a lever 143 rotatably provided on the lower case 107b. The lever 143 is biased by a coil spring 144 in a direction in which the detection gear 142 of the encoder 131 meshes with the intermediate gear 130. The lever 143 and the coil spring 144 serve as a backlash prevention portion that prevents backlash between the tooth portion 141 of the intermediate gear 130 and the detection gear 142 and between the tooth portion 141 of the intermediate gear 130 and the rotating gears 139 of the second rotating member 129b and the third rotating member 120c. The tooth portion 141 of the intermediate gear 130 is pushed by the detection gear 142 to maintain engagement with the detection gear 142 and move in a direction orthogonal to the shaft portion 140. As a result, the backlash between the gears is prevented by meshing with the rotating gears 139 of the second rotating member 129b and the third rotating member 120c without rattling.

When the central shaft 155 of the adjusting member 102 is inserted into the engaging hole 132 of the first rotating member 129a, the encoder 131 detects the amount of rotation of the central shaft 155 of the adjusting member 102 via the rotating gear 139 of the first rotating member 129a, the rotating gear 139 of the second rotating member 129b, the intermediate gear 130 and the detection gear 142. When the central shaft 155 of the adjusting member 102 is inserted into the engaging hole 132 of the second rotating member 129b, the encoder 131 detects the amount of rotation of the central shaft 155 of the adjusting member 102 via the rotating gear 139 of the second rotating member 129b, the intermediate gear 130 and the detection gear 142. When the central shaft 155 of the adjusting member 102 is inserted into the engaging hole 132 of the third rotating member 129c, the encoder 131 detects the amount of rotation of the central shaft 155 of the adjusting member 102 via the rotating gear 139 of the third rotating member 129c, the intermediate gear 130 and the detection gear 142. The amount of rotation of the adjusting member 102 detected by the encoder 131 is transmitted to the substrate 121 of the base portion 105 via a cord 145 and a connector 146.

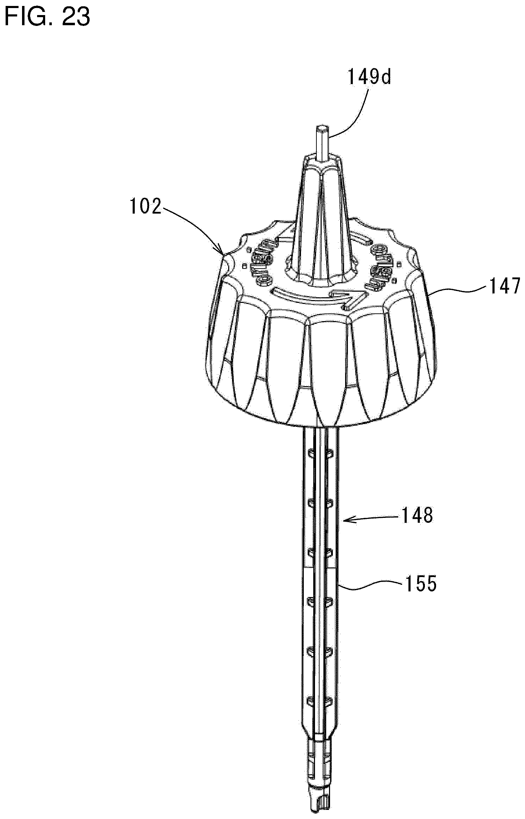

As shown in FIG. 23, the adjusting member 102 roughly includes a grip portion 147 and a shaft portion 148.

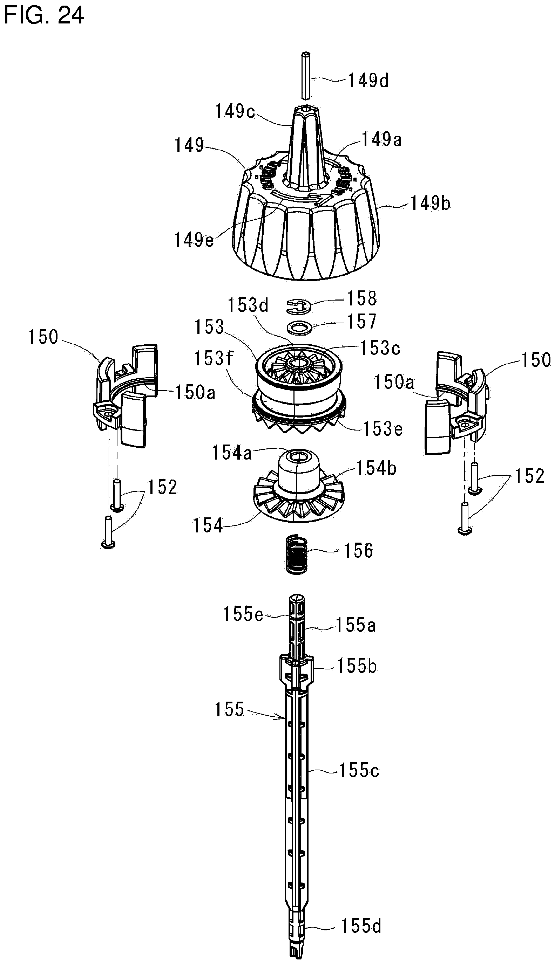

The grip portion 147 is composed of an outer member 149 and an inner member 150 as shown in FIG. 24.

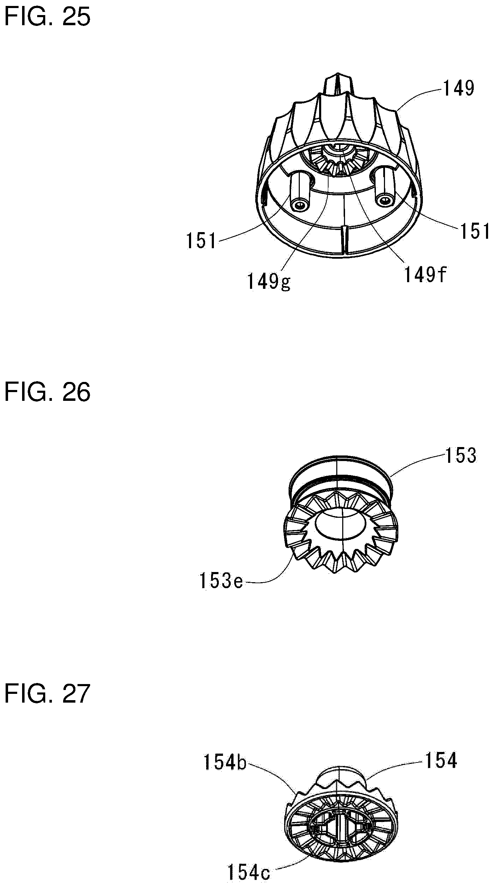

The outer member 149 has a large knob portion 149b having a truncated cone shape with an upper end closed by a ceiling wall 149a and an open lower end and a small knob portion 149c protruding axially from the top wall 149a. A hexagonal wrench 149d is attached to the upper end of the small knob portion 149c. The hexagonal wrench 149d is for engaging with a hexagonal hole (not shown) provided in the partition adjusting member 24 of the partition adjusting mechanism M1 of the medicine cassette 2 to drive the partition adjusting mechanism M1. An arrow mark 149e indicating the origin direction is provided on the surface of the ceiling wall 149a of the large knob portion 149b. As shown in FIG. 25, a shaft hole 149f and an engaging portion 149g consisting of irregularities arranged annularly around the shaft are formed on the rear surface of the ceiling wall 149a of the large knob portion 149b.

As shown in FIG. 24, the inner member 150 is formed in a half-cylindrical shape and is attached to a mounting seat 151 shown in FIG. 25 inside the outer member 149 with mounting screws 152. A flange 150a is formed on the inner circumference of the inner member 150 so as to protrude radially inward.

The shaft portion 148 is composed of a first member 153, a second member 154 and a central shaft 155.

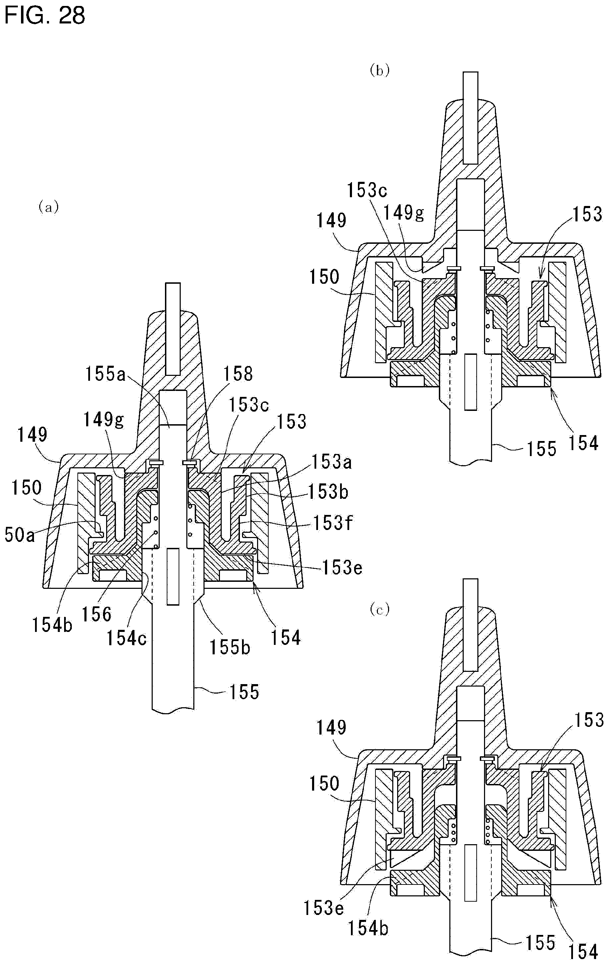

The first member 153 is sized to be accommodated inside the inner member 150 of the grip portion 147 and has an inner cylindrical portion 153a and an outer cylindrical portion 153b as shown in FIG. 28. An upper end of the inner cylindrical portion 153a is closed by an upper engaging portion 153c and a lower end thereof is open. A shaft hole 153d is formed in a center of the upper engaging portion 153c and concave and convex portions are formed on the upper surface thereof so as to be annularly arranged around the axis. The upper engaging portion 153c of the inner cylindrical portion 153a can be engaged with the engaging portion 149g of the grip portion 147. A lower end of the inner cylindrical portion 153a is open and has a lower engaging portion 153e on the outer periphery. As shown in FIG. 26, the lower surface of the lower engaging portion 153e is formed with irregularities arranged in an annular shape around the axis. An upper end of the outer cylindrical portion 153b is open and a lower end thereof is connected to an upper surface of the lower engaging portion 153e. An outer peripheral concave portion 153f into which the flange 150a of the inner member 150 of the grip portion 147 enters is formed on the outer periphery of the outer cylindrical portion 153b.

An upper portion of the second member 154 is formed in a cylindrical shape having a size that fits inside the inner cylindrical portion 153a of the first member 153. An upper end of the second member 154 is closed and a shaft hole 154a is formed in the center. A lower end of the second member 154 is open and an annular engaging portion 154b is formed on the outer circumference. An upper surface of the engaging portion 154b is formed with irregularities arranged in an annular shape around the axis. The engaging portion 154b of the second member 154 can be engaged with the lower engaging portion 153e of the first member 153. As shown in FIG. 27, four engaging grooves 154c extending in the axial direction are formed on the inner surface of the second member 154 at regular intervals in the circumferential direction.

The central shaft 155 has a base end portion 155a, a first engaging portion 155b, a second engaging portion 155c and a distal end portion 155d in this order from top to bottom in FIG. 24. The base end portion 155a is formed in a cylindrical shape to fit into the shaft hole 154a of the second member 154, the shaft hole 153d of the first member 153, and the shaft hole 149f of the outer member 149 of the grip portion 147. An outer peripheral groove 155e is formed on the outer peripheral surface of the base end portion 155a, with which a retaining ring 158, which will be described later, is engaged. The first engaging portion 155b has a cross-shaped cross section and can be engaged with the engaging groove 154c of the second member 154. The second engaging portion 155c has a cross-shaped cross section smaller than that of the first engaging portion 155b and is capable of engaging with an engaging groove 135 of the engaging hole 132 of the rotating member 129 of the guide portion 107. The distal end portion 155d is cylindrical and its distal end can be engaged with the engaging portions 33c, 52b and 95d of the adjusting members 33, 52 and 64 of the medicine cassette 2, respectively.

The central shaft 155 is retained by attaching a coil spring 156 to the base end portion 155a, penetrating the base end portion 155a into the shaft hole 154a of the second member 154 and the shaft hole 153d of the first member 153 and attaching the retaining ring 158 via a washer 157 to the outer peripheral groove 155e of the base end portion 155a projecting from the first member 153. The coil spring 156 is mounted between the first engaging portion 155b and the second member 154 in a compressed state. As a result, the first engaging portion 155b of the central shaft 155 engages the engaging groove 154c of the second member 154 and the second member 154 is biased toward the first member 153 by the coil spring 156. As a result, the engaging portion 154b of the second member 154 engages with the upper engaging portion 153c of the first member 153 so that the first member 153 and the second member 154 can rotate together.

The grip portion 147 is attached to the shaft portion 148 by fixing it to the mounting seat 151 of the outer member 149 with an attachment screw 152 in a state in which the half-split inner member 150 is assembled to the first member 153 of the shaft portion 148 so that the flange 150a of the inner member 150 fits into the outer peripheral concave portion 153f of the first member 153 of the shaft portion 148. Thereby, the grip portion 147 is axially slidable with respect to the shaft portion 148 and is movable between an engaged position where the engaging portion 149g engages with the upper engaging portion 153c of the first member 153 and engages with the central shaft 155 of the shaft portion 148 so as to be capable of rotating integrally and a non-engaged position where the engaging portion 149g is separated from the upper engaging portion 153c of the first member 153 and idles with respect to the central shaft 155.

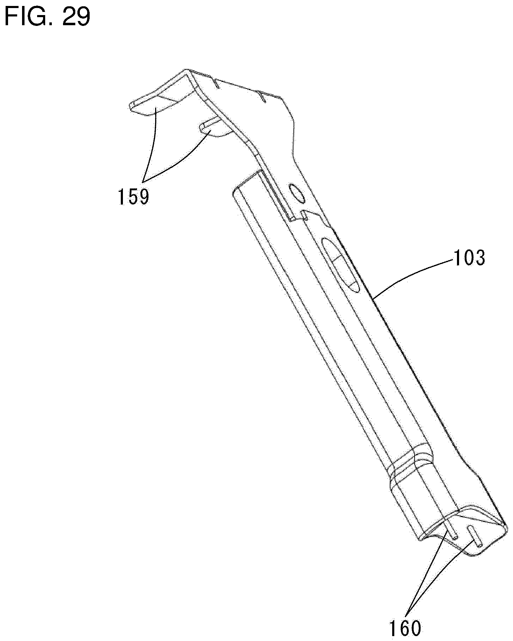

The tool 103 has a metal fitting 159 for removing a lifting unit from the cassette body 5 when the lifting unit (not shown) is attached to lift the medicine cassette 2 and a pin 160 for removing the rotor cover 30 as shown in FIG. 29.

### <Medicine dispensing system 500>

A system configuration of a medicine dispensing system 500 including the medicine dispensing device 1, the adjusting device 100 and the control device 200 described above will be described below.

As shown in FIG. 30, the medicine dispensing system 500 includes the medicine dispensing device 1, the adjusting device 100 and the control device 200. The medicine dispensing device 1, the adjusting device 100 and the control device 200 are connected via a communication path N. The communication path N may be a wire material such as a signal line, a USB cable or a LAN cable, or may be a wireless communication path using short-range wireless communication such as a wireless LAN or Bluetooth (registered trademark). The control device 200 can control the operations of the medicine dispensing device 1 and the adjusting device 100. The control device 200 is, for example, an information processing device such as a personal computer.

Any one of the medicine dispensing device 1, the adjusting device 100 and the control device 200 may be regarded as the medicine dispensing system or the adjusting system according to the present invention. In addition, constituent elements of the medicine dispensing system according to the present invention may be provided separately in any two or more of the medicine dispensing device 1, the adjusting device 100 and the control device 200. In this case, a system including the two or more devices is an example of the medicine delivery system or adjusting system according to the present invention. Note that the adjusting device 100 and the control device 200 may be mounted on or built into the medicine dispensing device 1.

In addition, in the present embodiment, the medicine dispensing device 1 will be described by taking as an example a configuration in which the tablets can be dispensed from the medicine cassette 2, but the medicine dispensing system 500 may include, instead of the medicine dispensing device 1, a medicine dispensing device that can dispense the medicine from a medicine dispensing part containing various dosage forms of medicine such as powdered medicine or liquid medicine.

### <Adjusting device 100>

As shown in FIGs. 30 and 31 , the adjusting device 100 includes an operation amount detection part 501 and an initial position detection part 502 and is connected to the control device 200. The operation amount detection part 501 includes an encoder 131 and inputs a detection result of the encoder 131 to the control device 200. The initial position detection part 502 also includes zero point detection sensors 113 to 116 and inputs the detection results from zero point detection sensors 113 to 116 to the control device 200.

### <Medicine dispensing device 1>

As shown in FIG. 30, the medicine dispensing device 1 includes a control part 310, a manual dispensing unit 302, a dispensing detection part 303, a code reading part 304, a data recording part 305, a data reading part 306, the medicine cassette 2 and the like.

The control part 310 has control devices such as a CPU, ROM, RAM and EEPROM (registered trademark). The CPU is a processor that executes various kinds of arithmetic processing. The ROM is a non-volatile storage unit in which information such as a control program for causing the CPU to execute various processes is stored in advance. The RAM is a volatile storage unit, and the EEPROM is a nonvolatile storage unit. The RAM and EEPROM are used as temporary memory (work areas) for various processes executed by the CPU.

Specifically, the control part 310 includes various processing parts such as a dispensing execution processing part 311. Specifically, the control part 310 functions as the various processing parts by using the CPU to execute various processing according to programs pre-stored in the ROM or the like. Part or all of the processing parts may be an electronic circuit.

The dispensing execution processing part 311 controls the medicine dispensing device 1 based on the prescription data input from the control part 210 of the control device 200, and a process for dispensing the tablets is executed using the medicine cassette 2 and the manual dispensing unit 302.

The manual dispensing unit 302 is used to dispense the tablets of a type not stored in the medicine cassette 2 and is generally also called a DTA (Detachable Tablet Adapter). The manual dispensing unit 302 is also used to dispense medicines that are not suitable for dispensing from the medicine cassette 2, such as half-tablets or quarter-tablets less than one tablet. Specifically, the manual dispensing unit 302 includes a plurality of DTA cells arranged in a matrix (lattice). Into each of the DTA cells, a tablet of a type that is included as a prescription medicine in the prescription data and that cannot be dispensed from the medicine cassette 2 is put in units of dosing times. Thereafter, based on the prescription data, the manual dispensing unit 302 opens a bottom surface of each of the DTA cells by a predetermined driving means such as a motor at the timing at which the tablets loaded into the DTA cells are required, so that it is possible to dispense tablets that have been placed in each of the DTA cells. A manual dispensing unit that can dispense medicines in units of DTA cells like the manual dispensing unit 302 is disclosed in, for example, JP2006-110386A.

The dispensing detection part 303 includes an optical sensor that detects tablets dispensed from the medicine cassette 2 in the medicine dispensing device 1 and inputs detection results from the optical sensor to the control device 200. Accordingly, the control device 200 can detect whether or not the tablets have been dispensed from the medicine cassette 2 and the number of the dispensed tablets based on the detection result of the dispensing detection part 303. Further, the dispensing detection part 303 includes an imaging unit that captures an image of a tablet dispensed from the medicine cassette 2 in the medicine dispensing device 1 and inputs the tablet image captured by the imaging unit to the control device 200.

The code reading part 304 can read information from a code such as a one-dimensional code or a two-dimensional code. For example, the code reading part 304 is used to read medicine identification information of the tablets from the code attached to the medicine box containing the tablets or to read prescription identification information from the code indicated on the prescription. The medicine identification information is predetermined information corresponding to each type of tablet as information capable of identifying the tablet.

The data recording part 305 is a so-called RFID reader/writer and is built in below a filling table 1a of the medicine dispensing device 1. Further, the data recording part 305 is provided at a position where the RFID tag 206 of the medicine cassette 2 can be accessed when the medicine cassette 2 is placed on the filling table 1a. The data recording part 305 is controlled by the control device 200 and can read data from the RFID tag 206 provided on the medicine cassette 2 and write data to the RFID tag 206.

The data reading part 306 is a so-called RFID reader and is provided at a position where the RFID tag 206 of the medicine cassette 2 mounted on the base 3 can be accessed when the medicine cassette 2 is mounted on the base 3 of the medicine dispensing device 1. The data reading part 306 is controlled by the control device 200 and can read data from the RFID tag 206 provided on the medicine cassette 2. Note that, as another embodiment, the data reading part 306 may be a reader/writer capable of writing data to the RFID tag 206. In this case, while the medicine cassette 2 is attached to the base 3, dispensing confirmation information, which will be described later, may be written to the RFID tag 206 of the medicine cassette 2.

The RFID tag 206 provided on the medicine cassette 2 is an example of a non-volatile storage part capable of storing data. Note that, instead of the RFID tag 206, a storage part in which data can be read and written by a contact type connection method, for example, may be used. The RFID tag 206 has storage areas including a writable region 261 and a non-writable region 262 as shown in FIG 30. The writable region 261 is a region from which data can be read by the data recording part 305 and the data reading part 306 and a region in which data can be written by the data recording unit 305 as well. The non-writable region 262 is a region in which the data recording part 305 and the data reading part 306 can read data, but the data recording part 305 cannot write data.

Specifically, in the writable region 261, medicine allocation information D21 (see FIG. 32B) including at least medicine identification information such as a medicine code or medicine name for identifying the type of tablet allocated to the medicine cassette 2 provided with the RFID tag 206 is stored. The medicine allocation information D21 includes, in addition to the medicine identification information such as medicine codes, information such as stability coefficients, arrangement restrictions, size types, branch numbers, model numbers, dispensing confirmation flags and cassette numbers. Note that the tablet dimensions (length, width, height, thickness) or the dimensions of the tablet guide path 8b suitable for tablets (depth, height, width) may be stored in the medicine allocation information D21.

The stability coefficient is a coefficient that indicates an ease of bouncing and rolling due to the shape and hardness of the tablet, and for example, it is used to calculate the time (maximum) for a tablet dispensed from each medicine cassette 2 to drop and stabilize at the discharge port. The arrangement restriction is information relating to the restriction of the mounting position when the medicine cassette 2 is mounted on the medicine dispensing device 1, and for example, the information is information for restricting the positions where the medicine cassettes 2 can be loaded, such as "all stages possible", "half or less" and "lower two rows only". The size type is the size of the medicine cassette 2 and is determined according to the number of tablets that can be accommodated, such as L, LM, M, S and SS. The branch number is a number allocated to distinguish between each when there are a plurality of medicine cassettes 2 containing tablets of the same type. The model number is an identification number of the medicine dispensing device 1 in which the medicine cassette 2 is mounted when there are a plurality of medicine dispensing devices 1.

An initial value of the dispensing confirmation flag is "1". Then, the control device 200 sets the value to "0" when determining that a test dispensing operation, which will be described later, which is executed after the adjusting part of the medicine cassette 2 is adjusted according to the type of tablets to be dispensed allocated to the medicine cassette 2 has been completed. In this embodiment, the dispensing confirmation flag "0" is an example of the dispensing confirmation information.

The cassette number is cassette identification information allocated to the medicine cassette 2 in order to identify the medicine cassette 2 in the medicine dispensing device 1. For example, the control device 200 sets the cassette number of the medicine cassette 2 according to a predetermined user operation and stores the cassette number in the medicine allocation information D21 of the RFID tag 206 of the drug cassette 2 by the data recording part 305. The control device 200 prohibits duplicate registration of the same cassette number in the medicine dispensing device 1.

On the other hand, cassette information D22 including information such as a cassette ID indicating unique identification information for identifying the medicine cassette 2 provided with the RFID tag 206 and the cassette type of the medicine cassette 2 (see FIG. 32B) is stored in advance in the non-writable region 262. The cassette type is information for identifying a plurality of types of medicine cassettes 2 having different suitability conditions for the tablets that can be dispensed. Specifically, the suitability conditions include one or both of minimum and maximum values for sizes such as tablet width, height, and thickness. The suitability conditions may also include the shape of tablets, such as capsule tablets or round tablets.

### <Control device 200>

As shown in FIGs. 30 and 31, the control device 200 is an information processing device such as a computer including the control part 210, a display part 201, a storage part 202, an operation part 203 and the like and is connected to the medicine dispensing device 1 and the adjusting device 100.

The display part 201 is displaying means such as a liquid crystal display, an organic EL display and a projector capable of displaying various kinds of information and also has a speaker capable of outputting sound.

The storage part 202 stores various data such as a medicine mounting master D11, an allocation correspondence information D31 and a base master D41.

As shown in FIG. 32(A), in the medicine mounting master D11, a medicine code, a medicine name, stability factor, placement restriction and a cassette type are associated with each other and stored for each tablet. The medicine code or the medicine name is an example of the medicine identification information that can identify the type of tablet. Further, the medicine mounting master D11 includes information on tablets registered in advance as tablets that can be dispensed by the medicine dispensing device 1. On the other hand, the storage part 202 may store a medicine master in which information similar to that of the medicine mounting master D11 is stored for medicines of types equal to or greater than the number of types of tablets registered in the medicine mounting master D11.

Further, in the medicine mounting master D11, dimensions such as the length, width, height and thickness of each tablet are stored in association with each other. Furthermore, in the medicine mounting master D11, for each tablet, an adjustment dimension such as adjustment depth, adjustment height and adjustment width indicating dimensions such as depth, height and width of the groove of the tablet guide path 8b suitable for tablets and an adjustment entry position of the partition member 20 into the tablet guide path 8b suitable for the tablet are stored in association with each other.

In this regard, when the adjustment dimensions are not stored in the medicine mounting master D11 and correction coefficients and the like used for calculating the adjustment dimensions are stored in the medicine mounting master D11, the control device 200 may derive the adjustment dimension based on the correction factor and the tablet size. Similarly, when the adjustment entry position is not stored in the medicine mounting master D11 and the adjustment entry position is stored in the medicine mounting master D11 with correction coefficients and the like (tablet thickness, etc.), the control device 200 may derive the adjustment entry position based on the correction factor and the tablet size.

In addition, in the medicine mounting master D11, medicine classifications (classification codes), medicine component (component code), JAN code, RSS code, YJ code, GS1 code, bottle code, dosage form, unit, specific gravity, medicine type, compounding change, excipient medicine, precautions, size type, tablet appearance image and the like are stored in association with each medicine. Further, the medicine mounting master D11 may be edited by the control device 200 based on a user operation or the like.

As shown in FIG. 32(C), in the allocation correspondence information D31, the cassette IDs which are the unique identification information of the medicine cassette 2 are associated with the medicine codes which are the medicine identification information of the tablet allocated to the medicine cassette 2. As a result, the control part 210 refers to the correspondence between the medicine code and the cassette ID in the allocation correspondence information D31 to control the medicine dispenser 1, and it is possible to dispense a specific type of tablet indicated in the prescription data from the medicine cassette 2 to which the type of tablet is allocated. Further, the allocation correspondence information D31 may store information about more medicine cassettes 2 than the number of medicine cassettes 2 that can be attached to the medicine dispenser 1 and the medicine dispensing device 1 may be able to dispense tablets using the medicine cassette 2 attached to the medicine dispensing device 1 among the medicine cassettes 2 stored in the allocation correspondence information D31. Moreover, the allocation correspondence information D31 may be included in the medicine mounting master D11.

As shown in FIG. 32(D), in the base master D41, for each medicine dispensing device 1 connected to the medicine dispensing system 500, the cassette ID of the medicine cassette 2 attached to the medicine dispensing device 1 and the base number for identifying the base 3 of the medicine dispensing device 1 to which the medicine cassette 2 is attached are stored in association with each other. When the medicine dispensing device 1 detects that the medicine cassette 2 has been attached or detached, the control part 210 updates the base master D41 according to the attachment/detachment result of the medicine cassette 2. Thereby, in the medicine dispensing system 500, the control part 210 can identify the medicine cassette 2 attached to each medicine dispensing device 1 and the base 3 to which the medicine cassette 2 is attached based on the base master D41 corresponding to each medicine dispensing device 1.

The operation part 203 is operation means such as a mouse, a keyboard and a touch panel that can accept user operations. Note that the display part 201 and the operation part 203 may be touch panel displays capable of accepting user operations.

The control part 210 has control devices such as a CPU, ROM, RAM and EEPROM (registered trademark). The CPU is a processor that executes various kinds of arithmetic processing. The ROM is a non-volatile storage unit in which information such as a control program for causing the CPU to execute various processes is stored in advance. The RAM is a volatile storage unit, and the EEPROM is a nonvolatile storage unit. The RAM and EEPROM are used as temporary memory (work areas) for various processes executed by the CPU.

The control part 210 includes an allocation processing part 211, a determination processing part 212, a storage processing part 213, a restriction processing part 214, a code output processing part 215, a reception processing part 216, a detection processing part 217, a display processing part 218, a notification processing part 219 and a dispensing processing unit 220. Specifically, the control unit 210 functions as the various processing parts by executing various processes using the CPU according to a medicine dispensing program pre-stored in the ROM or the storage part 202. Part or all of the processing parts may be an electronic circuit.

The allocation processing unit 211 accepts selection of the type of tablet to be allocated to the medicine cassette 2 according to the user's operation and allocates the type of tablet to the medicine cassette 2. For example, when the user performs an operation to read medicine identification information that can identify the type of tablet from the code using the code reading part 304, the allocation processing part 211 accepts the read tablet type as the type of tablet to be allocated. Alternatively, the allocation processing part 211 may display a list of candidate tablet types on the display part 201 and accept selection of a tablet type to be allocated to the medicine cassette 2 according to the user's operation on the operation part 203. Then, the allocation processing part 211 associates the medicine code corresponding to the selected tablet type with the cassette ID of the medicine cassette 2 and stores them in the allocation correspondence information D31, thereby allocating the tablet type to the medicine cassette 2.

The determination processing part 212 determines whether or not the tablet has been dispensed from the medicine cassette 2 provided with the adjusting part that can be adjusted to a plurality of states that allow different types of tablets to be dispensed. Specifically, the determination processing part 212 causes the medicine dispensing device 1 to execute a test dispensing operation for dispensing medicine from the medicine cassette 2 after the adjusting part adjusts according to the type of tablet to be dispensed allocated to the medicine cassette 2. That is, the test dispensing operation is not the medicine dispensing operation of the medicine cassette 2 based on the prescription data that is actually executed after the medicine cassette 2 is adjusted, and the test dispensing operation is executed after the medicine cassette 2 is adjusted and before the medicine dispensing operation of the medicine cassette 2 is executed based on the prescription data. Then, in the test dispensing operation, it is determined whether or not the tablets have been dispensed from the medicine cassette 2.

Specifically, in the test dispensing operation for the medicine cassette 2, which is executed after the adjusting part adjusts according to the type of tablet to be dispensed allocated to the medicine cassette 2, the determination processing part 212 determines that the tablets have been dispensed from the medicine cassette 2 on condition that the dispensing detection part 303 has detected that a predetermined number of tablets have been dispensed.

Further, after the adjusting part is adjusted according to the type of tablet to be dispensed allocated to the medicine cassette 2 and the test dispensing operation for the medicine cassette 2 is executed, the determination processing part 212 may determine that the medicine to be dispensed has been dispensed from the medicine cassette 2 on condition that a specific user operation has been performed. For example, the user visually confirms whether or not the tablets have been dispensed from the medicine cassette 2 by the test dispensing operation, and when the tablets have been normally dispensed from the medicine cassette 2, it is conceivable that the user performs the confirmation operation on the operation part 203. Then, the determination processing part 212 determines that the tablets have been dispensed from the medicine cassette 2 when the confirmation operation is performed after the test dispensing operation is performed.

Further, when the test dispensing operation for the medicine cassette 2, which is executed after the adjusting part adjusts according to the type of tablet to be dispensed assigned to the medicine cassette 2, is completed, the determination processing part 212 may determine that the medicine has been dispensed from the medicine cassette 2. That is, when the test dispensing operation is completed, the determination processing part 212 may assume that the tablets have been dispensed from the medicine cassette 2 without detection by the dispensing detection part 303 or confirmation operation by the user.

When the determination processing part 212 determines that the tablet has been dispensed from the medicine cassette 2, the storage processing part 213 stores the dispensing confirmation information in a preset storage part in a state in which the corresponding relationship between the medicine cassette 2 and the type of the tablet can be identified. Specifically, when the determination processing part 212 determines that the medicine has been dispensed from the medicine cassette 2, the storage processing part 213 updates the dispensing confirmation flag stored in the medicine allocation information D21 of the RFID tag 206 of the medicine cassette 2 from "1" to "0". In this way, by storing the dispensing confirmation information in the medicine allocation information D21 of the RFID tag 206 (an example of the storage part) of the medicine cassette 2, the dispensing confirmation information is in a state in which the corresponding relationship between both the medicine cassette 2 and the medicine code (type of medicine) of the medicine allocated to the medicine cassette 2 can be specified. As another embodiment, the dispensing confirmation information, the cassette ID or cassette number of the medicine cassette 2 and the medicine code indicating the type of medicine allocated to the medicine cassette 2 may be associated with each other and stored in the storage unit 202. In this case, the storage part 202 is an example of the storage part according to the present invention.

As the dispensing confirmation information stored in association with the medicine cassette 2, medicine identification information such as the medicine code may be used. Specifically, the control part 210 does not allocate medicine identification information to the medicine cassette 2 until tablets are dispensed from the medicine cassette 2 in a test dispensing operation described later, and the control part 210 allocates the medicine identification information to the medicine cassette 2 when the tablet is dispensed from the medicine cassette 2 in the test dispensing operation. For example, the control part 210 does not store the medicine code in the medicine allocation information D21 of the RFID tag 206 of the medicine cassette 2 until the tablet is dispensed from the medicine cassette 2 in the test dispensing operation, and when the tablet is dispensed from the medicine cassette 2 in the test dispensing operation, it is conceivable to store the medicine code in the medicine allocation information D21 of the RFID tag 206 of the medicine cassette 2.

When the dispensing confirmation information corresponding to the medicine cassette 2 is not stored in the RFID tag 206, the restriction processing part 214 restricts the medicine dispensing operation of dispensing the medicine from the medicine cassette 2 based on the prescription data. Specifically, when the dispensing confirmation flag stored in the RFID tag 206 of the medicine cassette 2 is "0", the restriction processing part 214 permits the medicine dispensing operation using the medicine cassette 2. On the other hand, when the dispensing confirmation flag stored in the RFID tag 206 of the medicine cassette 2 is "1", the restriction processing part 214 restricts the medicine dispensing operation using the medicine cassette 2. Note that the content of the restriction includes prohibiting the execution of the medicine dispensing operation or requiring confirmation operation by the user when starting the medicine dispensing operation.

As another embodiment, when the dispensing confirmation information corresponding to the medicine cassette 2 is not stored in the RFID tag 206, the dispensing execution processing unit 311 of the medicine dispensing device 1 instead of the restriction processing part 214 may restrict the medicine dispensing operation of dispensing the medicine from the medicine cassette 2 based on the prescription data. In this case, the dispensing execution processing part 311 is an example of the restriction processing part according to the present invention.

The code output processing part 215 can output label information corresponding to the label L1 including the verification code. The verification code is a one-dimensional code or a two-dimensional code readable by the code reading unit 304, is issued after adjustment of the medicine cassette 2 according to the type of tablet performed using the adjustment device 100 and indicates the type of tablet and the cassette ID of the medicine cassette. For example, the code output processing part 215 outputs the label information to a printer or label printing device (not shown) connected to the control device 200 or the medicine dispensing device 1 and prints it as a printed matter such as a card or sticker.

The reception processing part 216 can receive any one or a plurality of inputs of the quantity of medicines to be filled in the medicine cassette 2, the date of filling, or the expiration date before the start of the test dispensing operation. In other words, the user can input any one or more of the quantity of medicine to be filled into the medicine cassette 2, the date of filling, and the expiration date, even if the test dispensing operation has not ended. As a result, when the user fills the medicine cassette 2 with tablets during the test dispensing operation, the user can fill the tablets to be used later together.

The detection processing part 217 can detect a current adjustment value by the adjusting part that can manually adjust the dimension of the groove of the tablet guide path 8b, which is the tablet delivery path in the medicine cassette 2. Specifically, the detection processing part 217 can detect the current adjustment values of the groove depth, height and width of the tablet guide path 8b based on the detection results by the operation amount detection part 501 and the initial position detection part 502. For example, after the adjustment of the depth of the groove of the tablet guide path 8b is started and then the zero point (initial state) is detected by the initial position detection part 502, the detection processing part 217 calculates the current adjustment value of the groove depth of the tablet guide path 8b based on the operation amount detected by the operation amount detection part 501. Note that in the present embodiment, the zero point detected by the initial position detection part 502 is the position where the current adjustment value is zero.

The display processing part 218 displays the current adjustment value detected by the detection processing part 217 and the target value of adjustment so that they can be compared. Further, the display processing part 218 displays adjustment value information that can specify the difference between the current adjustment value and the target value. Specifically, the display processing part 218 displays a second region having an area, length, or width corresponding to the difference between the target value and the current adjustment value and a third region having an area, length, or width corresponding to the current adjustment value in a first region having an area, length, or width corresponding to the target value. Furthermore, the display processing part 218 displays the presence or absence of the dispensing confirmation information corresponding to the medicine cassette 2 on the display screen displayed when the medicine cassette 2 is filled with the medicine.

Further, the display processing part 218 is capable of displaying a list of a plurality of work process groups included in the manual adjustment work using the adjustment unit, and the display processing unit 218 when performing the list display corresponds to the first display processing part according to the present invention. Furthermore, after the work process group is displayed by the first display processing part, the display processing part 218 can sequentially display one or more work processes included in each of the work process groups, and the display processing part 218 when performing the display corresponds to the second display processing part according to the present invention. In addition, in the medicine dispensing system 500, the first display processing part and the second display processing part may be provided as different processing parts.

The notification processing part 219 notifies when the difference between the current adjustment value and the target value reaches a preset specific range or less. This allows the user to easily determine whether the adjustment of the grooves of the tablet guide path 8b by the adjusting part is appropriate. Note that the notification by the notification processing part 219 may be in various notification modes such as display output, audio output, or LED lighting state output.

The dispensing processing part 220 inputs the prescription data to the medicine dispensing device 1 as medicine dispensing data and causes the medicine dispensing device 1 to execute the medicine dispensing operation based on the prescription data. In addition, the dispensing processing part 220 may generate packaging data for causing the medicine dispensing device 1 to perform the packaging operation based on the prescription data and may input the packaging data to the medicine dispensing device 1 as the medicine dispensing data.

Hereinafter, an example of procedures of medicine allocation processing, adjustment support processing and medicine dispensing processing executed by the medicine dispensing system 500 according to this embodiment will be described. If the medicine allocation processing, the adjustment support processing and the medicine dispensing processing are executed in the medicine dispensing system 500 as a whole, the processing entity and execution timing of each step are not limited to those described here.

### [Medicine allocation processing]

First, with reference to FIG. 33, an example of the procedure of the medicine allocation processing will be described. For example, the control part 210 of the control device 200 starts the medicine allocation processing in response to a predetermined user operation on the operation unit 203. After a user places the medicine cassette 2 on the filling table 1a of the medicine dispensing device 1, the user performs a predetermined user operation to cause the control unit 210 to execute the medicine allocation processing.

### <Step S21>

In step S21, the control part 210 executes a processing for allowing the user to select the type of tablet to be newly allocated to the medicine cassette 2 and shifts the processing to step S22. Hereinafter, the type of tablet newly allocated to the medicine cassette 2 may be referred to as an allocated tablet. Note that step S21 is executed by the allocation processing part 211 of the control part 210.

Specifically, the user uses the code reading part 304 to read medicine identification information from a code such as a one-dimensional code or a two-dimensional code attached to a medicine cabinet containing tablets corresponding to the allocated tablets. Thereby, the control part 210 selects the tablet type corresponding to the medicine identification information read by the code reading part 304 as the allocated tablet.

In addition, as another embodiment, the control part 210 may cause the display part 201 to display a medicine selection screen for selecting the allocated tablet based on the medicine mounting master D11 and may accept an operation of selecting the type of tablet on the medicine selection screen.

### <Step S22>

In step S22, the control part 210 causes the display part 201 to display an allocation start screen P1 for accepting an operation to start allocation of the allocated tablets to the medicine cassette 2. As shown in FIG. 37, the allocation start screen P1 includes a display region A1 in which information about the allocated tablets selected in step S21 is displayed and a display region A2 where information about the type of tablets currently allocated to the medicine cassette 2 is displayed. Hereinafter, the type of tablet currently allocated to the medicine cassette 2 may be referred to as "already allocated tablet".

Specifically, the control part 210 causes the information on the allocated tablets to be displayed in the display region A1 based on the medicine mounting master D11. For example, the display region A1 displays a medicine code and a medicine name that can identify the allocated tablet, a cassette type of the medicine cassette 2 that matches the allocated tablet, an appearance image of the tablet that corresponds to the allocated tablet and the like.

Further, the control part 210 controls the data recording part 305 to read the medicine allocation information D21 and the cassette information D22 from the RFID tag 206 of the medicine cassette 2 placed on the filling table 1a. Further, the control part 210 causes the information on the already allocated tablets to be displayed in the display region A2. For example, the display region A2 displays a medicine code and a medicine name that can identify the already allocated tablet, the cassette type of the medicine cassette 2, the external image of the tablet corresponding to the tablet already allocated to the medicine cassette 2 and the like.

The allocation start screen P1 also includes an operation key K3 for expanding the information of the allocated tablets displayed in the display region A1. When the operation key K3 is operated, the control part 210 expands the display region A1 on the allocation start screen P1 as shown in FIG. 38. Then, the control part 210 additionally displays information about the allocated tablet such as the JAN code or GS1 code corresponding to the allocated tablet, tablet size (length, width, height, thickness), stability factor, arrangement restriction, size type in the expanded display region A1 based on the medicine mounting master D11. After that, when the operation key K3 is operated again, the control part 210 cancels the expansion of the display region A1 on the allocation start screen P1. Note that the control part 210 determines whether or not the allocated tablet selected in step S21 is suitable for the medicine cassette 2 placed on the filling table 1a and an error notification may be given if the allocated tablet selected in step S21 is not suitable.

### <Step S23>

In step S23, the control part 210 controls the data recording part 305 and reads information of "cassette ID" stored in the cassette information D22 as unique identification information of the medicine cassette 2 from the RFID tag 206 of the medicine cassette 2 placed on the filling table 1a. Further, the control part 210 causes the storage part 202 to store the unique identification information of the medicine cassette 2 to be allocated this time. The "cassette number" included in the medicine allocation information D21 may be used instead of the "cassette ID".

### <Step S24>

In step S24, the control part 210 determines whether or not an operation to start allocating the allocated tablets to the medicine cassette 2 has been performed. Specifically, the operation key K1 for accepting the start operation for allocating the tablets to the medicine cassette 2 is displayed on the allocation start screen P1. When the operation key K1 is operated, the control part 210 determines that the start operation of allocating the allocated tablets to the medicine cassette 2 has been performed. Here, when it is determined that the start operation has been performed (S24: Yes), the process proceeds to step S25. Until it is determined that the start operation has been performed (S24: No), the process waits in step S24. During this time, the control part 210 can accept an operation to change the allocated tablets, an operation to cancel the allocation, or the like.

### <Step S25>

In step S25, the control part 210 collates the information of the RFID tag 206 of the medicine cassette 2 placed on the filling table 1a. Specifically, the control part 210 controls the data recording part 305 and reads information of "cassette ID" stored in the cassette information D22 as the unique identification information of the medicine cassette 2 from the RFID tag 206 of the medicine cassette 2 placed on the filling table 1a. Then, the control part 210 collates the information of the "cassette ID" read from the RFID tag 206 with the "cassette ID", which is the unique identification information of the medicine cassette 2 to be allocated this time, stored in the storage part 202 in step S23. Here, when the collation result matches, the control part 210 causes the processing to proceed to step S26. It should be noted that when the collation result does not match, the control part 210 executes, for example, a predetermined error notification process and returns the process to step S24. This prevents the medicine cassette 2 placed on the filling table 1a from being changed before and after the allocation start operation in step S24.

### <Step S26>

In step S26, the control part 210 executes a processing for canceling the allocation of the current tablet type to the medicine cassette 2 and ends the medicine allocation processing. Specifically, the control part 210 controls the data recording part 305 to erase the contents of the medicine allocation information D21 stored in the RFID tag 206 of the medicine cassette 2 placed on the filling table 1a. Since the initial value of the dispensing confirmation flag is "1", it is reset to "1" in step S26 in the case where the dispensing confirmation flag of the medicine allocation information D21 is "0". That is, in step S26, the dispensing confirmation information is erased. Thus, when the tablet allocated to the medicine cassette 2 is changed, the dispensing confirmation flag is reset to "1" (dispensing confirmation information is deleted). Therefore, after the allocated tablet is changed, until the dispensing confirmation flag is newly updated to "0" (dispensing confirmation information is stored), in the medicine dispensing process (see FIG. 58), which will be described later, the medicine dispensing operation using the medicine cassette 2 is restricted.

Further, in step S26, the control part 210 deletes the medicine code associated with the medicine cassette 2 placed on the filling table 1a in the allocation correspondence information D31 stored in the storage part 202. That is, during the adjustment of the medicine cassette 2 by the adjusting device 100, the type of tablet is not allocated to the medicine cassette 2. This prevents the medicine cassette 2 in which the rotor 8 has not been properly adjusted from being used in the medicine dispensing device 1.

### [Adjustment support processing]

Next, an example of the procedure of the adjustment support processing will be described with reference to FIGs. 34 to 36. The control part 210 starts the adjustment support processing after the medicine allocation processing ends. Further, the control part 210 may execute the adjustment support processing at any timing according to a predetermined user operation after the medicine allocation processing ends.

### <Step S31>

In step S31, the control part 210 displays on the display part 201 a summary display screen P10 that displays a summary of the manual adjustment work performed by the user. Specifically, the control part 210 displays a list of a plurality of work process groups included in the manual adjustment work using the adjusting part of the adjustment device 100. The list display is performed by the display processing part 218 of the control part 210. Each of the process groups includes one or more work processes. The manual adjustment work is performed by the user using the adjusting device 100 to operate the adjusting part provided on the rotor 8 of the medicine cassette 2 and is a work of adjusting the dimensions of the grooves of the tablet guide path 8b of the rotor 8 of the medicine cassette 2 according to the allocation state. Step S31 is executed by the display processing part 218 of the control part 210.

As shown in FIG. 39, on the summary display screen P10, the contents of a plurality of preset process groups in the entire process of the manual adjustment work are collectively displayed on one screen. Specifically, on the summary display screen P10 shown in FIG. 39, four divided areas A10 corresponding to the four process groups of "remove the rotor", "adjust", "replace rotor", "fill and dispense test" are displayed. In each of the divided areas A10, work summary information such as characters, images, photographs or moving images indicating the main work content in the process group corresponding to the divided area A10 is displayed. This allows the user to easily grasp the outline of the manual adjustment work before starting the manual adjustment work.

Further, the summary display screen P10 displays an operation key K10 that the user operates after confirming the summary display screen P10. When the operation key K10 is operated, the control part 210 causes the processing to proceed to step S32.

### <Step S32>

In step S32, the control part 210 displays on the display part 201 a work support screen P11 for supporting the rotor removal process for removing the rotor 8 from the medicine cassette 2 by the user in the process group.

As shown in FIG. 40, a display region A111 in which characters, images, photographs, moving images or the like indicating the details of the work process included in the rotor removal process are displayed, a display region A112 showing the progress of the work process in the rotor removal process, and the like are displayed on the work support screen P11. For example, the rotor removal process includes four work processes, and when the work process currently being displayed in the display region A112 is the first work process, information indicating this is displayed in characters (for example, "1/4").

Further, on the work support screen P11, an operation key K111 and an operation key K112 for advancing the work process in the rotor removal process are displayed. The control part 210 advances one work process in the rotor removal process displayed on the work support screen P11 according to the operation of the operation key K 111 or the operation key K112. Furthermore, the work support screen P11 also displays an operation key K113 for returning to the work process in the rotor removal process. The control part 210 moves back one work process in the rotor removal process displayed on the work support screen P11 in response to the operation of the operation key K113. Then, when the operation key K111 is operated on the work support screen P11 displayed corresponding to the last work process included in the rotor removal process, the control part 210 shifts the processing to step S33.

As a result, the user can sequentially refer to the display in the display region A111 and easily perform the rotor removal process for removing the rotor 8 from the medicine cassette 2. For example, the rotor removing process includes a step of retracting the partition member 20 by operating the partition adjusting mechanism M1 of the medicine cassette 2, a step of removing the rotor 8 from the medicine cassette 2, a step of removing the rotor cover 30 of the rotor 8 and a step of mounting the rotor 8 on the rotor base 108 of the adjusting device 100. In this embodiment, a case where the rotor 8 is attached to and detached from the medicine cassette 2 when adjusting the rotor 8 using the adjusting device 100 will be described as an example, but the adjusting device 100 may be configured to adjust the rotor 8 with the rotor 8 attached to the medicine cassette 2.

### <Steps S33 to S35>

In steps S33 to S35, the control part 210 displays on the display part 201 a work support screen P21 for supporting a dimension adjusting process in which the user adjusts the dimensions (depth, height, width) of the groove of the tablet guide path 8b in the rotor 8 among the process groups. The dimension adjusting process includes three work processes of a height adjusting process, a depth adjusting process and a width adjusting process for adjusting the height, depth and width of the groove of the tablet guide path 8b of the rotor 8 in order.

As shown in FIG. 41, the work support screen P21 includes a display region A211 in which characters, images, photographs, videos and the like indicating the details of the work process included in the dimension adjusting process are displayed, a display region A212 showing the progress of the work process in the dimension adjusting process, and a display region A213 showing information on dimensional adjustment and the like.

The display region A213 includes display regions A214, A215 and A216 corresponding to the height, depth and width of the groove of the tablet guide path 8b of the rotor 8, respectively. The display regions A214 to A216 also include display regions A217 to A219 in which information about the start position, adjustment value and target value are displayed.

The display region A217 is a region for displaying whether the zero point detection sensors 113 to 116 have detected the zero point for the dimension to be adjusted. The display region A218 is a region for displaying adjustment difference information capable of specifying the difference between the current adjustment value and the adjustment target value for the dimension to be adjusted.

Then, as shown in FIGs. 41 to 43, in the display region A218, the control part 210 displays an adjustment display image F11 having an annular first region F111 having an area corresponding to the target value as the adjustment difference information. A numerical value indicating the current adjustment value is displayed in the central area F110 of the adjustment display image F11. Also displayed in the display region A218 is an arrow image F12 indicating the direction in which the user should rotate the adjusting member 102 in order to bring the current adjustment value closer to the target value. For example, an image suggesting clockwise rotation is displayed as the arrow image F12 until the current adjustment value reaches the target value, and when the current adjustment value reaches or exceeds the target value, an image suggesting counterclockwise rotation is displayed as the arrow image F12.

Further, the control part 210 displays a second region F112 having an area corresponding to the difference between the target value and the current adjustment value and the third region F113 having an area corresponding to the current adjustment value in the adjustment display image F11 in a manner that can be distinguished by coloring or the like. Then, the control part 210 updates the areas of the second region F112 and the third region F113 in the first region F111 according to the degree of attainment of the current adjustment value with respect to the target value. Specifically, the area of the third region F113 expands in the circumferential direction as the current adjustment value approaches the target value from a predetermined position in the first region F111, and when the current adjustment value and the target value match, the third region F113 is displayed over the entire first region F111. Accordingly, the user can perform adjustment work while easily grasping the magnitude of the difference between the current adjustment value and the target value by referring to the adjustment difference information, and it is also possible to easily grasp that the adjustment has been completed. The shape of the adjustment display image F11 is not limited to the annular shape, and may be, for example, a bar-shaped image that can represent the magnitude of numerical values by length or width.

The display region A219 is a region for displaying target values for adjusting the depth, height, width or the like of the groove of the tablet guide path 8b. Based on the dimensions of the adjustment depth, adjustment height and adjustment width corresponding to the allocated tablet registered in the medicine mounting master D11, the control part 210 identifies target values for adjusting the depth, height, width or the like of the groove of the tablet guide path 8b. The dimensions of the adjustment depth, the adjustment height and the adjustment width may be the same numerical values as the target values for adjustment of the groove depth, height, width or the like of the tablet guide path 8b, but the target value may be a value obtained by converting each dimension of adjustment depth, adjustment height and adjustment width into a displacement amount detected by the encoder 131. Further, the control part 210 may download the target values of the groove depth, height and width of the tablet guide path 8b from a host computer or a database on the Internet.

Here, referring to FIG. 44, the content of the height adjusting process performed by the user and the display content of the adjustment support screen P21 will be described, taking the height adjusting process in step S33 among steps S33 to S35 as an example. The display processing executed in steps S33 to S35 is executed by the display processing part 218 of the control part 210.

First, in the display region A213 of the adjustment support screen P21, the control unit 210 highlights the display region A214 corresponding to the height to be adjusted first to indicate that the height is being adjusted. Further, the control part 210 displays the content of the work in the display region A211 and displays the arrow image F12 indicating the rotation direction in which the adjustment member 102 should be rotated in the display region A218. Further, the control part 210 displays "undetected" indicating that zero point detection is not performed in the display region A217 of the display region A214 until the zero point detection is performed by the zero point detection sensor 113. Note that "undetected" is displayed in the display regions A215 and A216 as well as in the display region A217.

Here, the user takes out the adjusting member 102 from the accommodating opening 124 of the device body 101. Next, the user inserts the central shaft 155 of the device body 101 from the upper guide hole 125 of the "Length" of the device body 101 toward the lower guide hole 126 so that the central shaft 155 engages with the engaging portion 52b of the height adjusting mechanism M3 of the rotor 8 as shown in FIG. 44. As a result, the central shaft 155 of the adjusting member 102 engages with the engaging hole 132 of the rotating member 129 so that the central shaft 155 can rotate integrally with the rotating member 129. The rotation of rotating member 129 is transmitted to the detection gear 142 of the encoder 131 via the intermediate gear 130, the amount of rotation is detected by the encoder 131, and the amount of rotation is transmitted to the control device 200.

When the adjusting member 102 is held by the grip portion 147, the shaft portion 148 moves downward with respect to the grip portion 147 due to its own weight and the outer member 149g of the grip portion 147 is disengaged from the upper engaging portion 153c of the first member 153 as shown in FIG. 28(b). As a result, even if the grip portion 147 is rotated while the central shaft 155 is not engaged with the engaging portion 95d, the rotational force is not transmitted to the shaft portion 155, so the encoder 131 does not detect the rotation. When the central shaft 155 is pressed against the engaging portion 95d, the central shaft 155 moves relative to the grip portion 147 and the engaging portion 149g and the upper engaging portion 153c are engaged. As a result, by rotating the grip portion 147, the central shaft 155 is rotated. Further, when the central shaft 155 is forced to rotate beyond the zero point while the central shaft 155 is engaged with the engaging portion 95d, torque is applied and the torque limiter is activated. As a result, as shown in FIG. 28C, the lower engaging portion 153e of the first member 153 is disengaged from the engaging portion 154b of the second member 154 and the rotation of the grip portion 147 is not transmitted to the central shaft 155. As a result, the rotation mechanism such as the rotation member 129, the intermediate gear 130, the encoder 131 and the adjusting mechanism of the rotor 8 are not damaged.

Next, while the user presses the grip portion 147 downward, the user rotates the central shaft 155 in the direction of the origin indicated by the arrow on the grip portion 147 until the zero point detection sensors 113 is turned on. When the zero point detection sensor 113 is turned on, the central shaft 155 is rotated in the direction opposite to the origin direction until the zero point detection sensor 113 is turned off. Further, when the zero point detection sensor 113 is turned off, the central shaft 155 is rotated in the origin direction until the zero point detection sensor 113 is turned on.

Here, when the zero point detection sensor 113 is turned on for the second time, the control part 210 resets the current value of the height of the groove of the tablet guide path 8b and then starts detection of the current value of the rotating member 129 by the encoder 131. Thus, the control part 210 detects the current adjustment value based on the operation amount detected by the operation amount detection part 501 after the zero point detection is performed by the initial position detection part 502. That is, the control part 210 detects the current adjustment value by the adjusting part based on the detection results by the operation amount detection part 501 and the initial position detection part 502. Note that this detection processing is an example of a detection step executed by the detection processing part 217 of the control part 210.

Further, when the zero point detection sensor 113 detects the zero point for the second time, the control part 210 displays "Detection OK" indicating that the zero point has been detected in the display region A217 of the display region A214. Furthermore, the control part 210 causes the display region A218 in the display region A214 corresponding to the height to start displaying the second region F112 and the third region F113 in the adjustment display image F11 and causes the display region A219 to display the adjustment target value. In this embodiment, it is assumed that the zero point detection sensor 113 has been detected from the beginning and it is determined that the zero point detection has been performed when the zero point detection has been performed twice, but as another embodiment, it may be determined whether or not zero point detection has been performed only once.

After that, the control part 210 adds or subtracts the current adjustment value to update the adjustment display image F11 displayed as the adjustment difference information based on the operation amount of the rotating member 129 detected by the encoder 131. Specifically, the control part 210 causes the current adjustment value to be displayed in the region F110 and updates the third region F113 as needed according to the current adjustment value. That is, as the user rotates the adjusting member 102 and the current adjustment value approaches the target value, in the first region F111, the area of the third region F113 increases clockwise from the predetermined position and the area of the second region F112 decreases. This allows the user to grasp at a glance that the current adjustment value is approaching the target value.

Furthermore, after the zero point detection is performed by the zero point detection sensor 113, the control part 210 notifies the user of the adjustment status by reproducing a sound corresponding to the difference between the current adjustment value and the target value from the display part 201. Specifically, the preset ringing interval of a first electronic sound is shortened as the difference between the current adjustment value and the target value becomes smaller, and when the difference between the current adjustment value and the target value falls below a preset specific range, the control part 210 reproduces a preset second electronic sound or voice. Such notification processing is executed by the notification processing part 219 of the control part 210. Thereby, the user can perform the adjustment work while grasping the magnitude of the difference between the current adjustment value and the target value by the ringing interval of the first electronic sound. Further, the user can easily recognize that the adjustment has been completed by the reproduction of the second electronic sound or voice. Note that the control part 210 may execute either display of the adjustment display image F11 or reproduction of the sound.

Then, the user rotates the grip portion 147 in the direction opposite to the origin direction until the current value reaches the target value. At this time, it is preferable to hold and turn the large knob portion 149b. This is because the amount of rotation of the small knob 149c becomes large and may overshoot the target value. While the grip portion 147 is rotated, the current value is displayed on the display part A219. When the current value reaches the target value, the user stops rotating the grip portion 147 and removes the adjusting member 102 from the device main body 101.

Here, on the work support screen P21, an operation key K211 and an operation key K212 for advancing the work process in the dimension adjusting process are displayed, and the control part 210 advances one work process in the dimension adjusting process displayed on the work support screen P21 according to the operation of the operation key K211 or the operation key K212. The work support screen P21 also displays an operation key K213 for returning the work process in the dimension adjusting process, and the control part 210 moves back one work process in the dimension adjusting process displayed on the work support screen P21 in response to the operation of the operation key K213.

After the adjustment of the height of the groove of the tablet guide path 8b in step S33 is completed as described above, in the subsequent step S34, a process for supporting the adjustment of the depth of the groove of the tablet guide path 8b is executed. At this time, the user inserts the central axis 155 of the adjusting member 102 from the upper guide hole 125 of the device body 101 toward the lower guide hole 126 of "Depth" so that the engaging portion 33c of the depth adjusting mechanism M2 of the rotor 8 is engaged as shown in FIG. 44(b). Then, similar to the height adjustment, the adjusting member 102 is rotated to adjust the height of the groove of the tablet guide path 8b. Further, in step S34, as shown in FIG. 42, the adjustment status of the height adjustment of the groove of the tablet guide path 8b is displayed on the work support screen P21, similarly to the height adjustment. Since the height adjustment has been completed, the control part 210 causes the characters "OK" to be displayed in large size in the display region A124 as shown in FIG. 42. Further, the display region A124 may be grayed out.

Further, when the adjustment of the groove depth of the tablet guide path 8b in step S34 is completed, in the subsequent step S35, a process for supporting the adjustment of the groove width of the tablet guide path 8b is executed. At this time, the user inserts the central shaft 155 of the adjusting member 102 from the upper guide hole 125 of the "Width"" of the device body 101 toward the lower guide hole 126 so that the engaging portion 95d of the width adjusting mechanism M4 of the rotor 8 is engaged as shown in FIG. 44(a). Then, similarly to the height adjustment, the adjusting member 102 is rotated to adjust the width of the groove of the tablet guide path 8b. Further, in step S35, as shown in FIG. 43, the adjustment status of the adjustment of the width of the groove of the tablet guide path 8b is displayed on the work support screen P21, similarly to the height adjustment. In this embodiment, since two zero point detection sensors 115 and 116 are provided for the width of the groove of the tablet guide path 8b, the control device 200 determines that zero point detection has been completed for the width of the groove of the tablet guide path 8b when the detection signals of both the zero point detection sensor 115 and the zero point detection sensor 116 are ON. On the other hand, as another embodiment, only one zero point detection sensor 115 or 116 may be provided corresponding to the width of the groove of the tablet guide path 8b.

Then, when the operation key K211 is operated on the work support screen P11 for width adjustment displayed corresponding to the last work process included in the dimension adjusting process, the control part 210 causes the processing to proceed to step S33.

In this way, the user operates the adjusting part of the rotor 8 using the adjusting member 102 while referring to the work support screen P21, so that the user can easily perform work for adjusting the dimension of the groove of the tablet guide path 8b of the rotor 8.

### <Step S36>

In step S36, the control part 210 displays on the display part 201 a work support screen P31 for supporting the rotor return process for returning the rotor 8 to the medicine cassette 2 by the user in the process group.

As shown in FIG. 45, the work support screen P31 displays a display region A311 in which characters, images, photographs, moving images and the like indicating the content of the work process included in the rotor return process are displayed, a display region A312 showing the progress of the work process in the rotor return process and the like are displayed.

Further, on the work support screen P31, an operation key K311 and an operation key K312 for advancing the work process in the rotor return process are displayed, and the control part 210 advances one work process in the rotor return process displayed on the work support screen P11 according to the operation of the operation key K311 or the operation key K312. An operation key K313 for returning the work process in the rotor return process is also displayed on the work support screen P31, and the control part 210 moves back one work process in the rotor return process displayed on the work support screen P31 in response to the operation of the operation key K313. Then, when the operation key K311 is operated on the work support screen P31 displayed corresponding to the last work process included in the rotor return process, the control part 210 shifts the processing to step S37.

As a result, the user can sequentially refer to the display in the display region A311 and easily execute the rotor return process for returning the rotor 8 to the medicine cassette 2. For example, the rotor returning process includes a step of removing the rotor 8 from the rotor table 108 of the adjusting device 100, a step of attaching the rotor cover 30 to the rotor 8, a step of attaching the rotor 8 to the medicine cassette 2, a step of closing the lid 6 of the medicine cassette 2, a step of closing the partition member 20 by operating the partition adjusting mechanism M1 of the medicine cassette 2 and a step of placing the medicine cassette 2 on the filling table 1a of the medicine dispensing device 1.

Specifically, in the process of operating the partition adjusting mechanism M1 of the medicine cassette 2 to close the partition member 20, the user rotates the partition adjusting portion 24 of the partition adjusting mechanism M1 in the approach direction with the hexagonal wrench 149d of the grip portion 147 of the adjusting member 102 until the partition member 20 comes into contact with the rotor 8 and bends. Subsequently, the user turns the partition adjusting part 24 in the backward direction and stops rotating at a position where the partition member 20 is slightly separated from the rotor 8. Then, the user rotates the drive gear 14 of the medicine cassette 2 to rotate the rotor 8 and confirms that the partition member 20 does not hit the rotor 8.

### <Step S37>

In step S37, the control part 210 collates the information of the RFID tag 206 of the medicine cassette 2 placed on the filling table 1a. Specifically, the control part 210 controls the data recording part 305 and reads information of "cassette ID" stored in the cassette information D22 as the unique identification information of the medicine cassette 2 from the RFID tag 206 of the medicine cassette 2 placed on the filling table la. Then, the control part 210 collates the information of the "cassette ID" read from the RFID tag 206 with the "cassette ID", which is the unique identification information of the medicine cassette 2 to be allocated this time, stored in the storage unit 202 in step S23. Here, when the collation result matches, the control part 210 causes the processing to proceed to step S26, and when the collation result does not match, the control part 210 executes a predetermined error notification process. This prevents information from being written to the RFID tag 206 of the wrong medicine cassette 2 in the next step S38.

### <Step S38>

In step S38, the control part 210 uses the data recording part 305 to store information such as the medicine code, stability factor, arrangement restriction, size type, branch number and model number corresponding to the allocated tablet selected in step S21 in the medicine allocation information D21 of the writable region 261 of the RFID tag 206 of the medicine cassette 2. At this time, the dispensing confirmation flag in the medicine allocation information D21 is the initial value of "1" and will be updated to "0" in step S56 which will be described later.

As a result, by referring to the medicine allocation information D21, for example, when the medicine code is stored and the dispensing confirmation flag is "1", the control part 210 can specify that the medicine cassette 2 having the RFID tag 206 storing the medicine allocation information D21 is in a state after the manual adjustment work is completed and before execution of a test dispensing process described later. On the other hand, by referring to the medicine allocation information D21, for example, when the medicine code is not stored, the control part 210 can identify that the medicine cassette 2 having the RFID tag 206 storing the medicine allocation information D21 has not completed the manual adjustment work. Further, by referring to the medicine allocation information D21, for example, when the medicine code is stored and the dispensing confirmation flag is "0", the control part 210 can specify that the medicine cassette 2 having the RFID tag 206 storing the medicine allocation information D21 is in a state after the manual adjustment work is completed and after the test dispensing process described later is executed.

### <Step S39>

In step S39, the control part 210 updates the medicine identification information corresponding to the medicine cassette 2 in the allocation correspondence information D31 to the medicine identification information corresponding to the allocated tablet. In this way, in steps S38 and S39, the control part 210 allocates the allocated tablet to the medicine cassette 2 by associating the medicine cassette 2 with the allocated tablet. Note that steps S38 and S39 are executed by the allocation processing part 211 of the control part 210.

### <Step S40>

In step S40, the control part 210 determines whether or not the setting for outputting the label L1 including the verification code in the adjustment support processing is valid. For example, the control part 210 can enable or disable the output setting of the label L1 in the adjustment support processing in the initial setting of the medicine dispensing device 1 according to the user's operation. For example, the collation code includes medicine identification information such as the medicine code or medicine name corresponding to the allocated tablet and the cassette number as unique identification information of the medicine cassette 2. Here, when it is determined that the output setting for the label L1 is valid (S40: Yes), the processing proceeds to step S41. Further, when it is determined that the output setting for the label L1 is not valid (S40: No), the processing proceeds to step S401.

### <Step S41>

In step S41, the control part 210 executes a code output processing for outputting label information corresponding to the label L1 including the verification code and shifts the processing to step S42. In addition to the collation code, the label information includes characters indicating medicine identification information such as the medicine code or medicine name corresponding to the allocated tablet, characters indicating the cassette number as unique identification information of the medicine cassette 2 and other information such as an appearance image of the tablet corresponding to the allocated tablet. Note that step S40 is executed by the code output processing part 215 of the control unit 210.

Specifically, in step S41, the control part 210 executes the code output process according to the user's operation. For example, the control part 210 causes the display part 201 to display a print screen P41 shown in FIG. 46. The print screen P41 includes a region A411 in which a print preview of the label L1 is displayed and a region A412 in which a print key K41 for accepting user operations and the like are displayed. Then, when the print key K41 displayed on the print screen P41 is operated by the user, the label information is sent to a printer or a label printing device (not shown) connected to the control device 200 to print the label L1. For example, the label information includes two labels L1 indicating the collation code, and each of the labels L1 is inserted into the pockets 6a and 7a of the medicine cassette 2 or is attached to the front or side surface and top surface (lid 6) of the medicine cassette 2. The label information may include one or three or more labels L1 indicating the collation code. Further, the output form of the label information is not limited to printing and may be transmission output to a communication terminal using e-mail, SNS or the like.

### <Step S401>

In step S401, the control part 210 pops up an adjustment completion screen P42 indicating that the manual adjustment work has been completed on the allocation start screen P1 displayed in step S22 and shifts the processing to step S42. As shown in FIG. 47, the adjustment completion screen P42 displays that the manual adjustment work has been completed and that the rotor 8 can be removed. Then, when a user operation for closing the adjustment completion screen P42 is performed, the control part 210 shifts the processing to step S42.

### <Step S42>

As shown in FIG. 35, in step S42, following the manual adjustment work, the control part 210 determines whether or not to execute a test dispensing operation for dispensing tablets from the medicine cassette 2 after being adjusted by the manual adjustment work. Here, when it is determined that the test dispensing operation is to be executed continuously (S42: Yes), the processing proceeds to step S43. Further, when it is determined that the test dispensing operation is not to be executed continuously (S42: No), the adjustment support processing is terminated.

For example, as shown in FIGs. 48 and 49, the control part 210 causes the display part 201 to display a selection screen P51 for allowing the user to select whether or not to continue the test dispensing operation. The selection screen P51 displays an operation key K511 for selecting the subsequent execution of the test dispensing operation and an operation key K512 for selecting the subsequent execution of the test dispensing operation. Then, when the operation key K511 is operated, the control part 210 continuously displays the outline of the user's work necessary for executing the test dispensing operation in the form of characters, images, photographs, videos or the like as shown in FIG. 48. On the other hand, when the operation key K512 is operated, the control part 210 displays the outline of the user's work necessary for executing the test dispensing operation later in the form of characters, images, photographs, videos or the like as shown in FIG. 49.

### <Step S43>

In step S43, the control part 210 controls the data recording part 305 to read the medicine allocation information D21 and the cassette information D22 from the RFID tag 206 of the medicine cassette 2 placed on the filling table 1a and stores the information in the storage part 202 as information on the medicine cassette 2 to be subjected to the test dispensing operation of this time. The RFID tag 206 provided in the medicine cassette 2 is an example of a recording medium on which information can be recorded. A recording medium such as an attached label on which information similar to that of the RFID tag 206 is recorded (printed) may be used.

### <Step S44>

In step S44, the control part 210 determines whether or not the code reading part 304 has read the collation code included in the label L1. For example, the control part 210 causes the display part 201 to display a work support screen P61 that displays characters, images, photographs, moving images or the like for prompting the reading of the collation code using the code reading part 304. In addition, based on the information of the dispensing confirmation flag of the medicine allocation information D21, the control part 210 causes the display region A611 in the work support screen P61 to display the presence or absence of the dispensing confirmation information indicating that the tablet has been dispensed in the test dispensing operation ("verified", "unverified", etc.). The work support screen P61 is displayed by the display processing part 218 of the control part 210.

Here, when the user uses the code reading part 304 to read the collation code included in the label L1, in step S44, it is determined that the collation code has been read (S44: Yes), and the processing proceeds to step S45. Until the collation code is read (S44: Yes), the processing waits in step S44.

### <Step S45>

In step S45, the control part 210 collates the medicine allocation information D21 read from the RFID tag 206 in step S43 with the collation code read from the label L1 in step S44. Specifically, the control part 210 collates the cassette number included in the medicine allocation information D21 with the cassette number included in the collation code read from the label L1 in step S44. Then, when the collation result matches, the control part 210 shifts the processing to step S46, and when the collation result does not match, the control part 210 executes a predetermined error notification. Note that, when the collation result matches, the control part 210 displays the collation result in an identifiable manner on the work support screen P61.

### <Step S46>

In step S46, the control part 210 uses the code reading part 304 to determine whether or not the medicine identification information has been read from the code attached to the medicine box containing the allocated tablets. For example, the control part 210 causes the display part 201 to display characters, images, photographs, moving images or the like for prompting reading of the code of the tablet corresponding to the allocated tablet using the code reading part 304. Thereby, the user uses the code reading part 304 to read the medicine identification information from the code attached to the medicine box containing the allocated tablets. When it is determined that the medicine identification information has been read (S46: Yes), the processing proceeds to step S47, and until the medicine identification information is read (S46: Yes), the processing waits in step S46.

### <Step S47>

In step S47, the control part 210 collates the allocated medicine information D21 read from the RFID tag 206 in step S43 with the medicine identification information read in step S46. Specifically, the control part 210 collates the medicine identification information such as the medicine code included in the medicine allocation information D21 with the medicine identification information read in step S47. Then, when the collation result matches, the control part 210 causes the processing to proceed to step S48, and when the collation result does not match, control part 210 executes a predetermined error notification. Note that, when the collation result matches, the control part 210 displays the collation result in an identifiable manner on the work support screen P61.

### <Step S48>

In step S48, the control part 210 accepts input of one or more of the number of tablets to be filled in the medicine cassette 2, the filling date or the expiration date on the work support screen P61. That is, the control part 210 can receive input of information regarding tablets to be filled in the medicine cassette 2 before the test dispensing operation for the medicine cassette 2 is started. Note that step S48 is executed by reception processing part 216 of control unit 210. Then, when a registration key K61 displayed on the work support screen P61 is operated, the control part 210 shifts the processing to step S49. Further, when the registration key K61 is operated, the control part 210 causes the processing proceeds to step S49 even if the control part 210 has not received inputs such as the number of tablets to be filled in the medicine cassette 2, the date of filling or the expiration date.

### <Step S49>

In step S49, the control part 210 displays on the display part 201 a work support screen P71 for supporting the test dispensing process performed by the user when executing the test dispensing operation among the process groups. In particular, in step S49, the work support screen P71 is displayed for supporting the first test dispensing step performed by the user before the execution of the test dispensing operation among the test dispensing steps. Among the test dispensing steps, the work support screen P71 for supporting a second test dispensing step performed by the user after execution of the test dispensing operation is displayed in step S54, which will be described later.

As shown in FIG. 51, the work support screen P71 displays a display region A711 in which characters, images, photographs, moving images or the like indicating the contents of the work process included in the test dispensing process are displayed, a display region A712 showing the progress of the work process in the test dispensing process and the like.

Further, on the work support screen P71, an operation key K711 and an operation key K712 for advancing the work process in the test dispensing process are displayed, and the control part 210 advances one work process in the test dispensing process displayed on the work support screen P71 according to the operation of the operation key K711 or the operation key K712. The work support screen P71 also displays an operation key K713 for returning the work process in the test dispensing process, and the control part 210 moves back one work process in the test dispensing process displayed on the work support screen P71 in response to the operation of the operation key K713. Then, on the work support screen P71 displayed corresponding to the last work process included in the test dispensing process, the control part 210 shifts the processing to step S50 when the operation key K711 is operated.

Thus, the user can sequentially refer to the display of the display region A711 and easily execute the first test dispensing step performed by the user before executing the test dispensing operation. For example, the first test dispensing step includes a step of attaching a label L1 seal to the medicine cassette 2, a step of reading the collation code of the label L1 attached to the medicine cassette 2 with the code reading unit 304, a step of reading a code attached to a medicine box or the like in which the allocated tablets are stored, a step of filling the medicine cassette 2 with tablets corresponding to the allocated tablets and a step of mounting the medicine cassette 2 to the medicine dispensing device 1.

### <Step S50>

In step S50, the control part 210 causes the medicine dispensing device 1 to execute the test dispensing operation, and when the test dispensing operation is completed, the processing proceeds to step S51. Specifically, the control part 210 inputs test dispensing data preset for executing the test dispensing operation to the medicine dispensing device 1 and causes the medicine dispensing device 1 to perform the test dispensing operation based on the test dispensing data. Note that the processing of step S50 is executed by the determination processing part 212 of the control part 210.

The test dispensing data is data for dispensing a preset specific number of the allocated tablets from the medicine cassette 2. In particular, the test dispensing data may be data for packaging the specific number of the allocated tablets in a predetermined specific number of medicine packaging papers. For example, each of the specific number and the specific number of packages is a preset fixed value, and as for the types of tablets to be dispensed in the test dispensing operation, the allocated tablets are set by the control part 210 for each adjustment support processing.

At this time, in the medicine dispensing device 1, the number of allocated tablets indicated in the test dispensing data is dispensed from the medicine cassette 2, and a packaging processing is executed for packaging in one or a plurality of medicine wrapping papers. For example, the allocated tablets are divided into two medicine wrapping papers each containing five tablets. Further, in the medicine dispensing device 1, when the packaging process is executed, the number of tablets dispensed from the medicine cassette 2 is counted by the dispensing detector 303, a photograph of the tablet dispensed from the medicine cassette 2 is taken, and the number of tablets and the photograph of the tablets are transmitted to the control device 200. The photograph of the tablet taken by the dispensing detection part 303 may be taken for each tablet or may be taken for each medicine-wrapping paper after being wrapped with the medicine-wrapping paper.

### <Step S51>

In step S51, the control part 210 displays on the display part 201 a test dispensing result screen P81 showing the result of the test dispensing operation. As shown in FIGs. 52 and 53, a photographed image F811 photographed by the dispensing detection part 303 in the medicine dispensing device 1 in the test dispensing operation, a display region A811 in which information such as the adequacy of the number of tablets to be dispensed detected by the dispensing detection part 303 in the test dispensing operation is displayed, and the like are displayed on the test dispensing result screen P81. For example, FIG. 52 shows a display example of the test dispensing result screen P81 when the test dispensing operation is performed normally, and FIG. 53 shows a display example of the test dispensing result screen P81 when the test dispensing operation is not performed normally.

Note that the control part 210 may count the number of tablets to be dispensed in the test dispensing operation based on the photographed image F811 photographed by the dispensing detection part 303. Further, based on the photographed image F811, the control part 210 determines whether or not the type of tablets actually dispensed in the test dispensing operation is the assigned tablet and may display the determination result on the test dispensing result screen P81.

### <Step S52>

In step S52, the control part 210 determines whether or not the tablets have been correctly dispensed from the medicine cassette 2 by the test dispensing operation. Note that the processing of step S52 is an example of a determination step executed by the determination processing part 212 of the control part 210.

Specifically, the test dispensing result screen P81 displays an operation key K811 that is operated when the user confirms that there is no problem with the result of the test dispensing operation, and in step S52, the control part 210 determines whether or not the operation key K811 has been operated. That is, the control part 210 determines that the tablets have been properly dispensed from the medicine cassette 2 on condition that a specific user operation has been performed after the test dispensing operation for the medicine cassette 2 has been performed.

Here, when it is determined by the test dispensing operation that the tablets have been correctly dispensed from the medicine cassette 2 (S52: Yes), the processing proceeds to step S53. Further, when it is determined by the test dispensing operation that the tablets have not been correctly dispensed from the medicine cassette 2 (S52: No), the processing proceeds to step S521. The display of the photographed image F811 on the test dispensing result screen P81 may be omitted, and the user may visually confirm the tablets actually dispensed from the medicine dispensing device 1 and operate the operation key K811.

As another embodiment, the control part 210 automatically determines whether or not the number of tablets actually dispensed in the test dispensing operation is the specific number based on the detection result by the dispensing detection part 303, and the control part 210 may determine that the tablets have been correctly dispensed from the medicine cassette 2 by the test dispensing operation on condition that the determination results match.

Further, as another embodiment, the control part 210 automatically determines whether or not the type of tablet actually dispensed in the test dispensing operation is the allocated tablet based on the photographed image F811, and the control part 210 may determine that the tablets have been correctly dispensed from the medicine cassette 2 by the test dispensing operation on the condition that the determination results match.

Furthermore, as another embodiment, the control part 210 may determine that the tablets have been correctly dispensed from the medicine cassette 2 by the test dispensing operation on condition that the test dispensing operation for the medicine cassette 2 has been completed.

### <Step S521>

In step S521, the control part 210 determines whether or not the user has requested to redo the test dispensing operation. Specifically, the test dispensing result screen P81 displays an operation key K812 operated by the user when redoing the test dispensing operation, and in step S521, the control part 210 determines whether or not an operation key K812 has been operated. Here, when it is determined that a request to redo the test dispensing operation has been made (S521: Yes), the processing proceeds to step S522. Further, when it is determined that the request to redo the test dispensing operation has not been made (S521: No), the processing proceeds to step S524.

### <Steps S522 to S523>

In steps S522 and S523, the control part 210 accepts selection of a method for redoing the test dispensing operation.

Specifically, the control part 210 displays a selection screen P811 for accepting the selection of a method for redoing the test dispensing operation on the display part 201 on the test dispensing result screen P81. As shown in FIG. 54, the selection screen P811 displays an operation key K814 operated by the user when restarting from the test dispensing operation and an operation key K815 operated by the user when restarting from the manual adjustment operation.

Then, when the operation key K814 is operated, the control part 210 determines to redo the test dispensing operation (S522: Yes) and shifts the processing to step S50. If the test dispensing operation can be re-executed at least without re-executing the manual adjustment work, the destination of the processing is not limited to step S50.

On the other hand, when the operation key K815 is operated, the control part 210 determines to redo the manual adjustment work (S523: Yes) and shifts the processing to step S21. Incidentally, when at least the manual adjustment work and the test dispensing operation can be re-executed, the destination of the process is not limited to step S21.

### <Step S524>

In step S524, the control part 210 determines whether or not to terminate the adjustment support processing due to an abnormality in the test dispensing operation. Specifically, the test dispensing result screen P81 displays an operation key K813 that is operated by the user when the adjustment support processing is terminated due to an abnormality in the test dispensing operation, and in step S524, the control part 210 determines whether or not the operation key K813 has been operated. Here, when it is determined that the adjustment support processing should be ended halfway (S524: Yes), the processing proceeds to step S525. Further, when it is not determined to end the adjustment support processing halfway (S524: No), the processing returns to step S52. In addition, when the test dispensing operation is not performed normally even if the test dispensing operation is performed a preset number of times, the control part 210 may automatically determine to end the adjustment support processing halfway (S54: Yes).

### <Step S525>

In step S525, the control part 210 displays an error notification screen P812 as shown in FIG. 54, for example, on the display part 201 to notify the user of the error. For example, in the error notification, a message such as termination of the adjustment support processing due to an abnormality in the test dispensing operation is notified. Then, when a close key K816 displayed on the error notification screen P812 is operated, the control part 210 closes the error notification screen P812 and ends the adjustment support processing. Noted that the control part 210 may store the contents of the medicine allocation processing and the adjustment support processing in the storage unit 202 as history information when ending the medicine allocation processing and the adjustment support processing, respectively.

Specifically, in the test dispensing operation, It is conceivable that the control part 210 causes the error notification screen P812 to display that the tablet cannot be dispensed in the case where the dispensing detection part 303 does not detect any tablets dispensed from the medicine cassette 2 when a specific number of tablets are dispensed from the medicine cassette 2. Further, in the test dispensing operation, when dispensing operation of a specific number of tablets from the medicine cassette 2 is executed, the control part 210 determines the number of tablets dispensed from the medicine cassette 2 and detected by the dispensing detection part 303, and when the number of tablets is different from the specific number, the control part 210 may display an error indicating that the number of dispensed tablets is different (excess or shortage, etc.) on the error notification screen P812. Note that the control part 210 may display a message, an image or the like for informing the user of one or a plurality of check locations or check details preset according to the content of the error on the error notification screen P812.

### <Step S53>

When the user confirms that the result of the test dispensing operation is normal in step S52 (S52: Yes), in subsequent step S53, the control part 210 displays on display part 201 an approval screen P82 for receiving approval from the user. As shown in FIG. 56, the approval screen P82 displays the cassette number of the medicine cassette 2 adjusted in the current adjustment support processing and the medicine name of the allocated tablet, and the name of the user in charge of adjusting the medicine cassette 2 by the adjustment support processing can be input to the approval screen P82. After the user name is input, the control part 210 shifts the processing to step S54 when the operation key K821 displayed on the approval screen P82 is operated.

### <Step S54>

In step S54, the control part 210 displays the work support screen P71 for supporting the second test dispensing process performed by the user after the test dispensing operation is executed among the test dispensing processes. For example, the second test dispensing step includes a step of removing the medicine cassette 2 from the medicine dispensing device 1 and a step of placing the medicine cassette 2 on the filling table 1a of the medicine dispensing device 1. Since the work support screen P71 is the same as the work support screen P71 displayed in step S49 before execution of the test dispensing operation, detailed description thereof will be omitted, but for example, FIG. 57 shows the work support screen P71 on which a process of removing the medicine cassette 2 from the medicine dispensing device 1 is displayed. Thus, the user can sequentially refer to the display of the display region A711 and easily execute the second test dispensing step that the user performs after executing the test dispensing operation.

### <Step S55>

In step S55, the control part 210 collates the information of the RFID tag 206 of the medicine cassette 2 placed on the filling table 1a. Specifically, the control part 210 controls the data recording part 305 and reads information of "cassette ID" stored in the cassette information D22 as the unique identification information of the medicine cassette 2 from the RFID tag 206 of the medicine cassette 2 placed on the filling table 1a. Then, the control part 210 collates the information of the "cassette ID" read from the RFID tag 206 with the "cassette ID", which is the unique identification information of the medicine cassette 2 to be adjusted this time, stored in the storage part 202 in step S23. Then, when the collation result matches, the control part 210 shifts the processing to step S56, and when the collation result does not match, the control part 210 executes a predetermined error notification process. This prevents information from being written to the RFID tag 206 of the wrong medicine cassette 2 in the next step S56.

### <Step S56>

In step S56, the control part 210 uses the data recording part 305 to store in the RFID tag 206 of the medicine cassette 2 the dispensing confirmation information indicating that the test dispensing operation for the medicine cassette 2 has been completed. Specifically, the control part 210 updates the dispensing confirmation flag included in the medicine allocation information D21 in the writable region 261 of the RFID tag 206 from "1" to "0". Note that step S56 is an example of a storing step executed by the storage processing part 213 of the control part 210. In addition, in the present embodiment, a case in which approval by the user (S53) and collation of information of the RFID tag 206 (S55) are executed will be described as an example, but as another embodiment, the storing step of step S56 may be executed when the user confirms that the result of the test dispensing operation is normal in step S52.

### <Step S57>

In step S57, the control part 210 updates the medicine identification information corresponding to the medicine cassette 2 in the allocation correspondence information D31 to the medicine identification information corresponding to the allocated tablet selected in step S21. As a result, in the medicine dispensing device 1, the control part 210 can identify the correspondence relationship between the allocated tablets and the medicine cassettes 2 based on the allocation correspondence information D31. Note that the control part 210 may execute only one of the steps S39 and S57. Further, the control part 210 may provisionally register the correspondence between the cassette number of the medicine cassette 2 and the allocated tablet in step S39 and confirm the provisional registration as the main registration in step S57. Furthermore, in step S39, the control unit 210 may register the cassette number of the medicine cassette 2 in the allocation correspondence information D31 and register the medicine code of the allocated tablet in association with the cassette number in step S57.

In step S57, the control part 210 may store the dispensing confirmation information in the allocation correspondence information D31 in association with the cassette identification information such as the cassette IDs of the allocated tablets and the medicine cassette 2. Then, the control part 210 may limit dispensing based on the prescription data for the types of tablets and medicine cassettes 2 for which the dispensing confirmation information is not stored in the allocation correspondence information D31.

As described above, in the medicine dispensing system 500, by executing the medicine allocation processing and the adjustment support processing, it is possible to support the user in allocation work of the types of tablets to the medicine cassette 2 and in the manual adjustment work of the medicine cassette 2, thereby improving the work efficiency of the user.

Further, in the medicine dispensing system 500, in the adjustment support processing, a list of a plurality of work process groups included in the manual adjustment work using the adjusting part is displayed, and then one or more work processes included in each of the work process groups are displayed in order. Note that such processing is an example of the display step executed by the display processing part 218 of the control part 210. Thereby, the user can grasp the contents of each work process group to be performed in the entire manual adjustment work before starting the work process in each of the plurality of work process groups.

### [Medicine dispensing processing]

Next, with reference to FIG. 58, an example of the procedure of the medicine dispensing processing will be described. The medicine dispensing processing is an example of a dispensing step executed by dispensing processing part 220 of the control part 210 of the control device 200. Note that the medicine dispensing processing may be executed by the control part 310 of the medicine dispensing device 1.

### <Step S61>

In step S61, the control part 210 determines whether or not the operation part 203 has been operated to start dispensing for specific prescription data. For example, when arbitrary prescription data is selected by a user operation as a dispensing target from prescription data input to the control device 200 from a host system such as an electronic medical record system (not shown) and an operation for starting dispensing of the prescription data is performed on the operation part 203, the control part 210 determines that a dispensing start operation has been performed. Here, when it is determined that the dispensing start operation has been performed (S61: Yes), the processing proceeds to step S62. Further, until it is determined that the dispensing start operation has been performed (S61: No), the processing waits in step S61.

### <Step S62>

In step S62, the control part 210 identifies the control target for dispensing the tablet included as the prescription medicine in the prescription data based on the allocation correspondence information D31. Specifically, the control part 210 identifies the medicine cassette 2 corresponding to the type of tablet to be dispensed (drug code) based on the allocation correspondence information D31. Further, based on the base master D41, the control part 210 identifies the base 3 on which the identified medicine cassette 2 is mounted. When the prescription data includes a plurality of types of tablets as prescription medicines, a plurality of control targets for dispensing each of the tablets is specified. Then, when control targets for dispensing all tablets are identified, the processing proceeds to step S63.

Further, in step S62, the control part 210 identifies the manual dispensing unit 302 as a control target for tablets that cannot be dispensed from the medicine cassette 2 by the medicine dispenser 1 among the tablets included in the prescription data as prescription medicines. After that, the control part 210 causes the display part 201 to display a display screen or the like for supporting the loading of the tablets into the respective DTA cells of the manual dispensing unit 302. In the medicine dispensing operation to be described later, the tablets dispensed from the manual dispensing unit 302 are also packaged together with the tablets dispensed from the medicine cassette 2 into packaging papers corresponding to each dosing time.

### <Step S63>

In step S63, the control part 210 determines whether the test dispensing operation has already been completed for each of the medicine cassettes 2 specified in step S62. Specifically, the control part 210 controls the data reading part 306 provided on the base 3 on which each medicine cassette 2 is mounted and reads the allocated medicine information D21 from the RFID tag 206 of each medicine cassette 2 specified in step S62. Then, when the dispensing confirmation flag stored in the allocated medicine information D21 read from the medicine cassette 2 is "0", the control part 210 determines that the test dispensing operation for the medicine cassette 2 has already been completed.

Here, when it is determined that the test dispensing operation has already been completed (S63: Yes), the processing proceeds to step S67, and when it is determined that the test dispensing operation has not been completed yet (S63: No), the processing proceeds to step S64. In addition, in the configuration in which the dispensing confirmation information is stored in the storage part 202 for each of the medicine cassettes 2, the control part 210 may determine whether or not the test dispensing operation has already been completed based on the dispensing confirmation information stored in the storage part 202.

In the medicine dispensing processing, the control part 210 identifies the medicine cassette 2 to which the tablet type (medicine code) included in the prescription data as the prescribed medicine is allocated in step S62. Therefore, in step S63, when the type of tablet (medicine code) included as the prescribed medicine in the prescription data is stored in the medicine allocation information D21 and the dispensing confirmation flag is "1", it is determined that the medicine cassette 2 corresponding to the medicine allocation information D21 is in a state after the completion of the manual adjustment work and before execution of the test dispensing process (S63: Yes), and the processing is shifted to step S64.

### <Step S64>

In step S64, after the control part 210 displays a message on the display part 201 to the effect that the test dispensing operation of the medicine cassette 2 has not been completed to notify the user and executes processing for starting the test dispensing operation, the control part 210 shifts the process to step S65. For example, the control part 210 causes the display part 201 to display a display screen for inquiring whether or not to execute the test dispensing operation for the medicine cassette 2 for which the test dispensing operation has not been completed and receives an execution requesting operation of the test dispensing operation on the display screen. Then, when the execution requesting operation is received, the control part 210 executes the test dispensing operation by executing the processing after step S43 of the adjustment support processing. Note that the control part 210 can execute the medicine dispensing processing in parallel with other processes such as the medicine allocation processing or the adjustment support processing.

Further, the control part 210 can select the medicine cassette 2 to be subjected to the test dispensing operation at an arbitrary timing such as before the start of the medicine dispensing process, not limited to the timing when the medicine dispensing process is executed, according to the user's operation and also execute the test dispensing operation for the medicine cassette 2. For example, based on the medicine allocation information D21 of each medicine cassette 2, the control part 210 searches for the type of tablet or the medicine cassette 2 for which the test dispensing operation has not been completed and displays it as a selection candidate, and when any of the selection candidates is selected by the user's operation, the control part 210 starts the test dispensing operation for the medicine cassette 2 corresponding to the selection candidate.

### <Step S65>

In step S65, the control part 210 waits for completion of the test dispensing operation for the medicine cassette 2 for which the test dispensing operation has not been completed (S65: No). Specifically, the control part 210 determines that the test dispensing operation has been completed when the dispensing confirmation information is stored in the RFID tag 206 of the medicine cassette 2 for which the test dispensing operation has not been completed. Here, when it is determined that the test dispensing operation is completed (S65: Yes), the processing proceeds to step S66. On the other hand, until the test dispensing operation is completed (S65: No), the control part 210 causes the processing to wait in step S65, thereby prohibiting execution of the medicine dispensing operation in step S66, which will be described later. Thus, the control part 210 restricts the medicine dispensing operation when the dispensing confirmation information corresponding to the medicine cassette 2 is not stored in the RFID tag 206 .

As another embodiment, when a user operation to permit execution of the medicine dispensing operation is performed after the control part 210 notifies the user that the test dispensing operation has not been completed for any of the medicine cassettes 2 specified in step S62, the control part 210 may shift the processing to step S66 to start the medicine dispensing operation. In this case, the control part 210 also restricts execution of the medicine dispensing operation until the user's operation is performed.

### <Step S66>

In step S66, the control part 210 transmits to the medicine dispensing device 1 a medicine dispensing start instruction for causing the medicine dispensing device 1 to execute a medicine dispensing operation of dispensing tablets from each of the medicine cassettes 2 identified in step S62 and returns the processing to step S61. Specifically, the medicine dispensing start instruction includes information such as the cassette ID of the medicine cassette 2 specified based on the prescription data, the stability factor corresponding to the type of tablet allocated to the medicine cassette 2 and the number of tablets dispensed corresponding to the prescribed amount indicated in the prescription data. Accordingly, in the medicine dispensing device 1, the dispensing execution processing part 311 of the control part 310 dispenses the number of tablets to be dispensed from each of the medicine cassettes 2 based on the medicine dispensing start instruction. Further, when it is necessary to use the manual dispensing unit 302 in the medicine dispensing operation based on the prescription data, the control part 210 transmits the medicine dispensing start instruction including information on the tablets put into each of the DTA cells of the manual dispensing unit 302. As a result, the medicine dispensing device 1 can appropriately dispense tablets from the manual dispensing unit 302 in the medicine dispensing operation based on the medicine dispensing start instruction.

As described above, in the medicine dispensing system 500, by executing the medicine dispensing process, execution of the medicine dispensing operation using the medicine cassette 2 for which the test dispensing process has not been performed is suppressed. As a result, for example, a problem such as not dispensing the required number of tablets in the medicine dispensing operation can be prevented.

### [Other embodiments]

In the adjusting device 100 according to the above embodiment, the execution order of the height adjusting process, the depth adjusting process and the width adjusting process is predetermined, and by displaying the execution order, an optical sensor or the like for detecting whether the adjusting member 102 is inserted from the upper guide hole 125 toward the lower guide hole 126 corresponding to height, depth or width can be omitted. On the other hand, the adjusting device 100 may be provided with an insertion detection part such as an optical sensor for detecting insertion of the adjusting member 102 for each guide hole 125 corresponding to an adjustment position (height, depth, width) where the adjustment member 102 is inserted. Accordingly, the control part 210 can recognize the insertion position of the adjusting member 102, that is, the adjustment position, based on the detection result of the insertion detection part. Therefore, for example, the control part 210 may select which of the height adjusting process, the depth adjusting process, and the width adjusting process is to be executed according to the detection result of the insertion detection part. Therefore, there is no need to predetermine the adjustment order of the adjustment points, or the need for the user to select the adjustment points, and a user can perform the height adjusting process, the depth adjusting process, and the width adjusting process in any order.

The adjusting device 100 according to the above embodiment has a configuration in which the depth, height and width of the groove of the tablet guide path 8b can be adjusted, but as another embodiment, one or both of the depth, height and width of the groove may be adjustable.

The adjusting device 100 according to the above embodiment is configured such that the user manually adjusts the dimensions of the groove of the tablet guide path 8b using the adjusting member 102, but as another embodiment, the adjusting device 100 or the adjusting member 102 may be provided with a driving section such as a motor and a battery that supplies power to the driving section and the grip section 147 may be rotated by the driving force of the driving section.

In the medicine delivery system 500 according to the above embodiment, the rotor 8 is adjusted after being taken out from the medicine cassette 2, but as another embodiment, the configuration may be such that the dimensions of the groove of the tablet guide path 8b of the rotor 8 can be adjusted while the rotor 8 is attached to the medicine cassette 2. For example, with the lid 6 of the medicine cassette 2 opened and the rotor cover 30 removed, at least one of the depth, height and width of the groove of the tablet guide path 8b of the rotor 8 in the medicine cassette 2 or the entry position of the partition member 20 may be adjusted by the adjusting member 102.

As another configuration of the medicine dispensing device 1, each base 3 on which the medicine cassette 2 is mounted may be provided with an adjusting part capable of automatically adjusting the dispensing path in the medicine cassette 2. With such a configuration, after the medicine cassette 2 is attached to the base 3, the dispensing path of the medicine cassette 2 can be automatically adjusted by the adjusting part. As a result, human error, such as mistake of adjustment or forgetting to adjust the dispensing path of the medicine cassette 2, or erroneous mounting after adjusting the dispensing path of the medicine cassette 2, is less likely to occur. However, in the medicine dispensing system 500 according to the present embodiment, the adjusting device 100 for manually adjusting the dispensing path of the medicine cassette 2 by the user is provided separately from the medicine dispensing device 1, and the medicine dispensing device 1 is not provided with an adjusting part capable of automatically adjusting the dispensing path in the medicine cassette 2 on each base 3. Therefore, in the medicine dispensing system 500, the test dispensing operation is executed before executing the medicine dispensing operation based on the prescription data as described above. As a result, in the medicine delivery system 500, the occurrence of human errors such as mistake of adjustment, forgetting to adjust, and incorrect mounting of the medicine cassette 2 described above is suppressed.

### [Work support display function]

By the way, even if the user does not have tablets at hand, such as tablets that are not in stock at the facility where the medicine dispensing system 500 is used or new drugs that have not arrived, from the viewpoint of work efficiency, it is assumed that the type of tablet is allocated to the medicine cassette 2 and the manual adjustment work is to be performed on the medicine cassette 2 before the tablet arrives.

Therefore, the medicine dispensing system 500 has a work support display function in which the control part 210 searches for the types of tablets or medicine cassettes 2 for which the test dispensing operation has not been completed and displays the search results as selection candidates for execution of the test dispensing operation. Then, the control part 210 executes the test dispensing operation for the tablet type or the medicine cassette 2 selected from the selection candidates. Note that the control part 210 may execute the display of selection candidates for execution of the test dispensing operation according to the user's operation or may execute the display automatically at a preset specific timing. For example, the specific timing is a timing arbitrarily set by the user and stored in association with each medicine cassette 2 such as the scheduled arrival time of tablets of the type allocated to the medicine cassette 2 for which the test dispensing operation has not been performed or the scheduled execution time of performing the test dispensing operation when the manual adjustment work is performed.

Thereby, the user ends the adjustment support processing without executing the test dispensing operation when there is no tablet at hand. After that, at an arbitrary timing such as when tablets are received, it is possible to easily select the medicine cassette 2 for which the test dispensing operation is displayed by the work support display function and to execute the test dispensing operation.

Specifically, FIG. 59 is a flow chart showing an example of work support display processing executed by the control part 210 to realize the work support display function. Note that the control part 210 may execute the work support display process in parallel with other processes such as the medicine allocation processing (see FIG. 33), the adjustment support processing (see FIGs. 34 to 36), and the medicine dispensing processing (see FIG. 58). Further, part or all of the work support display processing may be executed by the control part 310 of the medicine dispensing device 1.

### <Step S71>

As shown in FIG. 59, in step S71, the control part 210 determines whether or not the user has performed an incomplete display operation to display the medicine cassette 2 for which the test dispensing operation has not been completed (corresponding to the incomplete medicine dispensing unit) on the operation unit 203. Here, when it is determined that the incomplete display operation has been performed (S71: Yes), the processing proceeds to step S72. Further, when it is determined that the incomplete display operation has not been performed (S71: No), the processing proceeds to step S711.

### <Step S72>

In step S72, the control part 210 searches for the medicine cassette 2 for which the test dispensing operation has not been completed based on the medicine allocation information D21. Specifically, the control part 210 searches for the medicine cassette 2 with the dispensing confirmation flag of "1" included in the medicine allocation information D21 as an incomplete medicine cassette 2. Here, a case where the medicine cassette 2 for which the test dispensing operation has not been completed is searched as an object to be processed will be described as an example, but instead of the medicine cassette 2, the type of tablet for which the test dispensing operation has not been completed may be retrieved and displayed as a processing target.

### <Step S73>

In step S73, the control part 210 causes the display part 201 to display an incomplete list display screen that lists the medicine cassettes 2 extracted as the search results of step S72 as selection candidates. For example, the incomplete list display screen displays the cassette number of the medicine cassette 2 for which the test dispensing operation has not been completed and the identification information (medicine code) of the tablet assigned to the medicine cassette 2. The display processing in step S73 is executed by the display processing part 218 of the control part 210.

### <Step S711>

In step S711, the control part 210 determines whether or not there is a medicine cassette 2 for which the specific timing has come. Here, when it is determined that there is a medicine cassette 2 that has reached the specific timing (S711: Yes), the processing proceeds to step S712, and when there is no medicine cassette 2 for which the specific timing has come (S711: No), the processing returns to step S71.

### <Step S712>

In step S712, the control part 210 causes the display part 201 to display a target list display screen for displaying, as selection candidates, one or more medicine cassettes 2 determined to have reached the specific timing in step S711. Note that the control part 210 may display only one or a plurality of medicine cassettes 2 determined to have reached the specific timing on the target list display screen, but as with the incomplete list display screen displayed in step S73, a list of medicine cassettes 2 for which the test dispensing operation has not yet been completed may be displayed. In addition, the control part 210 displays a list of the medicine cassettes 2 for which the test dispensing operation has not been completed at the present time, similarly to the incomplete list display screen displayed in step S73 and may display whether or not the specific timing has come for each of the medicine cassettes 2 in an identifiable manner. The display processing in step S712 is executed by the display processing part 218 of the control part 210.

### <Step S74>

In step S74, the control part 210 waits for the user's selection operation for executing the test dispensing operation for either of the medicine cassettes 2 displayed at step S73 or S712 (S74: No). Then, when the selection operation of the medicine cassette 2 is performed (S74: Yes), the test dispensation operation is executed by executing the processes after the step S43 with the medicine cassette 2 selected by the selection operation as the object to be processed. When a predetermined operation for closing the incomplete list display screen is performed, the control part 210 closes the incomplete list display screen and returns the processing to step S71.

When it is determined in step S74 that the medicine cassette 2 has been selected, the control part 210 notifies the user of the selected medicine cassette 2 in an identifiable manner. For example, the control part 210 specifies the cassette number of the selected medicine cassette 2 based on the medicine allocation information D21 of the medicine cassette 2 and causes the display unit 201 to display it. Further, when the base 3 to which the medicine cassette 2 is attached and detached is provided with a light emitting part such as an LED, the control part 210 may light or blink the light emitting part of the base 3 on which the selected medicine cassette 2 is mounted.

here, the dispensing confirmation information of the medicine allocation information D21 has been described as an example of specific information according to the present invention. On the other hand, it is conceivable that the medicine allocation information D21 includes, as an example of specific information according to the present invention, collation confirmation information indicating whether or not the collation operation in step S45 has been completed. Specifically, the collation confirmation information is a collation confirmation flag whose initial value is "1" and which is reset to "1" by the control unit 210 even when the type of tablet is assigned to the medicine cassette 2. Further, when the collation result of the label L1 in step S45 matches, the control part 210 determines that the collation operation of the label L1 is completed and sets the collation confirmation flag to "0". In this way, by setting the collation confirmation flag to "0", the control part 210 may store the fact that the collation operation for the medicine cassette 2 has been completed as the collation confirmation information, and by setting the collation confirmation flag to "1", the control part 210 may store the fact that the collation operation for the medicine cassette 2 has not been completed as the collation confirmation information. Note that the storage processing part 213 of the control part 210 executes processing for storing specific information such as the verification confirmation information in the storage part such as the RFID tag 206. Further, the collation confirmation information is not limited to the RFID tag 206 and may be stored in the storage part 202 in association with the cassette ID of the medicine cassette 2 or the cassette number and the medicine code indicating the type of medicine allocated to the medicine cassette 2, like the dispensing confirmation information. As a result, the control part 210 can determine whether or not the test dispensing operation has been completed for each of the medicine cassettes 2 based on the dispensing confirmation information and can also determine whether or not the collation operation has been completed based on the collation confirmation information.

Then, at the time of the incomplete display operation in step S71, the control part 210 may receive a selection operation to display a list of the medicine cassettes 2 (corresponding to the incomplete medicine dispensing section) for which the test dispensing operation has not been completed or to display a list of the medicine cassettes 2 for which the collation operation has not been completed. After that, when it is selected to display the list of the medicine cassettes 2 for which the test dispensing operation has not been completed at the time of the incomplete display operation, the control part 210 causes the list of the medicine cassettes 2 for which the test dispensing operation has not been completed to be displayed based on the dispensing confirmation flag in steps S72 and S73. On the other hand, when it is selected during the incomplete display operation to display the list of the medicine cassettes 2 for which the verification operation has not been completed, the control part 210 displays the list of medicine cassettes 2 for which the collation operation has not been completed based on the collation confirmation information in steps S72 and S73. The control part 210 may be able to display only one of the list of the medicine cassettes 2 for which the test dispensing operation has not been completed or the list of the medicine cassettes 2 for which the collation operation has not been completed or may be able to display both identifiably at the same time.

### [Medicine loading support function]

A user of the medicine dispensing device 1 may perform a medicine loading operation of loading a tablet into the medicine cassette 2 corresponding to the type of tablet. For example, a tablet dispensed from the medicine dispenser 1 may be returned to the medicine cassette 2. Furthermore, it is conceivable to return a tablet possessed by a patient or a tablet dispensed from another dispensing device or the like to the medicine cassette 2 of the medicine dispenser 1. Here, when the user returns the tablet to the medicine cassette 2, it is necessary to determine the type of the tablet. On the other hand, for example, in JP2020-25870A, a drug sorting device has been disclosed that automatically discriminates the type of tablets based on an image taken by a camera, sorts the tablets by type and stores them in a container. Note that the tablet type identification method is not limited to the one based on the image, and other methods such as component detection may be used. In addition, in a container used for storing the tablets sorted by the drug sorting device of this type, a recording medium such as an RFID tag may be provided for storing medicine identification information (medicine name, medicine code, etc.) of the tablets contained in the container. The medicine sorting device can record the types of tablets stored in the container on the recording medium of the container.

As a result, in the medicine dispensing device 1, the type of tablet stored in the container can be identified by reading the medicine identification information recorded in the RFID tag of the container with the data recording part 305. Information on the type of tablet to be stored in the container may be recorded in advance in the RFID tag of the container. Here, by displaying the medicine identification information read from the RFID tag of the container in the medicine dispensing device 1, the user can grasp the type of tablet contained in the container. However, when the medicine dispensing device 1 does not have the medicine cassette 2 corresponding to the type of the tablet contained in the container, the tablet cannot be returned to the medicine cassette 2.

On the other hand, in the medicine dispensing device 1, the control part 210 has a medicine loading support function capable of improving the work efficiency when loading the tablets accommodated in the container into the medicine cassette 2 by executing a medicine loading support processing, which will be described later. Hereinafter, an example of the medicine loading support processing will be described with reference to FIG. 60. Note that the control part 210 may execute the medicine loading support process in parallel with other processes such as medicine allocation processing (see FIG. 33), adjustment support processing (see FIGs. 34 to 36), medicine dispensing processing (see FIG. 58), and work support display processing (see FIG. 59). Further, part or all of the medicine loading support process may be executed by the control part 310 of the medicine dispensing device 1.

### <Step S81>

In step S81, the control part 210 waits for a loading start operation for loading tablets into the medicine cassette 2 (S81: No). Here, when it is determined that the loading start operation has been performed (S81: Yes), the processing proceeds to step S82.

### <Step S82>

In step S82, the control part 210 uses the data recording part 305 built in the lower part of the filling table 1a of the medicine dispensing device 1 to read the medicine identification information of the tablet contained in the container from the RFID tag of the container placed on the filling table 1a. The recording medium of the medicine identification information of the tablet contained in the container is not limited to RFID, and for example, a label or the like printed with a one-dimensional code or two-dimensional code indicating medicine identification information of the tablet is attached to the container, and in the medicine dispensing device 1, the medicine identification information may be read from the label using a bar code reader or the like.

### <Step S83>

In step S83, the control part 210 determines whether or not the medicine cassette 2 associated with the type of tablet corresponding to the medicine identification information read in step S82 is present in the medicine dispensing device 1 based on the allocation correspondence information D31 or the like. Here, when it is determined that the medicine cassette 2 associated with the tablet type is present in the medicine dispensing device 1 (S83: Yes), the processing proceeds to step S84, and when it is determined that the medicine cassette 2 associated with the tablet type is not present in the medicine dispensing device 1 (S83: No), the processing proceeds to step S831.

### <Step S831>

In step S831, the control part 210 determines whether or not the type of tablet corresponding to the medicine identification information read at step S82 is suitable with the medicine cassette 2 that can be used in the medicine dispensing device 1. Specifically, in the medicine mounting master D11, it is conceivable that cassette suitability information indicating the type of medicine cassette 2 suitable for each kind of medicine is set for each kind of medicine.

For example, when multiple types of medicine cassettes 2 with different sizes or shapes of dispensable tablets can be used with the medicine dispenser 1, for each type of tablet, the type of medicine cassette 2 suitable for the type of tablet is registered in the medicine mounting master D11 as the cassette suitability information. Further, Further, information indicating that preset tablet types including, for example, fragile tablet types, special shaped tablet types or tablet types with colored powder adhering to the medicine cassette 2 is not suitable with any of the drug cassettes 2 is registered in the drug mounting master D11 as the cassette suitability information. The control part 210 determines whether or not the type of tablet is suitable for the medicine cassette 2 based on the cassette suitability information of the medicine mounting master D11.

In addition, in the medicine dispensing device 1, when a plurality of types of medicine cassettes 2 can be used, in step S831, it is determined which of the plurality of types of medicine cassettes 2 the type of tablet is suitable for. For example, hereinafter, a medicine cassette 2 of a type in which a user can manually adjust the dispensing path using the adjustment device 100 may be referred to as a manual adjustment cassette 2A. In addition, in the medicine dispensing device 1, when one or more bases 3 are provided with an adjusting part capable of automatically adjusting the medicine cassette 2 to a plurality of states in which different types of medicines can be dispensed, it is conceivable that a medicine cassette 2 of a type that can be adjusted by the adjusting part (hereinafter sometimes referred to as "automatic adjusting cassette 2B") can be used. Furthermore, in the medicine dispensing device 1, it is conceivable that a medicine cassette 2 of a type capable of dispensing only a predetermined specific type of tablet (hereinafter sometimes referred to as a "fixed cassette 2C") is detachable from one or more bases 3. In this case, in step S831, the control part 210 determines which of the multiple types of medicine cassettes 2 of the manual adjusting cassette 2A, the automatic adjusting cassette 2B, or the fixed cassette 2C is suitable for the type of tablet corresponding to the drug identification information read in step S82.

Then, when it is determined that the type of tablet is suitable for any type of medicine cassette 2 (S831: Yes), the processing proceeds to step S832, and when it is determined that the type of tablet is not suitable for any type of medicine cassette 2 (S831: No), the processing proceeds to step S835.

### <Step S8311>

In step S8311, the control part 210 causes the display part 201 to display a specific message indicating that there is no medicine cassette 2 of a type that is suitable for the type of tablet corresponding to the medicine identification information read in step S82 to notify the user. For example, the display part 201 displays a message to the effect that the medicine cassette 2 cannot be used for the type of tablet corresponding to the medicine identification information.

### <Step S832>

On the other hand, in step S832, the control part 210 causes the display part 201 to display a cassette preparation guidance screen preset as a display screen to be displayed when the medicine cassette 2 associated with the type of tablet corresponding to the medicine identification information read in step S82 does not exist. In this embodiment, in order to explain the case where it is determined whether or not the type of tablet is suitable for the medicine cassette 2 in step S831, the cassette preparation guidance screen is displayed when there is no medicine cassette 2 associated with the type of tablet corresponding to the medicine identification information and the type of tablet is suitable for the medicine cassette 2. On the other hand, step S831 may be omitted, in this case, the cassette preparation guidance screen is displayed when the medicine cassette 2 associated with the type of tablet corresponding to the medicine identification information does not exist. For example, the cassette preparation guidance screen displays a message indicating that the medicine cassette 2 associated with the type of tablet does not exist.

Further, on the cassette preparation guidance screen, a message prompting preparation of the medicine cassette 2 corresponding to the type of tablet, a preparation start button for starting preparation of the medicine cassette 2, or the like may be displayed as guidance information. For example, when the medicine cassette 2 that is suitable for the type of tablet is the manual adjustment cassette 2A, a message prompting to allocate the tablet type to the manual adjustment cassette 2A, or an allocation start button for starting the process of allocating the tablet type to the manual adjustment cassette 2A may be displayed as guidance information on the cassette preparation guidance screen. Similarly, when the medicine cassette 2 that is suitable for the type of tablet is the automatic adjustment cassette 2B, a message prompting to allocate the tablet type to the automatic adjustment cassette 2B, or an allocation start button for starting the process of allocating the tablet type to the automatic adjustment cassette 2B may be displayed as guidance information on the cassette preparation guidance screen. Furthermore, when the medicine cassette 2 suitable for the type of tablet is the fixed cassette 2C, a message for prompting to place an order for the fixed cassette 2C corresponding to the type of tablet, or an order start button for starting the ordering of the fixed cassette 2C for the type of tablet may be displayed as guidance information on the cassette preparation guidance screen. In addition, when the types of tablets are suitable for a plurality of types of medicine cassettes 2, a plurality of pieces of guidance information may be displayed at the same time.

### <Step S833>

In step S833, the control part 210 determines whether or not to start preparing the medicine cassette 2 corresponding to the type of tablet. For example, when the user operates an operation button for starting preparation of the medicine cassette 2, such as the allocation start button or the ordering start button displayed on the cassette preparation guidance screen, the control part 210 determines to start preparation of the medicine cassette 2 corresponding to the type of tablet. Here, when it is determined that the medicine cassette 2 is to be prepared (S833: Yes), the processing proceeds to step S834. On the other hand, for example, when the user operates the cancel button or the like on the cassette preparation guidance screen, it is determined that the medicine cassette 2 is not prepared (S833: No), and the processing returns to step S81.

### <Step S834>

In step S834, the control part 210 executes cassette preparation processing for preparing the medicine cassette 2 corresponding to the type of tablet and then returns the processing to step S81.

For example, when the medicine cassette 2 suitable for the tablet type is the manual adjustment cassette 2A and the allocation start operation is operated, the control part 210 executes the medicine allocation processing and the adjustment support processing as the cassette preparation processing. In this case, in step S21 of the medicine allocation processing, the type of tablet corresponding to the medicine identification information read in step S82 is selected as the allocated tablet, and the subsequent processes are executed. As a result, a processing for allocating the type of the tablet to the medicine cassette 2, a processing for adjusting the dispensing path of the medicine cassette 2 according to the type of the tablet and the like are executed, so that it becomes possible to dispense tablets corresponding to the type. Incidentally, such adjustment support processing is executed when there is a manual adjustment cassette 2A to which tablet types have not been allocated among the manual adjustment cassettes 2A that can be used in the medicine dispenser 1.

Further, when the drug cassette 2 suitable for the type of tablet is the automatic adjustment cassette 2B and the allocation start operation is operated, as the cassette preparation process, the control part 210 may execute the medicine allocation process and the process for automatically adjusting the dispensing path of the automatic adjustment cassette 2B by the adjusting part. In addition, when the medicine cassette 2 suitable for the type of tablet is the automatic adjustment cassette 2B, the control part 210 may automatically execute the medicine allocation process and the automatic adjustment of the dispensing path of the automatic adjustment cassette 2B by the adjusting part without requiring a user operation such as the allocation start operation. Note that such adjustment support processing may be executed only when there is an automatic adjustment cassette 2B to which tablet types have not been allocated among the automatic adjustment cassettes 2B that can be used in the medicine dispensing device 1. Thus, the control part 210 when executing the medicine allocation process for the automatic adjustment cassette 2B and the automatic adjustment of the dispensing path of the automatic adjustment cassette 2B by the adjusting part is an example of the adjustment processing part according to the present invention.

Further, when the order start button is operated,
as the cassette preparation process, the control part 210 executes a processing for transmitting order information for the fixed cassette 2C corresponding to the type of tablet corresponding to the medicine identification information read in step S82 to a preset order destination. As a result, the fixed cassette 2C corresponding to the type of tablet is delivered from the ordering party to the user of the medicine dispensing device 1, and the tablet can be dispensed using the fixed cassette 2C.

Here, a case where step S834 can be executed as necessary will be described as an example, but as another embodiment, the cassette preparation process in step S834 may not be executed, and only a message or the like prompting the user to prepare the medicine cassette 2 may be displayed.

### <Steps S84 to S86>

When it is determined in step S83 that the medicine cassette 2 corresponding to the type of tablet exists in the medicine dispensing device 1 (S83: Yes), in subsequent steps S84 to S86, the control part 210 determines whether or not the test dispensing operation has been completed for the medicine cassette 22 in the same manner as in steps S63 to S65. When the test dispensing operation has not been incomplete, the control part 210 executes the test dispensing operation, and when the test dispensing operation has been completed, the processing proceeds to step S87. As another embodiment, when the test dispensing operation for the medicine cassette 22 has not been completed, the processing may proceed to step S87 without executing steps S85 and S86.

### <Step S87>

In step S87, the control part 210 executes a tablet loading processing for loading tablets of the type into the medicine cassette 2 corresponding to the type of tablet and returns the processing to step S81. Specifically, in the tablet loading processing, the control part 210 displays the position where the medicine cassette 2 corresponding to the type of tablet is mounted. For example, the control part 210 causes the display part 201 to display the cassette number or cassette ID of the medicine cassette 2 corresponding to the type of tablet based on the medicine allocation information D21 or the allocation correspondence information D31. Further, when the base 3 to which the medicine cassette 2 is attached and detached is provided with a light emitting part such as an LED, the control part 210 may light up the light emitting unit of the base 3 on which the medicine cassette 2 corresponding to the type of tablet is mounted. Further, it is conceivable that the medicine dispensing device 1 is configured to be movable between a first position where tablets can be dispensed from the medicine cassettes 2 and a second position for attaching and detaching the medicine cassettes 2. In this case, the control part 210 may move the medicine cassette 2 corresponding to the type of tablet to the second position in the tablet loading processing.

As described above, according to the medicine loading support function, when there is no medicine cassette 2 corresponding to the type of tablet that the user wants to put into the medicine dispenser 1, the user will be guided to the work that the user should do. Therefore, the work efficiency of the user is enhanced.

By the way, the RFID tag of the container may include recording medium identification information that enables identification of the medicine identification information stored in the RFID tag of the container together with the medicine identification information. As a result, the control part 210 can execute different processing depending on whether the medicine identification information read in step S82 is read from the RFID tag of the container or from a recording medium different from the RFID tag of the container.

For example, consider a situation in which the tablet type corresponding to the drug identification information fits both the manual adjustment cassette 2A and the automatic adjustment cassette 2B when the storage unit is used for returning tablets to the medicine cassette 2. In this case, when the medicine identification information is read from the RFID tag of the container, the control part 210 displays a message for prompting to allocate the type of tablet corresponding to the medicine identification information to the automatic adjustment cassette 2B, an allocation start button for starting the processing of allocating the types of tablets to the automatic adjustment cassette 2B, or the like as guidance information. On the other hand, when the medicine identification information is not read from the RFID tag of the container, the control part 210 displays a message for prompting to allocate the type of tablet corresponding to the medicine identification information to the manual adjustment cassette 2A, an allocation start button for starting the process of allocating the types of tablets to the manual adjustment cassette 2A, or the like as guidance information.

### [Cleaning notification function]

In the medicine dispensing device 1, the medicine cassette 2 or the tablet dispensing path from the medicine cassette 2 may become dirty with tablet powder or fragments. On the other hand, the medicine dispensing system 500 may have a cleaning notification function for displaying a cleaning notification screen for prompting cleaning of the medicine cassette 2 on the display part 201 when preset cleaning conditions are satisfied. As a result, it is expected that the user is urged to clean the medicine cassette 2 or the dispensing path as appropriate and that the medicine cassette 2 or the dispensing path or the like is appropriately cleaned so that a situation in which the tablets dispensed from the medicine cassette 2 are dirty is suppressed. The cleaning notification function is implemented by executing cleaning notification processing by control part 210 of control device 200. Further, the cleaning notification process may be executed by the control part 310 of the medicine dispensing device 1.

For example, one or more of the following first to fourth cleaning conditions may be set as the cleaning conditions. Then, the control part 210 determines that the cleaning condition is satisfied when any one of the following first to fourth cleaning conditions is satisfied. Further, the control part 210 may determine that the cleaning condition is satisfied when any one or more of the following first to fourth cleaning conditions are satisfied.

### <First cleaning condition>

The first cleaning condition is that a predetermined specific quantity of tablets are dispensed from the medicine cassette 2. The specific quantity may be set in advance as a judgment index for judging that cleaning of the medicine cassette 2 is necessary for each medicine cassette 2 or each type of tablet.

### < Second cleaning condition>

The second cleaning condition is that the preset first cleaning recommended medicine is dispensed from the medicine cassette 2 in a preset specific quantity. For example, in the medicine mounting master D11, first recommended cleaning medicine information indicating whether or not the tablet is the first recommended cleaning medicine may be stored for each tablet. Then, the control part 210 determines whether or not the second cleaning condition is satisfied based on the first recommended cleaning medicine information. The specific quantity may be set in advance for each medicine cassette 2 or for each type of tablet.

### <Third cleaning condition>

The third cleaning condition is such that a preset second recommended cleaning medicine is assigned to the chemical cassette 2, and after the second recommended cleaning medicine is dispensed from the medicine cassette 2, a different type of tablet from the second recommended cleaning medicine is allocated to the chemical cassette 2. For example, in the medicine mounting master D11, a second recommended cleaning medicine information indicating whether or not the tablet is the second recommended cleaning medicine may be stored for each tablet. Then, the control part 210 determines whether or not the third cleaning condition is satisfied based on the second recommended cleaning medicine information. In addition, among combinations of tablet types before and after allocation of the medicine cassette 2, a combination determined to satisfy the third cleaning condition may be set in advance.

### <Fourth cleaning condition>

The fourth cleaning condition is that after the tablet type is allocated to the medicine cassette 2, a certain period of time elapses without changing the tablet type allocated to the medicine cassette 2. The certain period of time may be set in advance for each medicine cassette 2 or for each type of tablet.

## Claims

1. A medicine dispensing system comprising:
a determination processing part that determines whether or not a medicine has been dispensed from a medicine dispensing part that can be adjusted to a plurality of states capable of dispensing different types of medicines; and
a storage processing part that stores dispensing confirmation information in a storage part in a state in which a correspondence relationship with the medicine dispensing part can be specified when the determination processing part determines that the medicine has been dispensed from the medicine dispensing part.

2. The medicine dispensing system as claimed in claim 1 further comprising a restriction processing part that restricts a medicine dispensing operation for dispensing the medicine from the medicine dispensing part based on a medicine dispensing data when the dispensing confirmation information corresponding to the medicine dispensing part is not stored in the storage part.

3. The medicine dispensing system as claimed in claim 1 or 2, wherein in a test dispensing operation that is executed after adjustment of the medicine dispensing part according to a type of the medicine to be dispensed allocated to the medicine dispensing part and dispensing the medicine from the medicine dispensing part, the determination processing part determines whether or not the medicine has been dispensed from the medicine dispensing part.

4. The medicine dispensing system as claimed in claim 3, wherein the storage processing part stores the dispensing confirmation information in a state in which a correspondence relationship between the dispensing confirmation information, the medicine dispensing part and the type of medicine allocated to the medicine dispensing part can be specified.

5. The medicine dispensing system as claimed in claim 3 or 4 further comprising a dispensing detection part capable of detecting the medicine dispensed from the medicine dispensing part, wherein in the test dispensing operation for the medicine dispensing part, the determination processing part determines that the medicine has been dispensed from the medicine dispensing part on condition that dispensation of a predetermined number of medicines has been detected by the dispensing detection part.

6. The medicine dispensing system as claimed in claim 3 or 4, wherein the determination processing part determines that the medicine has been dispensed from the medicine dispensing part on condition that a specific user operation has been performed after the test dispensing operation for the medicine dispensing part has been performed.

7. The medicine dispensing system as claimed in claim 3 or 4, wherein the determination processing part determines that the medicine has been dispensed from the medicine dispensing part on condition that the test dispensing operation for the medicine dispensing part is completed.

8. The medicine dispensing system as claimed in any one of claims 3 to 7 further comprising a reception processing part capable of receiving input of one or more of a quantity of the medicines to be filled in the medicine dispensing part, a date of filling and an expiration date before start of the test dispensing operation.

9. The medicine dispensing system as claimed in any one of claims 1 to 8 further comprising a display processing part that displays presence or absence of the dispensing confirmation information corresponding to the medicine dispensing part on a display screen that is displayed when the medicine dispensing part is filled with the medicine.

10. The medicine dispensing system as claimed in any one of claims 1 to 9, wherein the storage part is provided in the medicine dispensing part corresponding to the dispensing confirmation information.

11. The medicine dispensing system as claimed in any one of claims 1 to 10, wherein the storage processing part stores the dispensing confirmation information, identification information of the medicine dispensing part and the type of medicine allocated to the medicine dispensing part in association with each other in the storage part.

12. An adjusting system comprising:
a detection processing part capable of detecting a current adjustment value of a dimension of a dispensing path by an adjusting part capable of manually adjusting the dimension of the dispensing path of a medicine in a medicine dispensing part; and
a display processing part that displays the current adjustment value detected by the detection processing part and a target value for adjustment in a comparable manner.

13. The adjusting system as claimed in claim 12, wherein the display processing part displays adjustment difference information capable of specifying a difference between the current adjustment value and the target value.

14. The adjusting system as claimed in claim 13, wherein the display processing part displays a second region having an area, length or width corresponding to difference between the target value and the current adjustment value and a third region having an area, length or width corresponding to the current adjustment value in a first region having an area, length or width corresponding to the target value.

15. The adjusting system as claimed in any one of claims 12 to 14 further comprising a notification processing part that notifies a user when difference between the current adjustment value and the target value reaches a predetermined range or less.

16. The adjusting system as claimed in any one of claims 12 to 15 further comprising:
an initial position detection part capable of detecting that the adjusting part is in an initial state; and
an operation amount detection part capable of detecting an operation amount of the adjusting part,
wherein the detection processing part identifies the current adjustment value based on the operation amount of the adjusting part detected by the operation amount detection part after the initial position detection part detects that the adjusting part is in the initial state.

17. The adjusting system as claimed in any one of claims 12 to 16 comprising: a first display processing part for displaying a list of a plurality of work process groups included in manual adjustment work using the adjusting part; and a second display processing part that sequentially displays one or more work processes included in each of the work process groups after the work process group is displayed by the first display processing part.

18. A program for causing a processor to execute:
a determination step for determining whether or not a medicine has been dispensed from a medicine dispensing part that can be adjusted to a plurality of states capable of dispensing different types of medicines; and
a storage step for storing dispensing confirmation information in a storage part in a state in which a correspondence relationship with the medicine dispensing part can be specified when the determination step determines that the medicine has been dispensed from the medicine dispensing part.

19. A program for causing a processor to execute:
a detecting step capable of detecting a current adjustment value of a dimension of a dispensing path by an adjusting part capable of manually adjusting the dimension of the dispensing path of a medicine in a medicine dispensing part; and
a display step for displaying the current adjustment value and a target value for adjustment detected by the detection step in a comparable manner.

20. A medicine dispensing system comprising:
an allocation processing part for allocating a type of a medicine to a medicine dispensing part that can be adjusted to a plurality of states that can dispense different types of medicines;
a storage processing part that stores specific information as to whether or not a collating operation of information read from a recording medium provided in the medicine dispensing part has been completed after completion of an adjustment operation of the medicine dispensing part according to the type of the medicine allocated to the medicine dispensing part, or whether or not a dispensing operation for dispensing a type of medicine allocated to the medicine dispensing part from the medicine dispensing part has been completed after the completion of the adjustment operation; and
a display processing part that displays presence of one or more incomplete medicine dispensing parts for which the collation operation or the dispensing operation has not been completed, based on the specific information.

21. The medicine dispensing system as claimed in claim 20, wherein the display processing part displays a list of one or more of the incomplete medicine dispensing parts according to user operation.

22. The medicine dispensing system as claimed in claim 20 or 21, wherein the display processing part displays one or more of the incomplete medicine dispensing parts at a preset specific timing.

23. A medicine dispensing system comprising:
a storage processing part that stores a correspondence relationship between a medicine dispensing part that dispenses a medicine and a type of a medicine corresponding to the medicine dispensing part;
a determination processing part that determines based on the correspondence relationship whether or not the medicine dispensing part corresponding to the type of the medicine indicated by a medicine identification information read from a recording medium exists; and
a display processing part that displays a predetermined specific display screen when the determination processing part determines that the medicine dispensing part corresponding to the type of the medicine does not exist.

24. The medicine dispensing system as claimed in claim 23, wherein the display processing part displays, on the specific display screen, preparation guidance information prompting preparation of the medicine dispensing part corresponding to the type of the medicine indicated by the medicine identification information.

25. The medicine dispensing system as claimed in claim 24 comprising a plurality of types of the medicine dispensing parts, wherein the display processing part displays, on the specific display screen, preparation guidance information prompting preparation of the medicine dispensing part of a type suitable for the type of the medicine indicated by the medicine identification information.

26. The medicine dispensing system as claimed in claim 25, wherein the display processing part displays a specific message on the specific display screen without displaying the guidance information when there is no medicine dispensing part of a type suitable for the type of the medicine indicated by the medicine identification information.

27. The medicine dispensing system as claimed in any one of claims 23 to 26 comprising:
an adjusting part capable of automatically adjusting the medicine dispensing part to a plurality of states capable of dispensing different types of medicines; and
an adjustment processing part that automatically executes adjustment of the medicine dispensing part by the adjusting part after the specific display screen is displayed, in accordance with a user's operation or automatically.

28. The medicine dispensing system as claimed in any one of claims 23 to 26, wherein a mounting part to which the medicine dispensing part is mounted is not provided with an adjusting part capable of automatically adjusting the medicine dispensing part to a plurality of states that allow dispensing of different types of medicines.

29. The medicine dispensing system as claimed in any one of claims 23 to 28 further comprising a medicine discriminating device that identifies types of medicines, stores the medicines in corresponding containers for each type of the medicine and records the medicine identification information corresponding to the types of the medicines on the recording medium provided in the container.
